(19) Europäisches Patentamt
European Patent Office
Office européen des brevets

(11) **EP 4 587 833 B1**

(12) **EUROPÄISCHE PATENTSCHRIFT**

(45) Veröffentlichungstag und Bekanntmachung des Hinweises auf die Patenterteilung:
**25.02.2026 Patentblatt 2026/09**

(21) Anmeldenummer: **23785976.4**

(22) Anmeldetag: **15.09.2023**

(51) Internationale Patentklassifikation (IPC):
**G01N 33/543** (2006.01)    **C12N 15/82** (2006.01)

(52) Gemeinsame Patentklassifikation (CPC):
**G01N 33/54388;** G01N 2333/405; G01N 2333/415; G01N 2333/59

(86) Internationale Anmeldenummer:
**PCT/EP2023/075545**

(87) Internationale Veröffentlichungsnummer:
**WO 2024/056905 (21.03.2024 Gazette 2024/12)**

(54) **IMMUNOASSAY ZUM NACHWEISEN BIOLOGISCH AKTIVER PROTEINE**

IMMUNOASSAY FOR VERIFYING BIOLOGICALLY ACTIVE PROTEINS

DOSAGE IMMUNOLOGIQUE PERMETTANT DE VÉRIFIER DES PROTÉINES BIOLOGIQUEMENT ACTIVES

(84) Benannte Vertragsstaaten:
**AL AT BE BG CH CY CZ DE DK EE ES FI FR GB GR HR HU IE IS IT LI LT LU LV MC ME MK MT NL NO PL PT RO RS SE SI SK SM TR**

(30) Priorität: **15.09.2022 LU 103006**

(43) Veröffentlichungstag der Anmeldung:
**23.07.2025 Patentblatt 2025/30**

(73) Patentinhaber: **PHAEOSYNT GMBH**
**30163 Hannover (DE)**

(72) Erfinder:
• **EILERS, Alina**
**Hannover (DE)**
• **PLOENNINGS, Eva-Maria**
**Hannover (DE)**
• **PFEIL-COENEN, Stephanie**
**Hannover (DE)**

(74) Vertreter: **Gruner, Leopold Joachim**
**White ip | Patent & Legal GmbH**
**Königstraße 7**
**01097 Dresden (DE)**

(56) Entgegenhaltungen:
EP-A1- 2 444 495    EP-A1- 2 660 323
EP-A1- 2 671 950    WO-A1-2021/021605

WO-A2-01/29242        US-A- 6 080 560
US-A1- 2002 053 094

• O'KENNEDY RICHARD ET AL: "Technology advancements in antibody purification", ANTIBODY TECHNOLOGY JOURNAL, vol. Volume 6, 26 August 2016 (2016-08-26), pages 17 - 32, XP93107407, ISSN: 2230-3170, Retrieved from the Internet <URL:https://www.dovepress.com/getfile.php?fileID=32094> DOI: 10.2147/ANTI.S64762
• MELNIK STANISLAV ET AL: "Cloning and plant-based production of antibody MC10E7 for a lateral flow immunoassay to detect [4-arginine] microcystin in freshwater", vol. 16, no. 1, 5 June 2017 (2017-06-05), GB, pages 27 - 38, XP093028342, ISSN: 1467-7644, Retrieved from the Internet <URL:https://api.wiley.com/onlinelibrary/tdm/v1/articles/10.1111%2Fpbi.12746> DOI: 10.1111/pbi.12746
• FRANZISKA HEMPEL ET AL: "An engineered diatom acting like a plasma cell secreting human IgG antibodies with high efficiency", MICROBIAL CELL FACTORIES, vol. 11, no. 1, 1 January 2012 (2012-01-01), pages 126, XP055040968, ISSN: 1475-2859, DOI: 10.1186/1475-2859-11-126

- SLATTERY SAMUEL S. ET AL: "Phosphate-regulated expression of the SARS-CoV-2 receptor-binding domain in the diatom Phaeodactylum tricornutum for pandemic diagnostics", vol. 12, no. 1, 29 April 2022 (2022-04-29), XP093027998, Retrieved from the Internet <URL:https://www.ncbi.nlm.nih.gov/pmc/articles/PMC9051505/pdf/41598_2022_Article_11053.pdf> DOI: 10.1038/s41598-022-11053-7
- YANGMIN GONG ET AL: "Microalgae as platforms for production of recombinant proteins and valuable compounds: progress and prospects", JOURNAL OF INDUSTRIAL MICROBIOLOGY & BIOTECHNOLOGY, SPRINGER, BERLIN, DE, vol. 38, no. 12, 1 September 2011 (2011-09-01), pages 1879 - 1890, XP019982203, ISSN: 1476-5535, DOI: 10.1007/S10295-011-1032-6
- FRANZISKA HEMPEL ET AL: "Algae as Protein Factories: Expression of a Human Antibody and the Respective Antigen in the Diatom Phaeodactylum tricornutum", PLOS ONE, PUBLIC LIBRARY OF SCIENCE, US, vol. 6, no. 12, 1 December 2011 (2011-12-01), pages e28424 - 1, XP002681385, ISSN: 1932-6203, DOI: 10.1371/JOURNAL.PONE.0028424
- GRIESBECK C ET AL: "Chlamydomonas reinhardtii", MOLECULAR BIOTECHNOLOGY, SPRINGER US, NEW YORK, vol. 34, no. 2, Sp. Iss. SI, 1 October 2006 (2006-10-01), pages 213 - 223, XP002681386, ISSN: 1073-6085
- AYALA MARTA ET AL: "Production of Plantibodies in Nicotiana Plants", ANTIBODY-DRUG CONJUGATES; IN: METHODS IN MOLECULAR BIOLOGY; ISSN 1064-3745; VOL. 263; [METHODS IN MOLECULAR BIOLOGY; ISSN 1064-3745], HUMANA PRESS, US, vol. 483, 1 January 2009 (2009-01-01), pages 103 - 134, XP008118830, ISBN: 978-1-62703-541-5, [retrieved on 20090129], DOI: 10.1007/978-1-59745-407-0_7
- O'KENNEDY RICHARD ET AL: "Technology advancements in antibody purification", ANTIBODY TECHNOLOGY JOURNAL, vol. Volume 6, 26 August 2016 (2016-08-26), pages 17 - 32, XP093107407, ISSN: 2230-3170, Retrieved from the Internet <URL:https://www.dovepress.com/getfile.php?fileID=32094> DOI: 10.2147/ANTI.S64762

Bemerkungen:

Das gesamte Dokument mit Referenztabelle(n) und Sequenzliste(n) kann von der Webseite des EPA heruntergeladen werden

**Beschreibung**

**TECHNISCHES GEBIET**

Die vorliegende Erfindung betrifft einen Immunoassay zum Nachweisen eines biologisch aktiven Proteins, eine Vorrichtung, in der ein solcher Immunoassay angeordnet ist sowie die Verwendung eines Hilfsproteins und ein Verfahren zum Nachweis eines biologisch aktiven Antigens.

**STAND DER TECHNIK**

Es gibt viele verschiedene Immunoassays für die Analyse von Körperflüssigkeiten eines Individuums, wie bspw. Vollblut, Serum, Speichel, Milch oder Urin.

Ein Problem gängiger Immunoassays, die sich der Verwendung von Antikörpern bedienen, besteht darin, dass die Antikörper tierischen Ursprungs sind. So erfolgt die Produktion von Antikörpern entweder in Tieren oder in tierischen Zellkulturen. Dabei kommen vor allem CHO- (Eileiterzellen des chinesischen Hamsters) und HEK-293T-Zellen (menschliche embryonale Nierenzellen) zum Einsatz. Diese Zellkultur-basierte Produktion ist sehr kostenintensiv, daher werden vorhandene Kapazitäten vor allem bei der Produktion hochpreisiger, therapeutischer Antikörper eingesetzt. Im Bereich der Diagnostik eingesetzte Antikörper weisen viel niedrigere Margen auf und werden daher immer noch häufig in Tieren hergestellt. Solche in Tieren produzierten Antikörper besitzen neben dem damit verbundenen Tierleid auch die geringe Zuverlässigkeit der Bindungseigenschaften, die limitierte Verfügbarkeit sowie die Gefahr der Kontamination mit Humanpathogenen. Bisher gibt es keine technischen Lösungen, die die Herstellung rein veganer Assays kostengünstig und in wirtschaftlich relevanten Produktionsmengen erlauben würden.

Die Alternativen für die Herstellung von Antikörpern ebenso wie von anderen komplexen Proteinen sind recht eingeschränkt. Bakterien wie *Escherichia coli* können die notwendigen posttranslationalen Modifikationen an den Antikörpern nicht durchführen, außerdem sind die Produktionsraten extrem niedrig. Ähnliches gilt auch für Hefe- bzw. Pichia-basierte Produktionssysteme. Auch hier können die Antikörper durch die Wirtszellen selten korrekt gefaltet werden und die Produktionsraten sind ähnlich niedrig wie in Bakterien. Transgene Pflanzen werden seit vielen Jahren als alternative Produzenten diskutiert, aber auch hier erlauben die sehr niedrigen Produktionsraten keine wirtschaftliche Verwertung.

Es gibt Versuche, höhere Pflanzen zur Produktion von Antikörpern zu verwenden, wie zum Beispiel in der Patentschrift US 6 080 560 A offenbart. Weitere Ansätze werden in Publikationen offenbart, zum Beispiel von Melnik Stanislav et al. (*DOI: 10.1111/pbi. 12746*) und Ayala Marta et al. (*DOI: 10.1007/978-1-59745-407-0_7*). Das Problem bei diesen Pflanzen ist, dass die Produktionsraten von Antikörpern bei Pflanzen im Allgemeinen sehr niedrig sind, da typischerweise nur Zellen einiger Gewebe der Pflanze die Antikörpergene überhaupt in ihr Genom integriert haben, so dass nur bestimmte Gewebe geringe Mengen an Antikörpern produzieren. Die Pflanze ist eine Chimäre, sie enthält viele Gewebe, die gar keine Antikörpergene aufgenommen haben, was die Ausbeute weiter senkt. Damit ein Antikörper auch über mehrere Generationen produziert werden kann, muss zusätzlich gewährleistet sein, dass die Keimbahnzellen die Antikörpergene in ihrem Genom integriert haben, um eine dauerhafte Produktion zu gewährleisten. Schließlich erfordert die dauerhafte Produktion eine Selbstung oder Pfropfung der transgenen Pflanzen. Bei einer "natürlichen" sexuellen Vermehrung wäre das Transgen nach wenigen Generationen nicht mehr vorhanden.

Ein alternatives Verfahren nutzt die Agroinfiltration von gentechnisch veränderten Pflanzenviren, mit denen Pflanzen infiziert werden. Ein Problem bei diesen Pflanzen ist, dass sie transient modifiziert werden, die Antikörpergene also nicht in eines der Pflanzengenome integriert sind, d. h. nur über eine Generation hinweg stabil Antikörper exprimieren können. Bei diesem Ansatz kann tatsächlich jede Zelle der Pflanze die Antikörper produzieren, da sich das Virus in der Pflanze ausbreitet. Allerdings erfolgt hier keine Integration in das pflanzliche Genom und die Pflanze überlebt diese Prozedur nicht, sondern stirbt innerhalb weniger Tage. Das bedeutet, dass keine dauerhaft gleichbleibende Produktion gewährleistet werden kann und es sich rechtlich immer um Neugenerierungen transgener Pflanzen mit all den genehmigungsrechtlichen Konsequenzen handelt.

Darüber hinaus ist die Aufreinigung problematisch, da höhere Pflanzen erhebliche Mengen an faserigem Material produzieren, das die Abtrennung und Isolierung von Antikörpern erschwert und daher nicht geeignet ist, Antikörper in vergleichbarer Qualität und Quantität wie die tierischen Alternativen zu liefern.

Weiterhin sind bereits mehrere Forschungsdokumente und Patentschriften für die Expression von Antikörpern aus Kieselalgen bekannt. Beispiele in der Literatur für sezernierende Antikörper aus Kieselalgen sind unter anderem die von Hempel *et al.* (*DOI: 10.1186/1475-2859-11-126*), Samuels *et al.* (*DOI: 10.1038/s41598-022-11053-7*) und Hempel *et al.* (*DOI: 10.1371/JOURNAL.PONE.0028424*) publizierten Arbeiten. Ein gemeinsames Merkmal dieser Ansätze ist, dass diese Antikörper extrazellulär exprimiert werden, was den bedauerlichen Nachteil hat, dass diese Methoden nicht in der Lage sind, homogene, einheitliche Antikörper in hoher Qualität und in technisch realisierbaren Mengen zu produzieren, die kommerziell als Alternative zu etablierten Antikörpern aus tierischen Quellen verwendet werden können. Dies ist ebenfalls bei den folgenden Patentschriften der Fall. Die Produktion von Antikörpern in *Phaeodactylum tricornutum,* einer Mikroalge, wurde beschrieben. So offenbart bspw. die EP 2 671 950 A1 die Expression und Sekretion von rekombinanten, vollständig assemblierten Proteinkomplexen durch Mikroalgen. Hier werden die Mikroalgen extrazellulär exprimiert, was theoretisch zu einer leichteren Abtrennung der Proteine führt; ein bedauernswerterweise Nachteil dieser Methode sind die sehr niedrigen Produktionsraten, wodurch die Antikörper bedauernswerterweise nur durch sehr hohen Aufwand aufgereinigt werden können. Die Patentschriften EP 2 444 495 A1 und

EP 2 660 323 A1 beschreiben einen Ansatz zur Herstellung therapeutischer Antikörper unter Verwendung einer transformierten Mikroalge namens *Phaeodactylum tricornutum.* Die transformierte Mikroalge enthält eine Nukleinsäuresequenz, die für einen therapeutischen Antikörper, ein funktionelles Fragment oder ein Derivat davon kodiert, gekoppelt mit einem heterologen Signalpeptid, die alle operativ mit einem Promotor verbunden sind. Diese transformierten Mikroalgen exprimieren die therapeutischen Antikörper extrazellulär in das Medium, es handelt sich also um sekretierte Antikörper. Dieser Ansatz liefert bedauernswerterweise nur geringe Mengen therapeutischer Antikörper, was auf die ineffiziente Verwendung einer extrazellulären Expression zurückzuführen ist.

## AUFGABE

Der vorliegenden Erfindung liegt daher die technische Aufgabe zugrunde, einen Immunoassay bereitzustellen, der die aus dem Stand der Technik bekannten Nachteile überwindet, insbesondere die Bereitstellung von rekombinanten Antikörpern aus einer Kieselalge oder einzelligen Pflanze in hoher Qualität und technisch nutzbaren Mengen.

## LÖSUNG

Um die technische Aufgabe aus dem Stand der Technik zu lösen, umfasst eine bevorzugte Ausführungsform zum Nachweisen eines biologisch aktiven Antigens, insbesondere eines Hormons, Proteins oder Impfstoffs, in einer biologischen Probe eines Individuums, den Einsatz von rekombinanten Antikörpern, bspw. eines ersten Antikörpers, eines zweiten Antikörpers und/oder eines weiteren Antikörpers, die aus einer Kieselalge, einzelligen Pflanze oder Viridiplantae gewonnen wurden, wobei der rekombinante Antikörper ein heterologes kieselalgen-, pflanzen- oder mikroalgenspezifisches Signalpeptid und/oder ein von dem eines nativen Antikörpers, der aus einem Individuum (wie hierin definiert) gewonnen wurde, abweichendes Glykosylierungsmuster aufweist.

Nach einer bevorzugten Ausgestaltung wurden zumindest zwei Antikörper, bspw. der erste Antikörper und der zweite Antikörper, besonders bevorzugt alle eingesetzten Antikörper aus einer Kieselalge, einzelligen Pflanze oder Viridiplantae gewonnen.

Die Aufgabe wird durch einen Immunoassay mit den Merkmalen des Anspruchs 1 sowie eine Vorrichtung und ein Verfahren mit den Merkmalen der nebengeordneten Ansprüche gelöst. Weitere vorteilhafte Ausgestaltungen sind den Unteransprüchen, der Beschreibung und den Ausführungsbeispielen zu entnehmen. Die Vorteile des Immunoassays und der anderen Komponenten ergeben sich im Folgenden aus der weiteren Beschreibung.

Gemäß einer bevorzugten Ausführungsform der vorliegenden Erfindung wird ein Immunoassay zum Nachweisen eines biologisch aktiven Antigens, insbesondere eines Hormons, Proteins oder Impfstoffs in einer biologischen Probe eines Individuums bereitgestellt, der folgende Komponenten aufweist:

- einen Probenauftragsbereich zum Auftragen einer biologischen Probe eines Individuums, wobei die biologische Probe vorzugsweise Urin, Vollblut, Speichel, Milch oder Serum eines Individuums ist;
- einen Fangbereich, wobei der Fangbereich einen immobilisierten ersten Antikörper aufweist, der gegen das biologisch aktive Antigen, insbesondere gegen das Hormon, das Protein oder den Impfstoff, gerichtet ist;
- einen Konjugatbereich, wobei der Konjugatbereich einen zweiten Antikörper aufweist, der gegen das biologisch aktive Antigen, insbesondere gegen das Hormon, das Protein, das Peptid oder den Impfstoff, gerichtet ist; und optional
- einen Kontrollbereich, der einen Antikörper aufweist, der gegen den Detektionsantikörper gerichtet ist;

wobei der erste Antikörper und der zweite Antikörper ein rekombinanter Antikörper ist, der aus einer Kieselalge oder einzelligen Pflanze gewonnen wurde, wobei der Immunoassay zumindest einen weiteren Antikörper und/oder ein Hilfsprotein aufweist, wobei das Hilfsprotein als Blocker freie Oberflächen eines Reaktionsgefäßes oder einer Membran absättigt, wobei der weitere Antikörper und das Hilfsprotein aus einer Kieselalge und/oder Viridiplantae und/ oder einzelligen Pflanze gewonnen wurde, und wobei der rekombinante Antikörper in einer Reinheit von mindestens 90 % bereitgestellt wird, und wobei der Immunoassay vegan ist.

Offenbart ist ferner ein **Immunoassay** zum Nachweisen eines biologisch aktiven Antigens gelöst, insbesondere eines Hormons, Proteins oder Arzneistoffs in einer biologischen Probe eines Individuums, aufweisend:

- einen Probenauftragsbereich zum Auftragen einer biologischen Probe eines Individuums, wobei die biologische Probe vorzugsweise Urin, Vollblut, Speichel, Milch oder Serum ist;
- einen Fangbereich, wobei der Fangbereich einen immobilisierten ersten Antikörper aufweist, der gegen das biologisch aktive Antigen, insbesondere gegen das Hormon, das Protein, Peptid oder den Arzneistoff, gerichtet ist;
- einen Konjugatbereich, wobei der Konjugatbereich einen zweiten Antikörper aufweist, der gegen das biologisch aktive Antigen, insbesondere gegen das Hormon, das Protein, Peptid oder den Arzneistoff, gerichtet ist;

wobei der erste Antikörper und/oder der zweite Antikörper ein rekombinanter Antikörper ist, der aus einer Kieselalge oder einzelligen Pflanze gewonnen wurde,

wobei der rekombinante Antikörper in einer Reinheit von mindestens 90 %, bevorzugter von mindestens 95 % bereitgestellt wird. In einer alternativ bevorzugten Ausführung wird der rekombinante Antikörper in einer Reinheit von 80 bis 99 % Reinheit, bevorzugter 85 bis 99 %, speziell bevorzugt in 90 bis 99 % am bevorzugtesten 95 bis 99 % zur Verfügung gestellt.

Die biologische Probe eines Individuums kann Vollblut, Serum, Speichel, Urin oder Milch darstellen. Vorzugsweise ist die biologische Probe des Individuums Urin, Vollblut oder Serum, besonders bevorzugt Urin.

Nach einer bevorzugten Ausgestaltung der vorliegenden Erfindung ist der Immunoassay ein Lateral-Flow-Immunoassay. Beispielsweise weist der Lateral-Flow-Immunoassay einen Probenauftragsbereich, einen Konjugatbereich und einen Fangbereich auf, die auf einer Membran angeordnet sind, wobei ein Probenauftragsbereich und ein Fangbereich auf der Membran über einen Strömungsweg miteinander fluidverbunden sind, und wobei ein Konjugatbereich im Strömungsweg angeordnet ist. Beispielsweise ist die Membran eine Nitrocellulosemembran. Im folgenden bezieht sich der Term "Ausgestaltung" bzw. "Ausführungsform" oder vewandte Termina auf Ausgestaltungen in der Beschreibung, und verweisen nur dann auf die Erfindung, wenn sie unter den Schutzumfang fallen, der durch die Ansprüche definiert ist.

Bevorzugt stellt der Immunoassay eine Nitrocellulosemembran bereit.

Alternativ bevorzugt ist der Immunoassay ein Enzyme-linked Immunosorbent Assay (ELISA), bspw. ein direkter ELISA, ein indirekter ELISA, ein direkter Sandwich-ELISA oder ein indirekter Sandwich-ELISA, vorzugsweise ist der ELISA ein direkter Sandwich-ELISA oder ein indirekter Sandwich-ELISA.

Es kann zweckdienlich sein, dass der erste Antikörper und/oder der zweite Antikörper, insbesondere der mobilisierte der beiden Antikörper, mit einem Farbstoff und/oder einem optisch aktiven Nanopartikel, insbesondere einem Goldnanopartikel, markiert ist.

Gleichwohl kann vorgesehen sein, dass der erste Antikörper und/oder der zweite Antikörper, insbesondere der mobilisierte der beiden Antikörper, mit einem Enzym gekoppelt ist, welches dazu eingerichtet ist, einen Farbstoff oder eine Lumineszenzreaktion zu induzieren

## ALLGEMEINE VORTEILE

Für die Bereitstellung des Immunoassays oder einem Verfahren zum Nachweisen eines biologisch aktiven Antigens in einer biologischen Probe eines Individuums werden rekombinante Antikörper verwendet, die nicht mammalen Syntheseursprungs sind, sodass vorteilhaft auf Tiere oder tierische Zellkulturen, insbesondere mammale Zellkulturen, verzichtet werden kann.

Darüber hinaus können durch die Synthese/Expression der hierin eingesetzten rekombinanten Antikörper in einer Kieselalge nicht nur ähnlich hohe Expressionsraten bei viel niedrigerem Energie- und Ressourcenbedarf erzielt werden, sondern es entfällt auch die Gefahr, dass die Antikörper mit Humanpathogenen kontaminiert sein können, wie dies bei der Synthese/Expression in Tieren oder tierische Zellkulturen gegeben ist.

## AUSFÜHRLICHE BESCHREIBUNG DER ERFINDUNG

In einer Ausführungsform wird ein Immunoassay zum Nachweisen eines biologisch aktiven Antigens, insbesondere eines Hormons, Proteins oder Arzneistoffs in einer biologischen Probe eines Individuums, aufweisend:

- einen Probenauftragsbereich zum Auftragen einer biologischen Probe eines Individuums, wobei die biologische Probe vorzugsweise Urin, Vollblut, Speichel, Milch oder Serum ist;
- einen Fangbereich, wobei der Fangbereich einen immobilisierten ersten Antikörper aufweist, der gegen das biologisch aktive Antigen, insbesondere gegen das Hormon, das Protein oder den Arzneistoff, gerichtet ist;
- einen Konjugatbereich, wobei der Konjugatbereich einen zweiten Antikörper aufweist, der gegen das biologisch aktive Antigen, insbesondere gegen das Hormon, das Protein oder den Arzneistoff, gerichtet ist;

wobei der erste Antikörper und/oder der zweite Antikörper ein rekombinanter Antikörper ist, der aus einer Kieselalge, einzelligen Pflanze oder Viridiplantae, gewonnen wurde, wobei der rekombinante Antikörper ein heterologes kieselalgen-, pflanzen- oder mikroalgenspezifisches Signalpeptid aufweist.

In einer besonders bevorzugten Ausführungsform wird ein Immunoassay zum Nachweisen eines biologisch aktiven Antigens bereitgestellt, insbesondere zum Nachweis eines Hormons, Proteins oder Arzneistoffs in einer biologischen Probe eines Individuums, aufweisend:

- einen Probenauftragsbereich zum Auftragen einer biologischen Probe eines Individuums, wobei die biologische Probe vorzugsweise Urin, Vollblut, Speichel, Milch oder Serum ist;

- einen Fangbereich, wobei der Fangbereich einen immobilisierten ersten Antikörper aufweist, der gegen das biologisch aktive Antigen, insbesondere gegen das Hormon, das Protein, Peptid oder den Arzneistoff, gerichtet ist;

- einen Konjugatbereich, wobei der Konjugatbereich einen zweiten Antikörper aufweist, der gegen das biologisch aktive Antigen, insbesondere gegen das Hormon, das Protein, Peptid oder den Arzneistoff, gerichtet ist;

wobei der erste Antikörper und/oder der zweite Antikörper ein rekombinanter Antikörper ist, der aus einer Kieselalge oder einzelligen Pflanze gewonnen wurde, wobei der rekombinante Antikörper in einer Reinheit von mindestens 90 %, bevorzugter von mindestens 95 % bereitgestellt wird. In einer alternativ bevorzugten Ausführung wird der rekombinante Antikörper in einer Reinheit von 80 bis 99 % Reinheit, bevorzugter 85 bis 99 %, speziell bevorzugt in 90 bis 99 % am bevorzugtesten 95 bis 99 % zur Verfügung gestellt.

Dabei kann zwischen zwei verschiedenen Arten an Verunreinigung unterschieden werden:

- Verunreinigung durch Nichtproteine, beispielsweise Pigmente;

- Verunreinigungen durch weitere Proteine, die keine Antikörper sind.

Beide Gruppen an Verunreinigungen können die Aufreinigung und Funktion der Antikörper stören, dadurch dass die Stabilität der Antikörper negativ beeinflusst wird, aber auch durch Herabsetzung der Spezifität der Immunoassays.

Nichtproteine können im allgemeinen Reste aus dem Trennverfahren, insbesondere Zellteile, Bestandteile des Kulturmediums und bei höheren Pflanzen zum Beispiel Fasern sein. Bei der Produktion von Antikörpern in Kieselalgen gehören in diese Gruppe vor allem Pigmente, die spektralphotometrisch detektiert und einfach abgetrennt werden können, da sie ein sehr viel niedrigeres Molekulargewicht besitzen als die Antikörper und andere chemische Eigenschaften aufweisen. Dafür bieten sich gängige und dem Fachmann bekannte Methoden wie Dialyse, Ultrafiltration, Größenausschlusschromatographie oder ladungsabhängige-Trennung (z.B. Ionenaustauschchromatographie) an. Diese Abtrennung ist mit Aufwand verbunden, der, bezogen auf die Menge des isolierten Antikörpers, ansteigt, je geringer der Anteil der Antikörper an der Kultur ist. Nach Abtrennung kann die Menge der Nichtproteine bevorzugt gravimetrisch, im Fall von Pigmenten besonders bevorzugt spektroskopisch bestimmt werden.

Verunreinigungen durch Proteine, die keine Antikörper darstellen, können durch denaturierende SDS-Polyacrylamidgelelektrophorese (SDS-PAGE), kombiniert mit einer Coomassiefärbung einerseits, und einer Immunfärbung zur Identifizierung der Antikörper, bevorzugt der in Kieselalgen produzierten Antikörper-Ketten, andererseits analysiert werden. Dazu werden die aus Kieselalgen isolierten Proteingemische nach Zusatz eines entsprechenden Puffers und Denaturierung (10 min bei 80 °C) ihrer Größe nach aufgetrennt. Dieses Standardverfahren der Molekularbiologie ist dem Fachmann bekannt (z. B. Reinard, Molekularbiologische Methoden 2.0 (UTB, S. 229 ff; ISBN 978-3825287955). Die in der SDS-PAGE aufgetrennten Proteine werden mit Coomassie angefärbt und die Farbintensität densitometrisch gemessen und/oder mit einem Standard verglichen. So kann ermittelt werden, wie viel Protein in jeder Bande vorhanden ist.

Parallel dazu kann eine weitere SDS-PAGE mit identischem Auftrag der Proben durchgeführt werden, die jedoch nicht gefärbt, sondern auf eine Nitrocellulosemembran übertragen wird. Auf dieser Membran sind die Banden, die durch die beiden Ketten des Antikörpers verursacht werden, deutlich zu erkennen. Durch die Zugabe eines ersten spezifisch gegen die Antikörperketten gerichteten Antikörpers, der markiert ist, beispielsweise mit einem Biotin, Radioisotop, Reporterenzym, Oligonukleotid oder Fluorophor oder die Zugabe eines zweiten, markierten Antikörpers, welcher gegen den ersten Antikörper gerichtet ist, werden die beiden Ketten des nachzuweisenden Antikörpers deutlich sichtbar. Alle Banden, die nicht auf diese Weise markiert sind, sind kontaminierende Proteine.

In einer bevorzugten Ausführungsform wird das Verhältnis von rekombinantem Antikörper zu Gesamtprotein durch SDS-PAGE nach Auftrennung und spektroskopischer Quantifizierung der Nichtproteine bestimmt. Dieses Verfahren ermöglicht die Bestimmung der absoluten Menge der Antikörper, Gesamtproteine und Nichtproteine eines gewonnen, rekombinanten Antikörpers im Sinne der vorliegenden Erfindung.

Die Reinheit des gereinigten rekombinanten Antikörpers, der für einen Immunoassay gemäß der vorliegenden Erfindung bereitgestellt wird, wird nach der folgenden Formel berechnet:

$$\text{Reinheit (\%)}= \frac{\text{(Menge des rekombinanten Antikörpers)}}{\text{(Menge der Gesamtproteine)}+\text{(Menge der Nichtproteine)}} \times 100 \text{ \%}$$

Die Reinheit des rekombinanten Antikörpers wird zum Zeitpunkt nach der Aufreinigung bestimmt, bevor der Antikörper auf einen Immunoassay im Sinne der vorliegenden Erfindung aufgebracht oder anderweitig zur Verfügung gestellt wird.

In einer alternativen Ausführungsform der vorliegenden Erfindung wird der rekombinante Antikörper in einer Proteinreinheit von mindestens 90 %, bevorzugter von mindestens 95 % bereitgestellt wird. In einer alternativ bevorzugten Ausführung wird der rekombinante Antikörper in einer Proteinreinheit von 80 bis 99 % Reinheit, bevorzugter 85 bis 99 %, speziell bevorzugt in 90 bis 99 % am bevorzugtesten 95 bis 99 % zur Verfügung gestellt.

Die Proteinreinheit des rekombinanten Antikörpers wird nach der folgenden Formel berechnet:

$$\text{Proteinreinheit (\%)}= \frac{\text{(Menge des rekombinanten Antikörpers)}}{\text{(Menge der Gesamtproteine)}} \times 100 \text{ \%}$$

Nach einer bevorzugten Ausführungsform wird das Verhältnis von rekombinantem Antikörper zu Gesamtprotein durch SDS-PAGE bestimmt. Die Proteinreinheit des rekombinanten Antikörpers wird zum Zeitpunkt nach der Aufreinigung bestimmt, bevor der Antikörper auf einen Immunoassay im Sinne der vorliegenden Erfindung aufgebracht oder anderweitig zur Verfügung gestellt wird.

Besonders bevorzugt ist eine Ausführungsform, bei der durch die hohe Produktionsrate des erfindungsgemäßen rekombinanten Antikörpers dieser in einer hohen Konzentration von bevorzugt 20 - 1000 mg/L Kultur gewonnen wird. Dabei können Zelltrümmer und Kieselalgen-spezifische Proteine einfach abgetrennt werden, da störende Fasern nicht vorhanden sind. Dabei wird ein rekombinanter Antikörper in einer Reinheit von mindestens 90 %, bevorzugter von mindestens 95 % bereitgestellt. Durch die hohe Reinheit des Antikörpers, zusammen mit der hohen Homogenität und Gleichmäßigkeit des Glykosylierungsmusters kann ein Antigen spezifisch und in einer niedrigen Konzentration, also mit einer niedrigen Nachweisgrenze des Immunoassays detektiert werden, wodurch eine Verbesserung gegenüber den bisher üblichen Immunoassays auf Basis tierischer Antikörper erzielt wird. Durch die höhere Homogenität der Antikörper wird die Zahl der unspezifischen Kreuzreaktionen (unspezifische Signale) niedriger, wodurch das Ergebnis des Immunoassays im Sinne der vorliegenden Erfindung zuverlässiger ist, wobei die Sensitivität des Antikörpers gleichbleibend hoch ist.

Durch die höhere Reinheit der Antikörper werden diese stabiler und länger haltbar, weil mit den Fremdproteinen auch Antikörper-abbauende Proteasen entfernt werden. Weiterhin bewirken die Reinheit und Homogenität der Antikörper, dass die Zahl der unspezifischen Kreuzreaktionen (unspezifische Signale) niedriger ist, was zu einem zuverlässigeren und reproduzierbareren Ergebnis führt.

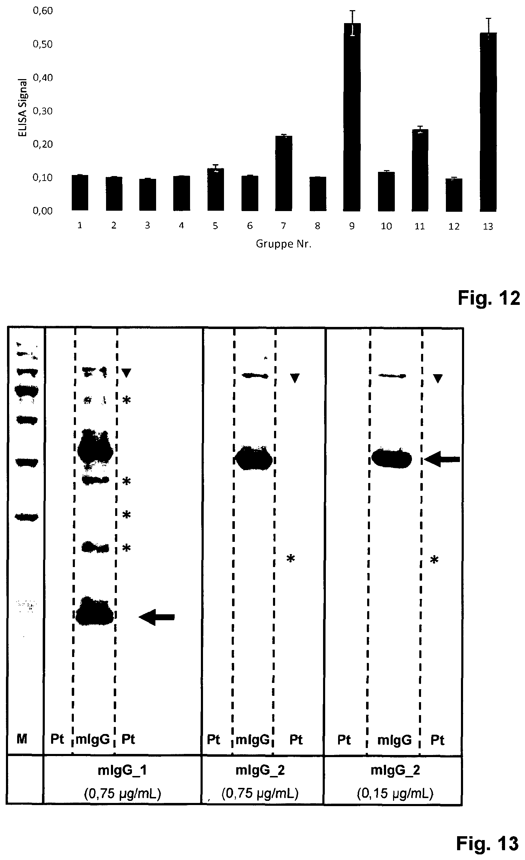

In einem besonders bevorzugten Ausführungsbeispiel, welches in Fig. 13 dargestellt ist, sind beim kommerziell erworbenen Antikörper deutlich die zusätzlichen Banden durch unspezifische Reaktivität zu erkennen. Diese fehlen auf den Gelbahnen des in Kieselalgen hergestellten Antikörpers, wodurch gezeigt wird, dass dieser eine höhere Spezifität als der kommerzielle, tierische Antiköper zeigt. Dies hat, neben der Reduzierung von falschpositiven Signalen, auch den Vorteil, dass eine geringere Menge Protein für den Immunoassay benötigt wird.

Ein "Immunoassay" ist ein Test, der die Bindung von Antikörpern an Antigene nutzt, um bestimmte Substanzen zu identifizieren und/oder die vorhandene Menge der Substanz zu quantifizieren. Immunoassays können zur Diagnose von Krankheiten, aber auch für die Analyse des physiologischen Zustands eines Individuums, bspw. menschlicher Individuen eingesetzt werden. Beispiele für Krankheitsdiagnosen sind die Erkennung verschiedener Krebskrankheiten, der Nachweis einer Infektion wie Covid19. Beispiele für die Analyse eines physiologischen Zustands sind die weibliche Empfängnis (Ovulationstests) oder Schwangerschaftstests. Immunoassay und Immunassay werden austauschbar verwendet.

Immunassays können in verschiedenen technischen Varianten eingesetzt werden. Bekannteste technische Varianten sind der Enzyme-linked Immunosorbent Assay (ELISA oder ELISA-Test) sowie der Lateral-Flow-Immunoassay (LFA), bei dem eine Membran, bspw. eine papierbasierte Plattform für den Nachweis und die Quantifizierung von Analyten in komplexen Gemischen dient, wobei die biologische Probe auf eine Testvorrichtung gegeben wird und die Ergebnisse innerhalb von 5-30 Minuten angezeigt werden. Bekannte Beispiele für Antikörper-basierte LFAs sind die Covid-19 Schnelltests oder Schwangerschaftstests.

Der Begriff "biologisch aktives Antigen" (auch als "Antigen" bezeichnet) bezeichnet im Sinne der vorliegenden Erfindung jede Substanz, insbesondere Moleküle ab einem Molekulargewicht von etwa 4.000 Dalton, bevorzugt von etwa 2.000 Dalton, am bevorzugtesten ab 770 Dalton, die den Körper dazu veranlasst, eine Immunreaktion gegen diese Substanz auszulösen. Zu den Antigenen gehören Toxine, Chemikalien, Bakterien, Viren, Proteine, Peptide oder andere Substanzen, die von außerhalb des Körpers stammen. Dabei werden bei der humoralen Immunreaktion von Wirbeltieren Antikörper produziert. Diese zur humoralen Immunantwort gebildeten Antikörper können auch unabhängig von einer Immunantwort und außerhalb des Körpers zum Erkennen, identifizieren und/oder Quantifizieren des Antigens dienen. Biologisch aktives Antigen und biologisch aktives Protein können im Sinne dieser Erfindung austauschbar verwendet werden.

Aus biologischen Gesichtspunkten versteht man unter biologisch aktiven Antigenen Moleküle, gegen die ein diese erkennender Antikörper in einem Individuum (wie hierin definiert) existiert. Insbesondere werden darunter Moleküle verstanden, die Proteine und/oder Hormone aus Menschen und Säugetieren darstellen, welche entweder als Marker für eine Erkrankung des Individuums (beispielsweise Diagnose von Brustkrebs über den Nachweis von Herceptin2-Rezeptor) oder als Marker für den physiologischen Zustand des Individuums fungieren (zum Beispiel Schwangerschaft). Alternativ bevorzugt werden darunter Moleküle verstanden, die Proteine und/oder Hormone in Tieren und/oder Pflanzen darstellen, insbesondere für Lebensmittelanalytik. Biologisch aktive Antigene, insbesondere Sequenzen von Hormonen, Proteinen oder Impfstoffen oder Arzneistoffen, ganz besonders bevorzugt Hormone, welche entweder als Marker für eine Erkrankung des Individuums (beispielsweise Diagnose von Brustkrebs über den Nachweis von Herceptin2Rezeptor) oder als Marker für den physiologischen Zustand des Individuums fungieren (zum Beispiel Schwangerschaft), sind dem Fachmann bekannt oder können einschlägigen Datenbanken und Fachbüchern entnommen werden.

Darüber hinaus werden als biologisch aktives Antigen Proteine von humanpathogenen Organismen insbesondere Bakterien (z. B. *Streptococcus mutans* (diese verursachen Karies) oder Viren (z.B. Spike Protein von Covid 19) verstanden.

Vorzugsweise ist das biologisch aktive Antigen ein Hormon eines Individuums, bspw. ein humanes Hormon ausgewählt aus der Gruppe umfassend Cortisol, Thyroxin, Somatotropin, Vasopressin, Testosteron, Östrogene. Entsprechende native Antikörper zum Nachweis der Hormone sind dem Fachmann bekannt oder können der einschlägigen Literatur entnommen werden.

Nach einer bevorzugten Ausgestaltung ist das biologisch aktive Antigen ein Peptidhormon eines Individuums, bspw. ein humanes Peptidhormon ausgewählt aus der Gruppe umfassend Gonadoliberin (10 Aminosäuren), Insulin (A-Kette: 21 Aminosäuren, B-Kette: 30 Aminosäuren), Somatostatin (14 Aminosäuren), Glucagon (29 Aminosäuren). Entsprechende native Antikörper zum Nachweis der Hormone sind dem Fachmann bekannt oder können der einschlägigen Literatur entnommen werden.

Nach einer bevorzugten Ausgestaltung ist das biologisch aktive Antigen ein Proteinhormon eines Individuums, bspw. ein humanes Proteinhormon, die beispielsweise als physiologische Marker dienen. Geeignete Proteinhormone sind bspw. ausgewählt aus der Gruppe umfassend Parathormon (84 Aminosäuren) HCG (Humanes Choriongonadotropin: $\alpha$-Untereinheit: 92 Aminosäuren, $\beta$-Untereinheit: 145 Aminosäuren). Entsprechende native Antikörper zum Nachweis der Hormone sind dem Fachmann bekannt oder können der einschlägigen Literatur entnommen werden.

Das biologisch aktive Antigen kann darüber hinaus ein Protein, insbesondere ein humanes Protein sein, das als Krankheitsmarker dient und ausgewählt ist aus der Gruppe umfassend Her2 (human epidermal growth receptor 2, welches auf Oberflächen von Krebszellen überexprimiert wird und einen Krebsmarker darstellt); IgE-Antikörper (welche aufgrund einer allergischen Reaktion vom menschlichen Körper produziert werden); kleine Signalproteine, wie Interleukin 5 (welches mit allergischen Erkrankungen in Verbindung gebracht wird, darunter allergische Rhinitis und Asthma); Interferone (die immunstimulierende, vor allem antivirale und antitumorale Wirkung besitzen und als Marker für virale Infektionen dienen können). Entsprechende native Antikörper zum Nachweis der Hormone sind dem Fachmann bekannt oder können der einschlägigen Literatur entnommen werden.

Das biologisch aktive Antigen kann ein Protein eines humanpathogenen Organismus sein, bspw. das Spikeprotein eines Coronavirus, wie des SARS-CoV-1 (zum Nachweis von SARS Infektionen) oder SARS-CoV-2 (zum Nachweis einer Covid-19 Infektion) oder das HIV-1 Nucleocapsid Protein (zum Nachweis einer HIV-Infektion).

Nach einer bevorzugten Ausgestaltung ist das biologisch aktive Antigen ein Hormon, ein Protein und/oder ein Arzneistoff. Besonders bevorzugt ist das biologisch aktive Antigen ein Hormon, insbesondere ein Hormon, welches als Marker für den physiologischen Zustand des Individuums fungiert (zum Beispiel Schwangerschaft), besonders bevorzugt das Choriongonadotropin, ganz besonders bevorzugt das humane Choriongonadotropin.

In einem Lateral-Flow-Immunoassay (hierin auch "LFA") finden typischerweise drei Antikörper Verwendung:

-    ein erster immobilisierter Antikörper, der im Fangbereich (auch als Testzone bezeichnet) angeordnet ist und auch als Fängerantikörper bezeichnet wird. Dieser ist wie der zweite Antikörper (auch als Detektionsantikörper bezeichnet) ebenfalls gegen das Antigen gerichtet, vorzugsweise gegen ein anderes Epitop des Antigens als der Detektionsantikörper;

-    ein zweiter Antikörper, der vorzugsweise im Konjugatbereich vorgesehen ist und auch als Detektionsantikörper bezeichnet wird, welcher gegen ein Epitop des Antigens gerichtet ist, z. B. ein Hormon, ein Protein oder ein Peptid, wie humanes Choriongonadotropin, wobei der zweite Antikörper vorzugsweise an Markerpartikel konjugiert ist, z. B. Gold-, Silber-, Latex-, Carbon-, Nanopartikel, Fluoreszenzfarbstoffe oder Enzyme.

- Ein weiterer Antikörper, der vorzugsweise in einem Kontrollbereich (Kontrollzone) angeordnet, vorzugsweise immobilisiert, ist und gegen einen Detektions- /Kontrollantikörper gerichtet ist.

Vorzugsweise werden der erste Antikörper (bspw. der Fängerantikörper), der zweite Antikörper (bspw. der Detektionsantikörper) und/oder der weitere Antikörper (bspw. der Fängerantikörper in der Kontrollzone), besonders bevorzugt alle Antikörper aus einer Kieselalge, einzelligen Pflanze oder Viridiplantae gewonnen und zeichnen sich durch die hierin definierten Charakteristika aus.

Besonders vorzugsweise werden der erste Antikörper (bspw. der Fängerantikörper), der zweite Antikörper (bspw. der Detektionsantikörper) und/oder der weitere Antikörper (bspw. der Fängerantikörper in der Kontrollzone), besonders bevorzugt alle Antikörper aus einer Kieselalge gewonnen und zeichnen sich durch die hierin definierten Charakteristika aus.

Ein Vorteil gegenüber der Verwendung tierischer Antikörper liegt zudem in der gleichbleibenden Qualität und der Reproduzierbarkeit der im Test verwendeten Antikörper und somit einer verbesserten Qualität des gesamten Immunoassays, da bspw. die antikörperproduzierenden Tiere nach einer gewissen Zeit sterben und Antikörper aus einem anderen Tier andere Eigenschaften aufweisen, d. h., die Qualität, der auf dem herkömmlichen Weg erzeugten Antikörper ist stark schwankend. Darüber hinaus handelt es sich bei Antikörpern und Hilfsproteinen, die aus einer Kieselalge, einzelligen Pflanze oder Viridiplantae gewonnen werden, im Gegensatz zu polyklonalen Antikörpern aus Individuen (wie hierin definiert) um exakt definierte Antikörper, d. h., deren Aminosäuresequenz vordefiniert und gleichbleibend ist.

Der Begriff "Individuum" (hier auch als "Proband" bezeichnet), wie dieser in der vorliegenden Erfindung verwendet wird, bezeichnet jedes Säugetier (z. B. Maus, Ratte, Kaninchen, Hund, Katze, Rind, Schwein, Schaf, Pferd oder Primat), insbesondere Nagetiere, Klauentiere, Huftiere, bevorzugt mit ungeraden Zehen, oder Primaten. In einer besonders bevorzugten Ausführungsform ist das Individuum ein Primat, insbesondere ein Mensch. Sofern nicht anders angegeben, bezeichnet der Begriff "Individuum" kein bestimmtes Alter und umfasst daher Erwachsene, ältere Individuen, Kinder und Neugeborene.

In einer bevorzugten Ausführungsform des Immunoassays ist das Individuum ein Säugetier, vorzugsweise ein Primat, noch bevorzugter ein Mensch.

Nach einer bevorzugten Ausgestaltung werden die rekombinanten Antikörper, bspw. der erste Antikörper und/oder der zweite Antikörper und/oder der weitere Antikörper, durch Expression aus der Kieselalge (auch Diatomeen), der Grünalge (auch Chlorobionta) oder einer Samenpflanze gewonnen. Besonders bevorzugt werden die rekombinanten Antikörper aus der Kieselalge oder der Grünalge, insbesondere in der Kieselalge, wie bspw. *Phaeodactylum tricornutum,* gewonnen. Ein Vorteil dabei ist, dass Antikörper aus Pflanzen, insbesondere aus Kieselalgen, keine endogenen Krankheitserreger enthalten können. Ein Beispiel hierfür ist der Rinderwahnsinn (BSE), weshalb tierische Antikörper, insbesondere für therapeutische Anwendungen, ein Risiko darstellen können. Deshalb müssen diese streng auf mögliche Humanpathogene überprüft werden, was bei den erfindungsgemäßen rekombinanten Antikörpern, besonders bevorzugt aus Kieselalgen gewonnenen, nicht notwendig ist.

Der Begriff "Antikörper" bezeichnet hierin ein Immunglobulin (Ig) oder ein Derivat eines Immunglobulins, wie es vom erworbenen Immunsystem von Wirbeltieren produziert wird. Beispiele für natürlich vorkommende Antikörper sind die Antikörper der Klasse M (IgM), G (IgG), A (IgA), und E (IgE), insbesondere aus Säugern wie Mensch, Kaninchen, Maus, Ratte, Kamel, Lama, Ziege und/oder Pferd. Weiterhin sind auch künstliche Formate, die auf solchen Proteinen beruhen eingeschlossen, Beispiele sind hier scFvs oder scFv-Fc.

In einigen, bevorzugten Ausführungsformen des Immunoassays bezeichnet "Antikörper" hierin ein Immunglobulin (Ig) oder ein Derivat eines Immunglobulins, wie es vom erworbenen Immunsystem von Wirbeltieren und/oder Knorpelfischen produziert wird. Beispiele für natürlich vorkommende Antikörper sind die Antikörper der Klasse M (IgM), D, (IgD) G (IgG), A (IgA), und E (IgE), NAR (IgNAR), insbesondere aus Säugern wie Mensch, Kaninchen, Maus, Ratte, Kamel, Lama, Ziege und/oder Pferd und/oder Knorpelfischen wie Haien. Weiterhin sind auch künstliche Formate, die auf solchen Proteinen beruhen eingeschlossen, Beispiele sind hier scFvs, scFv-Fc oder Einzeldomänenantikörper/Nanobodies.

Ein "nativer Antikörper" ist ein natürlicher Antikörper, wie dieser in einem Individuum, wie hierin definiert, insbesondere einem Vertebraten, besonders bevorzugt einem Säugetier, ganz besonders bevorzugt einem Primaten, insbesondere einem Menschen, vorzufinden ist.

Nach einer bevorzugten Ausgestaltung weist der Antikörper zumindest einen variablen Bereich und einen konstanten/konservierten Bereich auf.

Vorzugsweise ist der variable Bereich vertebralen, bevorzugt mammalen Ursprungs, besonders bevorzugt humanen, murinen, equinen, kaninen und/oder kamelartigen Ursprungs, ganz besonders bevorzugt humanen, murinen, equinen und/oder kamelartigen Ursprungs, insbesondere humanen, murinen und/oder equinen Ursprungs. Der variable Bereich kann in seiner Aminosäuresequenz zumindest 50 % Sequenzidentität zu homologen Sequenzbereichen eines vertebralen, bevorzugt mammalen (wie vorstehend definiert), vorzugsweise einem humanen, Antikörper aufweisen.

Nach einer bevorzugten Ausgestaltung ist der konstanten Bereich vertebralen, bevorzugt mammalen Ur-

**EP 4 587 833 B1**

sprungs, besonders bevorzugt humanen, kaninen, murinen, equinen, Ziegen- und/oder kamelartigen Ursprungs. Der konstanten Bereich kann in seiner Aminosäuresequenz zumindest 50% Sequenzidentität zu homologen Sequenzbereichen eines vertebralen, bevorzugt mammalen (wie vorstehend definiert), vorzugsweise einem humanen, Antikörper aufweisen.

Offenbart ist die Bereitstellung einer Nukleinsäuresequenz mit einer erhöhten Expressionsrate zur Produktion von rekombinanten Proteinen ausgestaltet, aufweisend zumindest eine Expressionskassette zur Expression eines oder mehrerer Peptide. Dabei weist die Expressionskassette erfindungsgemäß wenigstens ein Promotorelement und wenigstens eine erste Transkriptionseinheit, die für ein Protein codiert, auf, wobei das Promotorelement aus der Nukleinsäuresequenz von SEQ ID NO: 1 oder einer Nukleinsäuresequenz mit einer Homologie von zumindest 70 %, bevorzugt zumindest 80 %, besonders bevorzugt zumindest 90 %, ganz besonders bevorzugt zumindest 95 %, weiter bevorzugt zumindest 99 % zur SEQ ID NO: 1 besteht.

Der Begriff "Promotor" im Sinne der vorliegenden Erfindung bezeichnet eine Polynukleotidsequenz, die stromaufwärts eines Gens lokalisiert ist und die Transkription eines funktionsfähigen Gens reguliert. Der Promotor bildet eine Erkennungs- und Bindestelle für eine RNA-Polymerase, welche die Transkription des Gens einleitet.

Der Begriff "Homologie" bezeichnet im Sinne der Erfindung die Ähnlichkeit zwischen Nukleotid-Sequenzen der DNA bzw. RNA und/oder zwischen Aminosäuresequenzen von Proteinen.

In einer bevorzugten Ausführungsform stellt die SEQ ID NO:1 einen im Folgenden als HASP1$^{mod}$ bezeichneten Promotor dar, der sich als besonders geeignet für die regulierte Proteinproduktion herausgestellt hat. Dabei ist der HASP1$^{mod}$-Promotor ein von dem natürlichen HASP1-Promotor abgeleitetes Promotorelement, wobei eine Teilsequenz des natürlichen HASP1-Promotors verdoppelt wurden ist.

Die SEQ ID NO:1 lautet wie folgt, wobei der unterstrichene Anteil die Dopplung darstellt:

5'-
CATACAGTGAATGTAACTTTCGAATTGACAGTATTAGTAGTCGTATTGACAGTGAGGCAC
GCCCCTCAATGTGCGAGGTGGAAAATATACCAGCATGACAATGAATCTTGGAGATTCTT
TTGCTGTCATCAAGATTCACCGCCAAATCTTCAGGAACCTATCACGTCCACAGGCGATG
TTAATTCTTGAGTCGTCAAAACAAAGTCCTGTCCTACCTGTAGAAGTTGACAGCGAGCAA
TTGTATGCAAACTTCTGACTTTGTTATAATAACATTAAAGGTAATTAAGTATCTTCAATTAG
GCATTTTGTCACTGTCAGTCCGTTCCGACAATATAGGTAGATTTGGAATGAATCTTTTCT
ATGCTCATACAGTGAATGTAACTTTCGAATTGACAGTATTAGTAGTCGTATTGACAGTGA
GGCACGCCCCTCAATGTGCGAGGTGGAAAATATACCAGCATGACAATGAATCTTGGAGA
TTCTTTTGCTGTCATCAAGATTCACCGCCAAATCTTCAGGAACCTATCACGTCCACAGGC
GATGTTAATTCTTGAGTCGTCAAAACAAAGTCCTGTCCTACCTGTAGAAGTTGACAGCGA
GCAATTGTATGCAAACTTCTGACTTTGTTATAATAACATTAAAGGTAATTAAGTATCTTCA
ATTAGGCATTTTGTCACTGTCAGTCCGTTCCGACAATATAGGTAGATTTGGAATGAATCT
TTTCTATGCTGCTGCGAATCTTGTACACCTTTGAGGCCGTAGATTCTGTCCGACGAAGC
GATAATTATTGCAAAATACATGGACTCATTATTTTGATTCGATTTCTTTTTGGTATCCGAC
TCGAAAAGATCCATCACGGCGAGC-3'

Offenbart ist die Bereitstellung von Nukleinsäuresequenzen, deren Promotorelement einzelne HASP1-Sequenzabschnitte repetitiv aufweisen, beispielsweise bezogen auf das Startcodon ATG zwischen -100 und -1, zwischen -200 bis -101, zwischen -300 bis -201, zwischen -400 bis -301 und/oder zwischen - 500 bis -401. Dabei können diese Abschnitte in beliebiger Konstellation und Kopienzahl kombiniert werden. So ergibt sich eine neue Sequenz, die weniger als 85 % Homologie zum nativen HASP1 Promotor ergibt.

Nach einer bevorzugten Ausgestaltung umfasst wenigstens eine Transkriptionseinheit ein Polynukleotid, welches für eine Aminosäuresequenz von SEQ ID NO: 2 oder eine Aminosäuresequenz mit einer Homologie von zumindest 70 %, bevorzugt zumindest 80 %, besonders bevorzugt zumindest 90 %, ganz besonders bevorzugt zumindest 95 %, weiter bevorzugt zumindest 99 % zur SEQ ID NO:2 codiert.

Die SEQ ID NO:2 stellt dabei eine Aminosäuresequenz der hinge-Region eines Antikörpers dar, welche aus equinen Immunglobulin-Sequenzen abgeleitet wurde und sich als besonders Protease-resistent herausgestellt hat. Die Verwendung dieser Protease-resistenten Hinge-Region reduziert maßgeblich die Proteolyse der hergestellten Antikörper verschiedener Formate und aus verschiedenen Spezies, sowohl *in vivo* als auch *in vitro*.

Die SEQ ID NO:2 lautet wie folgt:

VIKEPCCCPKCP

In einer bevorzugten Ausführungsform kann ein Vektor oder eine isolierte Nukleinsäure bereitgestellt werden, welche/r eine erfindungsgemäße Nukleinsäure in einfacher oder repetitiver Form umfasst, sowie die Bereitstellung eine Zelle umfassend einen erfindungsgemäßen Vektor oder eine erfindungsgemäße isolierte Nukleinsäure oder eine erfindungsgemäße Nukleinsäuresequenz oder eine erfindungsgemäße Aminosäuresequenz.

Weiterhin wird eine Aminosäure umfassend SEQ ID NO: 2 oder eine Aminosäuresequenz mit einer Homologie von zumindest 70 %, bevorzugt zumindest 80 %, besonders bevorzugt zumindest 90 %, ganz besonders bevorzugt zumindest 95 %, weiter bevorzugt zumindest 99 % zur SEQ ID NO: 2 bereitgestellt.

In der vorliegenden Schrift ist die Zelle dabei eine photosynthetisch aktive Zelle, insbesondere eine einzellige Pflanze, bevorzugt eine Kieselalge.

Vorzugsweise weist der rekombinante Antikörper im Bereich der Gelenkregion eine Cysteinreiche Aminosäuresequenz aus zumindest 20 Aminosäuren, vorzugsweise zumindest 15 Aminosäuren, besonders bevorzugt zumindest 12 Aminosäuren auf, wobei zumindest 33 % der Aminosäuren Cystein umfassen. Nach einer besonders bevorzugten Ausführungsform umfasst oder besteht die Aminosäuresequenz in der Gelenkregion aus (a) der Sequenz VIKEPCCCPKCP oder (b) einer um maximal 30 %, insbesondere um maximal 20 %, besonders bevorzugt um maximal 15 % von dieser Aminosäuresequenz abweichenden Sequenzidentität oder (c) einer Aminosäuresequenz, die gegenüber der Variante gemäß (a) lediglich einen Aminosäureaustausch aufweist.

In einer besonders bevorzugten Ausführungsform beträgt die Produktionsrate von 20 mg/L bis 1000 mg/L, besonders bevorzugt von 30 mg/L bis 1000 mg/L Antikörpern pro Liter Kultur. Diese hohe Produktionsrate ist für eine technische Verwertung der Antikörper zwingend erforderlich, da eine Abtrennung und Aufreinigung bei Werten unter 20 mg/L technisch nicht praktikabel und damit auch für die wirtschaftliche Anwendbarkeit bei einem vertretbaren Aufreinigungsaufwand zwingend erforderlich ist. Diese Werte liegen in dem für kommerziell genutzte, tierische CHO-Zellkulturen (*Chinese Hamster Ovary*) typischen Bereich und damit weit über der bisher erreichten Kapazität für Kiesel-, Mikroalgen, einzelligen Pflanzen und/oder Viridiplantae. Die Erkenntnis, dass Kieselalgen oder andere Mikroalgen mit ihren Produktionskapazitäten zu echten Konkurrenten von CHO-Zellen werden könnten, war bisher nicht absehbar.

Besonders bevorzugt ist eine Ausführungsform, in der rekombinante Antikörper in einer Konzentration von 20 mg/L bis 1000 mg/L, bevorzugter 30 mg/L bis 800 mg/L, alternativ mindestens 30 mg/L bis 160 mg/L Kultur einer Kieselalge oder einzelligen Pflanze gewonnen werden. Diese hohe Produktionsrate ist Ergebnis der Entdeckung der Erfinder, dass verschiedene Modifikationen an einer Kieselalge zu einer unerwartet hohen Steigerung, beispielsweise um einen Faktor von mindestens über 100 im Vergleich zum Stand der Technik führen können. Dies erlaubt die technische Ausführbarkeit der Gewinnung und Reinigung von Antikörpern aus Kieselalgen, die eine hohe Reinheit sowie Homogenität aufweisen. Die Produktionsrate in der Kieselalge konnte von herkömmlicherweise maximal 3 mg Antikörper pro Liter Kultur auf beispielsweise mindestens 160 mg Antikörper pro Liter Kultur erhöht werden. Besonders bevorzugt kann die Produktionsrate in der Kieselalge von einem herkömmlichen Höchstwert von 3 mg Antikörper pro Liter Kultur auf 300 mg Antikörper pro Liter Kultur erhöht werden, was einem Faktor von 100 entspricht. In einigen sehr bevorzugten Ausführungsformen kann die Produktionsrate auf bis zu 1000 mg/L Kultur gesteigert werden, was einem Faktor von 333 entspricht.

Eine Kultur umfasst die Kieselalgen, Kulturmedium und alle weiteren Zusätze, die für die Bereitstellung der rekombinanten Antikörper notwendig sind.

Ein Kulturmedium umfasst eine Flüssigkeit vorzugsweise eine wässrige, salzhaltige Flüssigkeit, die geeignete Nährstoffe, Temperaturen, pH-Werte und andere Bedingungen bietet, die das Wachstum und die Vermehrung von Zellen, besonders bevorzugt von Mikroalgen, noch bevorzugter von Kieselalgen, fördern.

In einigen Ausführungen wird der rekombinante Antikörper mindestens in einer Konzentration von 100 mg/L Kultur bereitgestellt, bevorzugter von mindestens 250 mg/L; am meisten bevorzugt von mindestens 500 mg/L. Dabei werden die bisher berichteten Werte um einen Faktor von mindestens 33, bevorzugter von mindestens 83, am meisten bevorzugt von Faktor 166 übertroffen.

Nach einer bevorzugten Ausführungsform des Immunoassays weist die Glykosylierung des ersten Antikörpers und/oder des zweiten Antikörpers ein modifiziertes Glykosylierungsmuster im Vergleich zum entsprechenden nativen Antikörper auf, vorzugsweise weist die Glykosylierung ein homogeneres Muster mit einem Homogenitätsfaktor im Bereich von 1 bis 3 auf, besonders bevorzugt weist die Glykosylierung ein homogenes, mannosereiches N-Glykanmuster mit einem Homogenitätsfaktor im Bereich vorzugsweise im Bereich von 1 bis 3 auf.

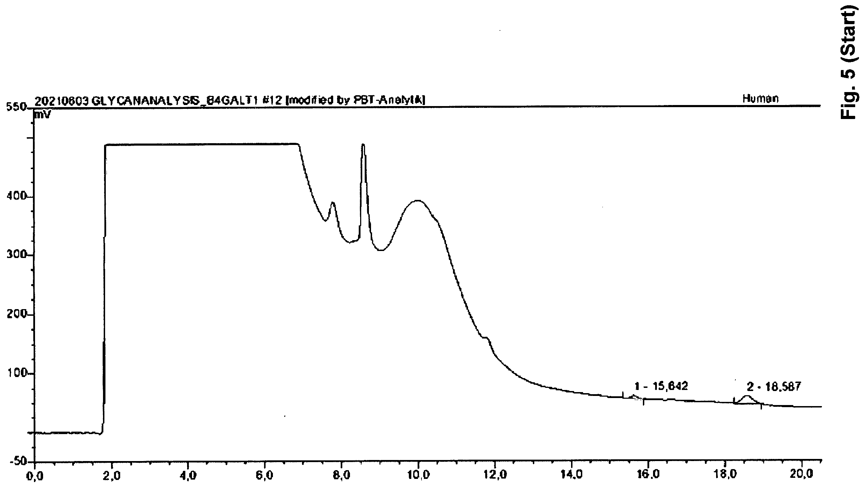

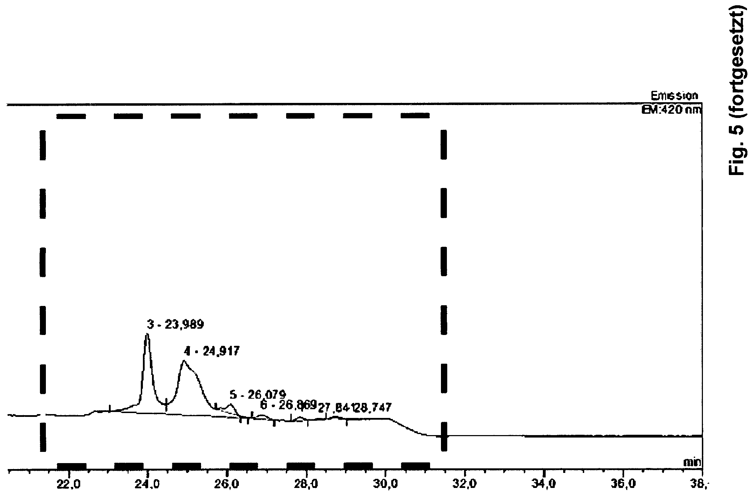

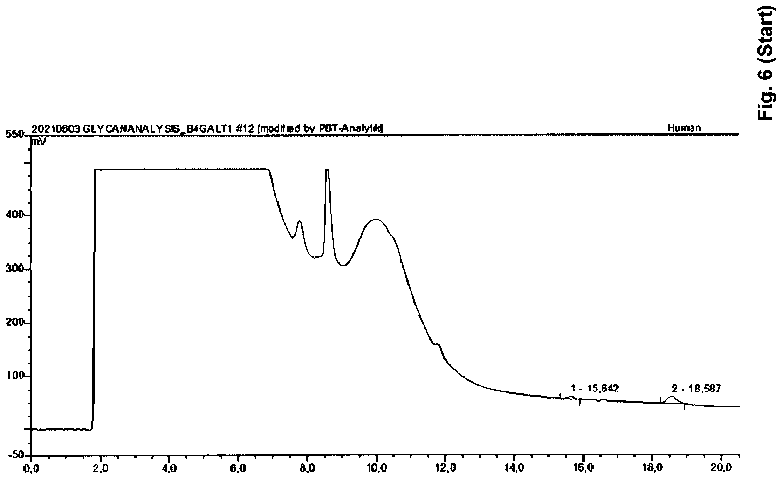

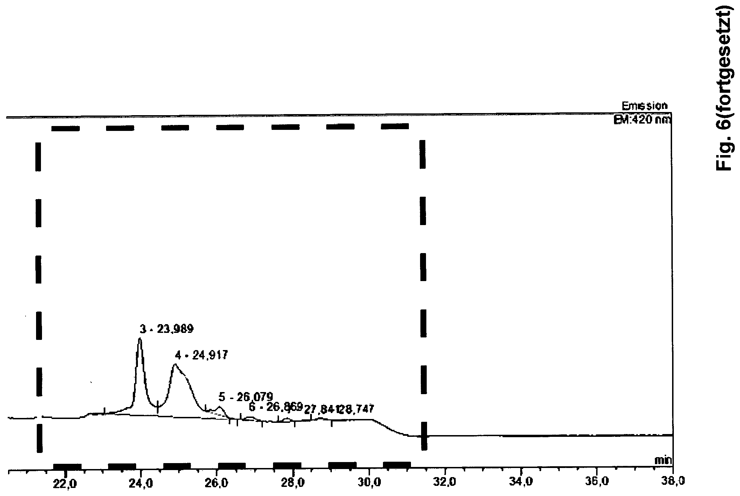

Unter dem Homogenitätsfaktor versteht man das Verhältnis der Anzahl der Baseline-getrennten, definierten Peaks im Chromatogramm, bestimmt durch HPLC (High-Performance Liquid Chromatography) und/oder UPLC (Ultra-Performance Liquid Chromatography) gekoppelt mit MS und/oder HRMS und/oder UV/Vis und/oder Diodenarray, zwischen einem Antikörper im Sinne der vorliegenden Verbindung, vorzugsweise einem aus einer Kieselalge exprimierten Antikörper, und dem entsprechenden nativen Antikörper und/oder einem tierischen Antikörper. Ein Homogenitätsfaktor von 1 bedeutet, dass 1 Peak weniger vorhanden ist, von 2, dass 2 Peaks weniger vorhanden sind und so weiter. In einer besonders bevorzugten Ausführungsform (Fig. 5-7) wird ein Homogenitätsfaktor von 3 erreicht, die Vergleichsantikörper, welche aus einer humanen Zellkultur stammen (Fig. 5 und 6) haben jeweils 6 Peaks, während der rekombinante Antikörper im Sinne der vorliegenden Erfindung nur 3 Peaks aufweist.

In einer bevorzugten Ausführungsform des Immunoassay weist die Glykosylierung des ersten Antikörpers und/oder des zweiten Antikörpers eine erhöhte Homogenität des Glykosylierungsmusters im Vergleich zum entsprechenden nativen Antikörper auf, vorzugsweise ein homogenes, mannosereiches N-Glykanmuster, ohne zusätzliche Zugabe von Mannose zum Kulturmedium zu erfordern.

Die Unterschiede der Glykosylierungsmuster von Antikörpern zu den Systemen von Säugetieren ergeben sich aus dem Vorhandensein verschiedener "mannosereicher" N-Glykane (von Mannose-5 bis Mannose-9). Im Gegensatz zu natürlichen Säugetierzellen, die einen relativ niedrigen Mannosegehalt haben, weisen kommerziell verwendete Zellkulturen wie CHO-Zellen aufgrund der absichtlichen Zugabe von Mannose jedoch einen höheren Mannosegehalt auf. CHO basierte Antikörper weisen oftmals eine deutlich heterogene Verteilung der Glykosylierung auf. In Fig. 5-7 ist eine deutliche Differenzierung zu erkennen. Die Konsistenz der Glykosylierung ist in CHO-, Expi- und anderen tierischen oder humanen Zellen, bzw. Zellkulturen oft nicht gegeben (erkennbar an den zahlreichen Peaks im umrahmten Bereich von Abbildung 5).

Diese Inkonsistenz beeinflusst die Spezifität und Stabilität der Antikörper. Daher wird dem Medium tierischer oder humaner Zellkulturen häufig Mannose zugesetzt, um eine größere Einheitlichkeit zu erreichen (trotz der damit verbundenen Nachteile). Diatomeen weisen von Natur aus einen hohen Mannosegehalt auf. Darüber hinaus weisen unsere Antikörper eine bemerkenswerte Homogenität auf, d. h. Antikörper, die von verschiedenen Zellen in einer Kultur stammen, weisen einheitliche Glykosylierungsmuster auf (Abbildung 5). Dadurch wird die Wahrscheinlichkeit unerwünschter Eigenschaften wie die Erkennung von Fremdproteinen und Schwankungen in der Antikörperstabilität erheblich verringert.

Rekombinante Antikörper werden heute entweder in Tieren produziert oder mittels humaner oder tierbasierten Expressionssystemen, also eukaryotischen Zelllinien hergestellt. Dabei werden vorzugsweise Säugerzellsysteme vor allem dann eingesetzt, wenn es sich wie bei Antikörpern um komplexe Proteine handelt, bei denen aufwendige posttranslationale Modifikationen für die analytische oder therapeutische Wirksamkeit essentiell sind. Zu diesen posttranslationalen Modifikationen gehören die Glykosylierungsmuster von Antikörpern. Ein großes Problem derzeit hergestellter rekombinanter Proteine ist, dass uneinheitliche Glykosylierungsmuster vorliegen. So werden rekombinante Antikörper aus derzeit eingesetzten Expressionssystem, insbesondere auch aus Individuen häufig hyperglykosyliert, d. h., es werden bspw. verstärkt Mannose-Reste eingefügt, die häufig zusätzlich noch mit ungewöhnlichen Verzweigungen versehen sind. Diese können "abbrechen" (degradieren) und/oder dies kann dazu führen, dass die Proteine nicht wirksam sind, oder dass unerwünschte Nebenreaktionen durch das Immunsystem auftreten.

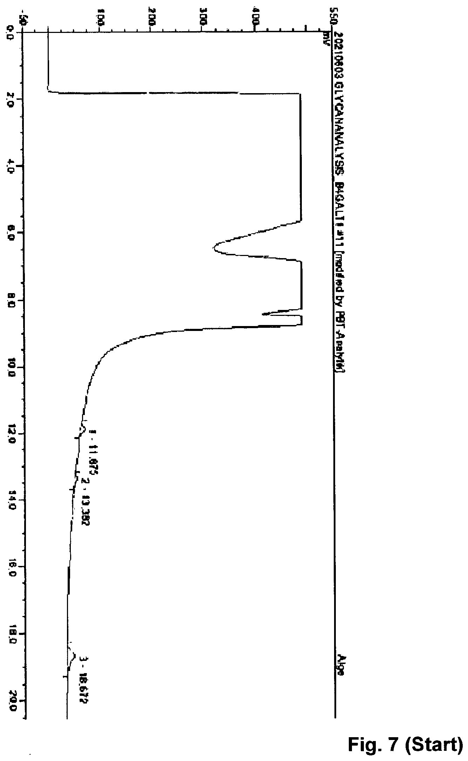

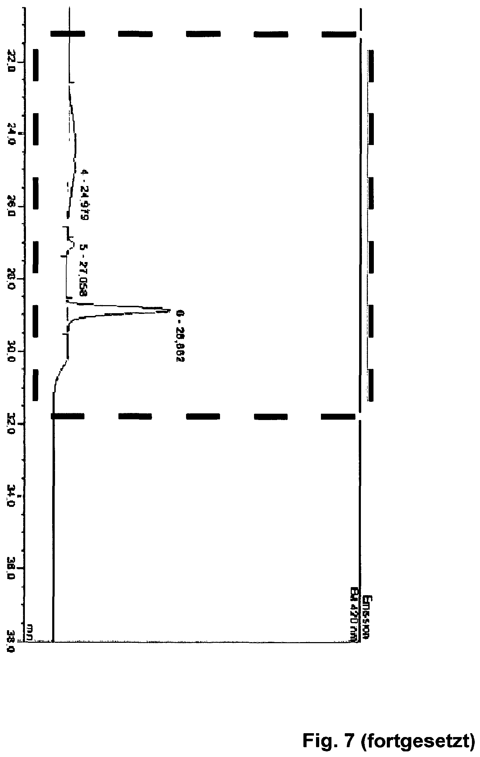

Nach einer bevorzugten Ausgestaltung unterscheidet sich die Glykosylierung (auch als Glykosylierungsmuster bezeichnet) des Antikörpers (wie hierin definiert), bspw. des ersten Antikörpers und/oder des zweiten Antikörpers und/oder jedes weiteren Antikörpers, gegenüber dem entsprechenden nativen Antikörper, wie dieser in dem Individuum vorkommt. Besonders bevorzugt ist das Glykosylierungsmuster der rekombinanten Antikörper (wie hierin definiert) homogener als das Glykosylierungsmuster des nativen Antikörpers, wie es in einem Individuum (wie hierin definiert) exprimiert wurde (vgl. bspw. Fig. 5 und 6 gegenüber Fig. 7). So weist das Glykosylierungsmuster der rekombinanten Antikörper (wie hierin definiert) gegenüber dem nativen Antikörper bspw. weniger Verzweigungen auf, die wie oben genannt "abbrechen" (degradieren) können und/oder (in Zusammenwirken) dazu führen können, dass die Antikörper nicht wirksam sind, oder dass unerwünschte Nebenreaktionen durch das Immunsystem auftreten. Ein hierin offenbarter rekombinanter Antikörper (also ein Antikörper, der aus Kieselalgen, einzelligen Pflanzen oder Viridiplantaen gewonnen wurde) stellt somit ein sog. Biosimilar da. Vorzugsweise werden derartige Antikörper, deren Glykosylierungsmuster gegenüber dem entsprechenden nativen Antikörper abweichen, als Marker für eine Erkrankung des Individuums oder als Marker für den physiologischen Zustand des Individuums präferiert.

In einer bevorzugten Ausführungsform des Immunoassays ist der rekombinante Antikörper ein Mosaik-Antikörper, wobei der Mosaik-Antikörper zumindest eine erste Sequenz umfasst, die zumindest aus einem ersten Organismus ausgewählt ist, und zumindest eine zweite Sequenz umfasst, die zumindest aus einem zweiten Organismus ausgewählt ist. Der erste und der zweite Organismus sind verschiedene Organismen. Die erste Sequenz kann die schwere Kette oder ein Teil der schweren Kette sein, die zweite Sequenz kann die leichte Kette oder ein Teil der leichten Kette sein. Die Hinge Region als ein definierter Abschnitt der Sequenz der schweren Kette kann entweder aus dem gleichen Organismus wie die restliche Sequenz der schweren Kette oder bevorzugt aus einem anderen Organismus stammen.

In einer alternativen Ausführungsform kann die erste Sequenz aus einem ersten und einem zweiten Organismus ausgewählt sein. In einer weiteren alternativen Ausführungsform kann die zweite Sequenz aus einem ersten und einem zweiten Organismus ausgewählt sein.

Die Bereitstellung von Mosaik-Antikörpern führt zu neuartigen und nicht natürlich vorkommenden Antikörpern. Diese Antikörper werden mit Hilfe von in-silico-Prozessen sorgfältig entworfen, was zu Sequenzen führt, die in der Natur nicht vorkommen. Prozessoptimierungen haben zur Schaffung von Antikörperregionen geführt, die Sequenzübereinstimmungen mit Datenbanksequenzen aus einer Reihe von tierischen und menschlichen Quellen aufweisen. Folglich handelt es sich bei den hergestellten Antikörpern um Mosaik-Antikörper oder auch Mosaik-Proteine, die genetische Sequenzen aus verschiedenen Arten enthalten.

Die offenbarten Methoden stellen eine Abweichung von den herkömmlichen Methoden zur Herstellung chimärer Antikörper dar. Zwar wurden chimäre Antikörper bereits in tierischen Zellkulturen hergestellt, doch erfordert dieser Ansatz die Verwendung transgener Tiere. Im Gegensatz dazu ermöglicht die erwähnte Methode die Produktion von chimären Antikörpern in Kieselalgen, so dass keine transgenen Organismen, insbesondere keine transgenen Tiere, erforderlich sind.

Die mit dieser Methode synthetisierten Antikörper weisen beispielsweise eine Grundstruktur auf, die der Sequenz des menschlichen Immunglobulins IgG4 entspricht.

In einer bevorzugten Ausführungsform kann dieses Mosaik-Protein folgenden skizzierten Aufbau aufweisen, bestehend aus einer schweren Kette, leichten Kette und Hinge-Region:

a) Schwere Kette (beispielhafter Aufbau):

- $C_H1$ - $C_H3$ - ohne Hinge-Region zu mindestens 80 % homolog zu humanen IgG4; beispielsweise umfasst die $C_H1$ ohne Hinge 116 - 118 Aminosäuren, $C_H2$ und $C_H3$ zusammen 215 - 220 Aminosäuren, wobei die Hinge-Region in der Regel 10 - 14 Aminosäuren umfasst, die 80 % Homologie bezieht sich auf die minimal 331 und maximal 338 Aminosäuren von $C_H1$, $C_H2$ und $C_H3$, was bei 80 % eine Zahl von 264 - 270 Aminosäuren ergibt.
- Hinge-Region entspricht der geschützten Sequenz zu mindestens 80 %, weitere Vorlagen für die Hinge-Region, die besonders widerstandsfähig gegenüber proteolytischen Abbau ist, und vorzugsweise aus der Ordnung der *Perissodactyla,* insbesondere bevorzugt der *Equidae* stammt.
- Variable Kette ist homolog zu den IgG-Sequenzen sehr unterschiedlicher Organismen, vorzugsweise ausgewählt aus der Liste bestehend aus Mensch, Maus und Kaninchen.

b) Leichte Kette (Beispielhafter Aufbau):

- Die konstante Region der leichten Kette ist homolog zu solchen aus der Ordnung der *Perissodactyla,* insbesondere bevorzugt der *Equidae.*
- Variable Kette ist homolog zu den Leichte Kette IgG-Sequenzen sehr unterschiedlicher Organismen, vorzugsweise ausgewählt aus der Liste bestehend aus Mensch, Maus, Kaninchen, besonders bevorzugt jedoch zu variablen Ketten von humanen Leichten Ketten des Kappa-Typs. Auch hier wurde festgestellt, dass diese einen Einfluss auf die Produktionsmengen und Proteaseresistenz haben.

Vorzugsweise führen die unterschiedlichen Kombinationen je nach verwendeter Sequenz zu einer gesteigerten oder erniedrigten Produktionsrate des Zielproteins bzw. Antikörpers.

Ein wichtiger Aspekt ist die Variabilität der Domänenkombinationen innerhalb der Sequenzen. Je nach den gewählten Sequenzanordnungen werden unterschiedliche Produktionsraten für das Zielprotein erzielt. Diese Flexibilität ermöglicht es, die Proteinproduktion auf das gewünschte Niveau einzustellen.

Zusammenfassend lässt sich sagen, dass diese Offenlegung ein Ansatz zur Herstellung von Antikörpern mit Hilfe von Kieselalgen vorstellt, der nicht natürlich vorkommende Mosaik-Antikörper und/oder Mosaikproteine mit Sequenzen aus mehreren Arten hervorbringt. Diese Innovation umgeht die Abhängigkeit von transgenen Tieren und bietet Vorteile wie anpassbare Proteindesigns, verbesserte Produktionskontrolle und potenzielle Anwendungen in verschiedenen Bereichen, einschließlich Diagnostik und Therapeutik. Insbesondere Eigenschaften wie Stabilität, Selektivität und Produktionsrate können durch das gezielte Design dieser Antikörper gezielt optimiert und kontrolliert werden.

In einer bevorzugten Ausführungsform weist der Immunoassay zumindest einen weiteren Antikörper und/oder ein Hilfsprotein auf, wobei vorzugsweise der weitere Antikörper und/oder das Hilfsprotein einer Kieselalge und/oder einzelligen Pflanze und/oder Viridiplantae gewonnen wird und besonders bevorzugt vegan ist.

Vegan im Sinne der vorliegenden Erfindung, explizit ein veganes Immunoassay im Sinne der vorliegenden Erfindung, ist ein Assay, das mit biotechnologischen Verfahren und Methoden unter Einhaltung der Grundsätze des Veganismus hergestellt wird. Das bedeutet, dass diese Produkte ohne den Einsatz von tierischen Materialien und/oder tierischer Zellkulturen oder Nebenprodukten entwickelt werden und dass bei ihrer Herstellung keine Tierversuche durchgeführt werden.

Der Immunoassay kann neben dem ersten und/oder zweiten Antikörper auch einen weiteren Antikörper und/oder ein weiteres Hilfsprotein aufweisen. Hilfsproteine dienen bspw. als Blocker, die freie Oberflächen (d.h. Bereiche, wo Antikörper nicht immobilisiert sind und unspezifische Bindungen erfolgen können) eines Reaktionsgefäßes oder einer Membran abzusättigen. Beispielsweise ist das Hilfsprotein das Bovine Serumalbumin (BSA), ein Casein, ein modifiziertes BSA oder ein modifiziertes Casein, welches als Blocker fungiert, bspw. um freie Oberflächen abzusättigen.

Hilfsproteine (wie hierin definiert) können mehrere Funktionen übernehmen:

- Absättigen der Membranen: die Membran (bspw. die Nitrozellulosemembran) bindet mit hoher Effizienz Proteine jeglicher Herkunft. Daher können Antikörper in einem Immunoassay, bspw. einem LFA fest an die Membran gebunden werden. Zwischen den Bereichen, in denen Antikörper die Membran absättigen, gibt es aber immer noch Membranregionen, wo kein Protein gebunden ist. Proteine aus der zu testenden Probe würden daran binden und so zu falschen Ergebnissen führen. Ist das Antigen immobilisiert (z. B. beim ELISA), würden die testenden Antikörper spezifisch an das Antigen binden, aber auch unspezifisch an die Oberfläche. Eine Auswertung wäre nicht möglich. Diese Proteine blockieren also die proteinbindenden Oberflächen, so dass Antikörper nur noch spezifisch an ihre Antigene binden können und nehmen daher eine zentrale Fuknktion bei Immunoassays ein.

- Hilfreich sind diese Hilfsproteine auch während der Inkubation von Antikörpern und Antigen. Der Überschuss an Hilfsprotein, bspw. BSA und/oder Casein aus der Milch sorgt dafür, dass in der Probe enthaltene Proteasen, die Antigen oder Antikörper abbauen könnten, auch BSA oder Casein als Substrat für die Proteolyse angeboten werden. Da Casein / BSA im Überschuss in der Lösung sind, ist die Gefahr, dass zufällig ein Antikörper proteolytisch geschädigt wird, (statistisch) geringer je mehr BSA in der Lösung ist.

Gemäß einer besonders bevorzugten Ausführungsform kann der Immunoassay neben dem rekombinanten Antikörper auch einen weiteren Antikörper und/oder ein oder mehrere weitere Hilfsproteine aufweisen. Im Allgemeinen dienen Hilfsproteine bspw. der Absättigung (Blockieren) freier Bindungsstellen auf der Matrix (d. h., Bereiche, wo noch keine Proteine aus der zu analysierenden Probe immobilisiert sind). Da die Matrices generell Proteine binden und Antikörper Proteine sind, würden an solchen freien Bereichen Antikörper unspezifisch gebunden werden und könnten nicht mehr an ihre immobilisierten Antigene binden. Daher werden bei allen Immunoassays solche Bereiche eines Reaktionsgefäßes, Mikrotiterplatte oder einer Membran mit einem oder mehreren Hilfsproteinen abgesättigt. Oft, insbesondere im Stand der Technik, sind solche Hilfsproteine tierischen Ursprungs, wie das Bovine Serumalbumin (BSA), Kaseine, welche als Blockierungsagenz freie Oberflächen auf der Matrix absättigen. Kasein(e) und Casein(e) werden austauschbar verwendet.

In einer besonders bevorzugten Ausführungsform können Hilfsproteine (wie hierin definiert) mehrere Funktionen übernehmen:

- Absättigen der Membranen: die Membran (bspw. die Nitrozellulosemembran) bindet mit hoher Effizienz Proteine jeglicher Herkunft. Daher können zu analysierende Proteine in einem Immunoassay, bspw. Antikörper in einem LFA fest an die Membran gebunden werden. Zwischen den Bereichen, in denen Proteine an die Membran gebunden haben, gibt es aber immer noch Membranregionen, wo kein Protein gebunden ist. Bei einem Immunoassay wie ELISA oder Immunoblot würden die Antikörper daran gebunden werden und könnten nicht mehr ihre Antigene detektieren. Bei LFAs würden Proteine aus der zu testenden Probe von diesen freien Membranregionen gebunden werden und so zu falschen Ergebnissen führen. Diese Hilfsproteine blockieren also die proteinbindenden Oberflächen, so dass Antikörper nur noch spezifisch an ihre Antigene binden können und nehmen daher eine zentrale Funktion bei Immunoassays ein.

- Hilfreich sind diese Hilfsproteine auch während der Inkubation von Antikörpern und Antigen. Der Überschuss an Hilfsprotein, bspw. BSA und/oder Kasein aus der Milch sorgt dafür, dass in der Probe enthaltene Proteasen, die Antigen oder Antikörper abbauen könnten, auch BSA oder Kasein als Substrat für die Proteolyse angeboten werden. Da Kasein / BSA im Überschuss in der Lösung sind, ist die Gefahr, dass zufällig ein Antikörper proteolytisch geschädigt wird, (statistisch) geringer je mehr BSA in der Lösung ist.

Herkömmlich gewonnenes BSA und auch Kasein sind tierischen Ursprungs. Es ist zwar initial preiswert erhältlich, allerdings erfordert die Aufreinigung des BSA recht großen technischen Aufwand. Insbesondere die Entfernung von (human)-pathogenen Viren und Prionen (z. B. BSE, "Rinderwahnsinn") erfordert großen Aufwand. Andere Quellen, wie Bakterien oder Hefen können BSA nicht heterolog produzieren, weil es nicht nur glykosyliert ist, sondern auch mehrere post-translational modifizierte Aminosäuren enthält. Die hierin offenbarte Gewinnung von veganem BSA aus Kieselalge, einzelligen Pflanze oder Viridiplantae, insbesondere aus Kieselalgen hat daher den Vorteil, dass die Aufreinigung und Entfernung von (human)-pathogenen Viren und Prionen nicht erforderlich sind.

Die weiteren Antikörper oder Hilfsstoffe sind vorzugsweise rekombinante Antikörper bzw. rekombinante Proteine, die (ebenfalls, wie hierin definiert) aus einer Kieselalge, einzelligen Pflanze oder Viridiplantae gewonnen werden und daher ein heterologes kieselalgen-, pflanzen- oder mikroalgenspezifisches Signalpeptid und/oder ein von dem eines nativen Antikörpers, der aus einem Individuum (wie hierin definiert) gewonnen wurde, abweichendes Glykosylierungsmuster aufweisen.

In einer besonders bevorzugten Ausführungsform sind weitere Antikörper oder Hilfsstoffe rekombinante Antikörper bzw. rekombinante Proteine, die (ebenfalls, wie hierin definiert) aus einer Kieselalge oder einzelligen Pflanze gewonnen werden und daher ein heterologes kieselalgen-, pflanzen- oder mikroalgenspezifisches Signalpeptid und/oder ein von dem eines nativen Antikörpers, der aus einem Individuum (wie hierin definiert) gewonnen wurde, abweichendes Glykosylierungsmuster aufweisen.

In einer bevorzugten Ausführungsform des Immunoassays hat die Aminosäuresequenz des ersten Antikörpers und/oder des zweiten Antikörpers einen vertebralen, vorzugsweise einen mammalen, besonders bevorzugt einen humanen Antikörper; oder sie hat eine Aminosäuresequenz oder besteht aus einer Aminosäuresequenz, die mindestens 80 %, vorzugsweise mindestens 85 %, besonders bevorzugt mindestens 90 %, am meisten bevorzugt mindestens 95 %, insbesondere mindestens 97 % Sequenzidentität mit homologen Sequenzbereichen eines vertebralen und/oder mammalen und/oder humanen Antikörpers aufweist. Dadurch kann Kompatibilität mit einer Reihe von angezielten Antigenen gewährleistet werden und eine hohe Qualität der Antikörper erreicht werden.

In einer bevorzugten Ausführungsform ist die Nukleotidsäuresequenz, die für den ersten Antikörper und/oder den zweiten Antikörper kodiert, für den Wirtsorganismus, von dem der erste Antikörper und/oder der zweite Antikörper abgeleitet ist, codonoptimiert, bevorzugt für *Phaeodactylum tricornutum* codonoptimiert.

Bei der Durchführung der Codonoptimierung wird gleichzeitig auch die Basensequenz angepasst, um beispielsweise die Klonierung der rekombinanten DNA zu erleichtern. Ein Aspekt hierbei ist die Entfernung nicht benötigter bzw. nicht erwünschter Erkennungsstellen von Restriktionsenzymen in der rekombinanten DNA.

Offenbart aber nicht beansprucht ist auch eine Nukleinsäure, die für einen ersten Antikörper, einen zweiten Antikörper, einen weiteren Antikörper und/oder ein Hilfsprotein codiert, wobei die Sequenz der Nukleinsäure für die Expression in einer Kieselalge, einzelligen Pflanze oder Viridiplantae, insbesondere in einer Kieselalge codonoptimiert ist.

Offenbart aber nicht beansprucht ist eine Nukleinsäure, die für einen ersten Antikörper, einen zweiten Antikörper, einen weiteren Antikörper und/oder ein Hilfsprotein codiert, wobei die Sequenz der Nukleinsäure für die Expression in einer Kieselalge oder einzelligen Pflanze, insbesondere in einer Kieselalge codonoptimiert ist.

Die Erfinder haben zudem ein Verfahren entwickelt, welches für die Codonoptimierung der Sequenz nicht - wie üblich - jedes Codon für sich berücksichtigt. Vielmehr wird mittels einer positionsspezifischen Matrix ein Profil der Codonhäufigkeit über die gesamte Ursprungssequenz erstellt und mit diesem Profil diese Codonhäufigkeit an jeder Position auf die für Kieselalgen codonoptimierte Sequenz übertragen. Hierdurch wird insbesondere die Faltung der Antikörperketten direkt im Anschluss an die Translation optimiert, da schwer faltbare Bereiche etwas langsamer translatiert werden als leicht faltbare Bereiche der Antikörperketten. Das wirkt sich direkt auf die Menge an produzierten Antikörpern aus und ist ebenfalls für die hohe Homogenität der in Kieselalgen hergestellten Antikörper relevant.

Zudem hat eine für die Expression in einem Wirtsorganismus (wie hierin definiert) codonoptimierte Sequenz einer Nukleinsäure den Vorteil, dass die Faltung des Antikörpers entsprechen dem nativen Pendant des Antikörpers verbessert ist, was zu einer erhöhten Stabilität des Antikörpers und einer erhöhten biologischen Aktivität des in dem Wirtsorganismus (wie hierin definiert) exprimierten Antikörpers führt.

Darüber hinaus hat eine codonoptimierte Sequenz einer Nukleinsäure den Vorteil, dass die Expressionsrate in dem Wirtsorganismus gegenüber einer nicht codonoptimierten Sequenz einer Nukleinsäure zumindest um den Faktor 10, vorzugsweise zumindest um den Faktor 20, besonders bevorzugt zumindest um den Faktor 30, ganz besonders bevorzugt zumindest um den Faktor 40 erhöht ist. Beispielsweise kann dadurch die Produktionsrate in der Kieselalge von herkömmlicherweise maximal 3 mg Antikörper pro Liter Kultur auf 160 mg Antikörper pro Liter Kultur erhöht werden. Besonders bevorzugt wird kann dadurch die Produktionsrate in der Kieselalge von herkömmlicherweise maximal 3 mg Antikörper pro Liter Kultur auf 100-1000 mg Antikörper pro Liter Kultur erhöht werden. Besonders bevorzugt kann die Produktionsrate in der Kieselalge von einem herkömmlichen Höchstwert von 3 mg Antikörper pro Liter Kultur auf 300 mg Antikörper pro Liter Kultur erhöht werden, was einem Faktor von 100 entspricht. In einigen sehr bevorzugten Ausführungsformen kann die Produktionsrate auf bis zu 1000 mg/L Kultur gesteigert werden, was einem Faktor von 333 entspricht.

Bevorzugt ist der rekombinante Antikörper im Bereich einer Gelenkregion derart modifiziert, dass dieser gegenüber kieselalgen-, pflanzen- oder mikroalgenspezifischen Proteasen eine erhöhte Stabilität, vorzugsweise mit einem Stabilitäts-Faktor von 1,1 bis 5, im Vergleich zum nativen Antikörper aufweist. Dies erhöht die Ausbeute an Antikörpern bei der Aufreinigung (auch als Downstream-Prozess bezeichnet) und erzeugt weniger störende Abbauprodukte, die die Reinheit der gewonnenen Antikörper reduzieren, was zu einem homogeneren rekombinanten Antikörper aus der Kultivierung führt, der spezifischere Signale und weniger Kreuzreaktionen liefert.

Die erhöhte Stabilität ist bezogen auf die Stabilität von Kieselalgen-eigenen Enzymen, speziell Proteasen, die an der als Hinge- oder auch als Gelenkregion bezeichneten Region des Antikörpers angreifen können und diesen enzymatisch spalten. Ein Stabilitäts-Faktor ist assoziiert mit der erhöhten Zeit, die der Antikörper mit der modifizierten Hinge-Region gegenüber Proteasen unter Kultivierungsbedingungen stabil ist, was die Produktionsmengen, insbesondere die Ausbeute an funktionsfähigen rekombinanten Antikörpern nach Abtrennung, erhöht. Da weniger proteolytisch gespaltene Antikörperfragmente vorhanden sind, ist die Antikörperlösung homogener und erlaubt den spezifischeren Nachweis mit deutlich weniger Kreuzreaktionen.

In einer bevorzugten Ausführungsform wird der Stabilitäts-Faktor zwischen einem Antikörper mit nativer Hinge und einem rekombinanten Antikörper mit modifizierter Hinge im Sinne der vorliegenden Erfindungen wird vorzugsweise in einer SDS PAGE ermittelt. Der Stabilitätsfaktor kann über Abbauprodukte des rekombinanten Antikörpers, die zu zusätzlichen Banden in der Coomassie-Färbung führen, bestimmt werden. Durch Zugabe bestimmter Proteasen, bevorzugt kieselalgen-, tier-, human-, pflanzen- oder mikroalgenspezifischen Proteasen, zu dem rekombinanten Antikörper mit modifizierter Hinge und dem Antikörper mit nativer Hinge und Durchführung von SDS-PAGE in bestimmten Zeitabständen lässt sich feststellen, wie viel intakter Antikörper nach dieser Zeit vorhanden ist und wie viele proteolytisch erzeugte Fragmente in der SDS-PAGE nachgewiesen werden. Die absoluten Anteile können densitometrisch ermittelt werden und der Quotient aus der Konzentration des rekombinanten Antigens und dem nativen Antikörper ergibt den Stabilitäts-Faktor. In einer besonders bevorzugten Ausführungsform ist der Stabilitäts-Faktor zwischen 1,1 und 10, insbesondere bevorzugt zwischen 1,1 und 5, am bevorzugtesten zwischen 1,1 und 3.

Nach einer bevorzugten Ausgestaltung ist die Aminosäuresequenz des Antikörpers, bspw. des ersten Antikörpers und/oder des zweiten Antikörpers und/oder jeden weiteren Antikörpers, derart modifiziert, dass dieser gegenüber kieselalgen-, tier-, human-, pflanzen- oder mikroalgenspezifischen Proteasen eine erhöhte Stabilität aufweist.

Beispielsweise ist die Aminosäuresequenz des Antikörpers, bspw. des ersten Antikörpers und/oder des zweiten Antikörpers und/oder jeden weiteren Antikörpers, im Bereich der Gelenkregion (Hinge-Region) derart modifiziert, dass dieser gegenüber wirtsspezifischen, insbesondere gegenüber kieselalgen-, pflanzen- oder mikroalgenspezifischen Proteasen eine erhöhte Stabilität aufweist. Hierdurch kann die Stabilität des Antikörpers in dem Wirtsorganismus, in welchem der Antikörper exprimiert wird, und somit auch die Ausbeute an intaktem Antikörper aus der Kultur erhöht wird.

Besonders bevorzugt ist eine Ausführungsform des Immunoassay, wobei der rekombinante Antikörper aus einer stabil transformierten Kieselalge oder einzelligen Pflanze, bevorzugt aus einer stabil transformierten Kieselalge, über mehrere Generationen exprimiert wird, bevorzugt für mindestens 60 Generationen, noch bevorzugter für mindestens 80 Generationen, am meisten bevorzugt für mindestens 100 Generationen. Dadurch können hohe Ausbeuten bei einer gleichzeitig hohen Qualität des Antikörpers garantiert werden, wodurch die Qualität von Assays über verschiedene Batches gewährleistet werden kann.

Eine Generation schließt mit einer Zellteilung ab und bezeichnet eine Einheit der zellulären Replikation. So entsprechen beispielsweise 60 Generationen 60 aufeinanderfolgenden Zellteilungen, die von einer Ausgangszelle ausgehen. Analog dazu bezieht sich die "Generationszeit" auf das Zeitintervall zwischen zwei aufeinander folgenden Generationen von Organismen in einer Population. Sie ist die Zeit, die eine einzelne Zelle oder ein Organismus benötigt, um sich zu teilen und zwei neue Zellen oder Organismen hervorzubringen. Die Generationszeit ist ein grundlegender Parameter, der die Wachstumsrate von einzelligen Organismen charakterisiert. Sie gibt Aufschluss darüber, wie schnell sich eine Population von genetisch identischer Zellen unter optimalen Bedingungen vermehren kann (Mitose). Kürzere Generationszeiten deuten auf schnellere Wachstumsraten und höhere Reproduktionskapazitäten hin, während längere Generationszeiten auf langsamere Wachstumsraten und potenziell komplexere zelluläre Prozesse hindeuten.

In einer bevorzugten Ausführungsform liegt die Generationszeit zwischen 6 und 48 Stunden, vorzugsweise zwischen 12 und 24 Stunden. Diese Generationszeit ist deutlich kürzer als die von aus dem Stand der Technik bekannten höheren Viridiplantae, wie z. B. *N. tabacum.* Durch die kurze Generationszeit kann ein schnelleres Wachstum der Kultur und damit eine deutlich erhöhte Produktionskapazität für Antikörper erreicht werden, als es bei höheren Pflanzen möglich ist.

Transient veränderte höhere Pflanzen sind gentechnisch veränderte Pflanzen, bei denen fremdes genetisches Material, wie z. B. Gene, die für bestimmte Proteine oder Merkmale kodieren, für einen kurzen Zeitraum in die Pflanzenzellen eingebracht wird. Diese Veränderung ist vorübergehend und führt nicht zur Integration der Fremdgene in das Genom der Pflanze. Stattdessen werden die fremden Gene exprimiert und die gewünschten Eigenschaften nur für eine begrenzte Zeit erzeugt. Diese Information ist somit nach einer Generation verloren, was einen hohen Aufwand bei der Züchtung von Zellkulturen als auch bei der Kontrolle der Produktqualität führt. Stattdessen können die Antikörper im Sinne der vorliegenden Erfindung über zahlreiche Generationen stabil exprimiert werden, was eine hohe Qualität und kontrollierbare Bedingungen ermöglicht.

Eine stabil transformierte Kultur ermöglicht die Expression des Antikörpers über mindestens 60 Generationen, vorzugsweise über mindestens 80 und besonders bevorzugt über mindestens 100 Generationen.

In einer bevorzugten Ausführungsform wird der Antikörper in einer Kultur für mindestens 30 Tage, bevorzugter für mindestens 40 Tage und am meisten bevorzugt für mindestens 60 Tage nach der Inokulation der Kultur stabil exprimiert.

Zusätzlich zu den anderen Vorteilen gegenüber höheren Pflanzen, die transient oder stabil Antikörper produzieren, enthalten Kieselalgen keine Fasern, was die Reinigung von Antikörpern erheblich erleichtert.

Besonders bevorzugt wird ein Antikörper im Zusammenhang mit dem Immunoassay intrazellulär exprimiert, bevorzugt in einer stabil transformierten Kieselalge. Bisher wurde im Stand der Technik bei der Gewinnung von Antikörpern aus Kieselalgen (Diatomeen) extrazelluläre Sekretion verwendet, da die Antikörper so direkt in das Kulturmedium abgegeben werden. Theoretisch würde dies zu einer erleichterten Isolierung führen, aber die Autoren haben die unerwartete Beobachtung gemacht, dass die intrazelluläre Sekretion zu deutlich höheren Produktionsmengen führt.

Diese herausragende Leistung der Erfinder führte zusammen mit den anderen hier beschriebenen Eigenschaften der Antikörper zu einer dramatischen Steigerung der Antikörperproduktion, vorzugsweise um den Faktor von 100 oder mehr.

In einer bevorzugten Ausführungsform kann der erste und/oder der zweite Antikörper ein Antikörper sein, der gegen das humane Choriongonadotropin gerichtet ist, vorzugsweise ist der Antikörper ein hCG-Antikörper. hCG ist ein Glykoprotein-Hormon, dass bereits vor dem Einnisten des Embryos produziert wird und sehr früh in einer Schwangerschaft eine solche anzeigt und das während der Schwangerschaft hauptsächlich von der Plazenta produziert wird. Es spielt eine entscheidende Rolle bei der Aufrechterhaltung des Gelbkörpers, der seinerseits Progesteron produziert, um die frühen Stadien der Schwangerschaft zu unterstützen. Gegen hCG gerichtete Antikörper können in verschiedenen Anwendungen eingesetzt werden, darunter auch in diagnostischen Tests für die Schwangerschaft. Schwangerschaftstests weisen das Vorhandensein von hCG in Urin oder Blut nach, was auf eine Schwangerschaft hinweist. Diese Antikörper werden als Erkennungselemente verwendet, um an hCG-Moleküle zu binden und ein messbares Signal zu erzeugen, das eine Schwangerschaft bestätigt. Dies ermöglich den frühzeitigen Nachweis einer Schwangerschaft, insbesondere als PoCT. PoC im Sinne der vorliegenden Erfindung bedeutet Point-of-Care, analog dazu bedeutet PoCT Point-of-Care-Testing, zu Deutsch *patientennahe Selbstdiagnostik.* Dieses Konzept bezieht sich auf medizinische Diagnosetests, die in der Nähe des Patienten durchgeführt werden, in der Regel außerhalb der traditionellen Laborumgebung.

In einer bevorzugten Ausführungsform wird der Immunoassay mit einem rekombinanten Antikörper, der gegen das hCG-Protein gerichtet ist, als Lateral Flow Assay zur Verfügung gestellt, insbesondere bevorzugt als Kit. Dadurch kann der Immunoassay so patientennah zur Verfügung gestellt werden, was eine einfache, schnelle Diagnostik vor Ort erlaubt.

In einer bevorzugten Ausführungsform ist das biologisch aktive Antigen ein Teil des Epitopes eines Virus, bevorzugt eines pathogenen Virus, beispielsweise Influenza, SARS-CoV-2, RSV, Adenovirus, Strep A, Norovirus, Rotavirus, HIV. Bevorzugt nutzt der Immunoassay hochspezifische Antikörper, die den ausgewählten Teil des Epitopes erkennen und daran binden, was eine schnelle und genaue Identifizierung von Virusinfektionen ermöglicht. Dieser Ansatz erleichtert die Frühdiagnose, die eine frühzeitige und gezielte Behandlung ermöglicht sowie weiterhin die genaue Überwachung des Infektionsverlaufs und die Möglichkeit, Ausbrüche schnell zu erkennen, was letztlich zu rechtzeigen Reaktionen der öffentlichen Gesundheit und wirksamen Eindämmungsstrategien beiträgt.

In einer bevorzugten Ausführungsform handelt es sich bei dem biologisch aktiven Antigen um eine Tumorassoziierte Sequenz, bevorzugt um einen HLA-Komplex und/oder einen sequenzierten Teil eines Tumor-Epitops und/oder einen Tumor-Marker, beispielsweise IFN-$\gamma$, IL-8, PSA, CEA, AFP, DCP, CA 125, HER2/neu. Dies ermöglicht eine frühzeitige Erkennung und Behandlung bösartiger Entartungen und kann somit die Heilungschancen für einen Patienten drastisch erhöhen. In einer bevorzugten Ausführungsform als Lateral-Flow-Assay kann dieser als PoCT zur Frühdiagnose dienen. In einer besonders bevorzugten Ausführungsform als LFA oder ELISA dient der Immunoassay als diagnostisches Mittel in Laboren oder medizinischen Einrichtungen durch medizinisches Personal durchgeführt.

IFN-$\gamma$ (Interferon-gamma) ist ein Zytokin, das von Immunzellen als Reaktion auf Infektionen produziert wird und eine Schlüsselrolle bei Immunreaktionen gegen Krankheitserreger spielt. Ein antikörperbasierter Immunoassay ermöglicht den präzisen Nachweis von IFN-$\gamma$ in Patientenproben und hilft so bei der Diagnose von Störungen des Immunsystems, der Überwachung von Autoimmunerkrankungen und der Beurteilung des Ansprechens auf eine Immuntherapie.

IL-8 (Interleukin-8) ist ein Chemokin, das an Entzündungsreaktionen und der Rekrutierung von Immunzellen beteiligt ist. Der Nachweis von IL-8 mittels antikörperbasierter Immunoassays hilft, entzündungsbedingte Zustände wie Autoimmunerkrankungen, Allergien und Infektionen zu verstehen und ermöglicht eine genaue Überwachung und Bewertung der Behandlung.

PSA (Prostata-spezifisches Antigen) ist ein Protein, das von der Prostata produziert wird. Erhöhte Werte können auf Prostataprobleme einschließlich Krebs hinweisen. Antikörper-basierte Immunoassays ermöglichen eine genaue Messung des PSA und helfen bei der Früherkennung von Prostatakrebs, der Risikobewertung und der Überwachung der Wirksamkeit von Behandlungen.

CEA (carcinoembryonales Antigen) ist ein Glykoprotein, das bei bestimmten Krebsarten, insbesondere bei Dickdarmkrebs, in erhöhter Konzentration vorkommt. Antikörper-basierte Immunoassays ermöglichen den empfindlichen Nachweis von CEA, was bei der Krebsdiagnose, der Überwachung des Behandlungsfortschritts und der Erkennung möglicher Rückfälle hilfreich ist.

AFP (Alpha-Fetoprotein) ist ein Protein, das während der fetalen Entwicklung gebildet wird. Erhöhte Werte bei Erwachsenen können auf Lebererkrankungen oder bestimmte Krebsarten wie Leberkrebs hinweisen. Ein Immunoassay auf Antikörperbasis ermöglicht den genauen Nachweis von AFP und unterstützt so die Frühdiagnose und die Überwachung des Behandlungserfolgs.

DCP (Des-Gamma-Carboxy-Prothrombin) ist ein Protein, das von den Leberzellen produziert wird. Erhöhte DCP-Werte werden mit Leberkrebs in Verbindung gebracht. Antikörper-basierte Immunoassays ermöglichen eine genaue Messung von DCP und helfen so bei der Früherkennung von Leberzellkarzinomen und der Überwachung des Behandlungserfolgs.

CA 125 (Cancer Antigen 125) ist ein Protein, das bei einigen Krebsarten, insbesondere Eierstockkrebs, erhöht ist. Antikörper-basierte Immunoassays bieten eine zuverlässige Methode zur Messung des CA 125-Spiegels, was bei der Diagnose von Eierstockkrebs, der Überwachung des Krankheitsverlaufs und der Beurteilung des Behandlungserfolgs hilfreich ist.

HER2/neu (humaner epidermaler Wachstumsfaktor-Rezeptor 2) ist ein Protein, das an der Regulierung des Zellwachstums beteiligt ist. Erhöhte Werte werden mit bestimmten aggressiven Brustkrebsarten in Verbindung gebracht. Der Nachweis von HER2/neu mittels antikörperbasierter Immunoassays hilft, geeignete Behandlungsstrategien zu identifizieren, den Krankheitsverlauf vorherzusagen und den Therapieerfolg bei Brustkrebspatientinnen zu überwachen.

In einer bevorzugten Ausführungsform des Immunoassay handelt es sich bei dem biologisch aktiven Antigen um eine charakteristische Sequenz zur Identifikation eines Proteins, vorzugsweise eines Enzym-Tags, besonders bevorzugt ausgewählt aus der Liste bestehend aus 6xHis-Tag, Strep-Tag, c-Myk-Tag, Flag-Tag und GST-Tag. Dies ermöglicht es einem Immunoassay, diese Protein-Tags nachzuweisen und damit die Spezifität und Empfindlichkeit zu bieten, um Proteine in verschiedenen Forschungszusammenhängen genau zu quantifizieren, zu reinigen und zu charakterisieren.

Der 6xHis-Tag ist eine kurze Peptidsequenz mit sechs Histidinresten, die häufig genetisch an Proteine fusioniert wird. Diese Fusionsmarkierung ermöglicht eine effiziente Reinigung des markierten Proteins mit Hilfe der immobilisierten Metallaffinitätschromatographie (IMAC) aufgrund der starken Bindungsaffinität zwischen Histidin und zweiwertigen Metallionen, wie Nickel. In einem antikörperbasierten Immunoassay können spezifische Antikörper den 6xHis-Tag erkennen und an ihn binden, was den Nachweis und die Quantifizierung des Proteins erleichtert. Der technische Vorteil des Immunoassays liegt in seiner hohen Spezifität und Empfindlichkeit, die eine präzise Messung des markierten Proteins selbst in komplexen biologischen Proben ermöglicht.

Der Strep-Tag ist ein Peptid-Tag, der durch eine Acht-Aminosäuresequenz (WSHPQFEK) gekennzeichnet ist, die eine hohe Bindungsaffinität für das Streptavidin-Protein aufweist. Wenn der Strep-Tag an ein Zielprotein fusioniert ist, ermöglicht er eine unkomplizierte Reinigung durch Interaktion mit Streptavidin-beschichteten Oberflächen. In einem antikörperbasierten Immunoassay können Antikörper, die den Strep-Tag erkennen, selektiv an den Tag des gewünschten Proteins binden. Dieser Ansatz ermöglicht einen effizienten und spezifischen Nachweis des Zielproteins, was ihn für verschiedene Forschungs- und Diagnoseanwendungen wertvoll macht.

Der c-Myc-Tag ist vom c-Myc-Protein abgeleitet und besteht aus zehn Aminosäuren (EQKLISEEDL). Er wird üblicherweise als Fusions-Tag verwendet, um den Nachweis und die Reinigung von Proteinen zu erleichtern. In antikörperbasierten Immunoassays können Antikörper gegen den c-Myc-Tag spezifisch an den Tag binden, was eine empfindliche Detektion und Quantifizierung des markierten Proteins ermöglicht. Der technische Vorteil dieser Technik liegt in ihrer Vielseitigkeit, da sie für ein breites Spektrum von Proteinstudien und -tests eingesetzt werden kann.

Der Flag-Tag ist eine Peptidsequenz (DYKDDDDK), die üblicherweise an den N- oder C-Terminus eines Proteins angehängt wird, um die Identifizierung und Isolierung des Proteins zu erleichtern. Sie wird von handelsüblichen Anti-Flag-Antikörpern erkannt und ermöglicht eine unkomplizierte Proteindetektion und -reinigung. Der technische Vorteil eines antikörperbasierten Immunoassays für den Flag-Tag liegt in seiner Robustheit und seiner breiten Verfügbarkeit, was ihn zu einer beliebten Wahl für Forscher macht, die mit rekombinanten Proteinen arbeiten.

Der GST-Tag, der von dem Enzym Glutathion-S-Transferase abgeleitet ist, wird häufig für die Proteinexpression, -reinigung und Interaktionsstudien verwendet. Die Verbindung des Tags mit Glutathion-konjugierten Matrizes ermöglicht eine effiziente Reinigung in einem Schritt. In Antikörper-basierten Immunoassays können Antikörper, die spezifisch für den GST-Tag sind, das markierte Protein nachweisen und quantifizieren. Der technische Vorteil dieses Immunoassays liegt in seiner Einfachheit und der Möglichkeit, durch den Affinitätsreinigungsschritt eine hohe Proteinausbeute und -reinheit zu erzielen.

In einer bevorzugten Ausführungsform des Immunoassay handelt es sich bei dem biologisch aktiven Antigen um eine Sequenz, die mit Ernährungsparametern assoziiert ist, beispielsweise Transcobalamin II, Ferritin, Homocystein, Eicosapentaensäure (EPA) und Docosahexaensäure (DHA), oder Calcitriol. Dies ermöglicht die Überprüfung einiger wichtiger Parameter, die es einem Individuum oder Subjekt, vorzugsweise einem Menschen, erlauben, seine Gesundheit zu überprüfen und zu verbessern. Insbesondere können Personen mit bestimmten Ernährungsgewohnheiten einen Mangel an bestimmten Vitaminen und Spurenelementen aufweisen, wobei ein Immunoassay gemäß der vorliegenden Erfindung, bevorzugt ausgestaltet als LFA, insbesondere bevorzugt als PoCT, dazu beitragen kann, diese Mängel zu erkennen und auszugleichen. In einer alternativen Ausführungsform ist das Ergebnis des Immunoassays dabei nur eine Empfehlung und keine medizinische Indikation.

Transcobalamin II (TCII) ist ein Transportprotein, das eine entscheidende Rolle beim Transport von Vitamin B12 (Cobalamin) im Körper spielt. Es bindet an Vitamin B12 und erleichtert dessen Transport zu den Zellen für verschiedene biochemische Prozesse. Das Vorhandensein von TCII kann auf einen Vitamin-B12-Mangel oder bestimmte Erkrankungen hinweisen. Ein Immunoassay auf Antikörperbasis, der auf TCII abzielt, ermöglicht den genauen Nachweis und die Quantifizierung dieses Proteins in klinischen Proben und hilft so bei der Diagnose und Überwachung von Störungen im Zusammenhang mit dem Vitamin-B12-Stoffwechsel.

Ferritin ist ein Protein, das Eisen auf kontrollierte Weise speichert und abgibt und so zur Eisenhomöostase im Körper beiträgt. Die Messung des Ferritinspiegels ist für die Beurteilung des Eisenstatus und die Diagnose von Erkrankungen wie Eisenmangelanämie oder Eisenüberladung von entscheidender Bedeutung. Ein Immunoassay auf Antikörperbasis, bevorzugt in Form eines LFA als PoCT, der auf Ferritin abzielt, ermöglicht die präzise Quantifizierung dieses Proteins in Blut- oder Gewebeproben und liefert wertvolle Informationen über den Eisengehalt und die allgemeine Gesundheit einer Person.

Homocystein ist eine Aminosäure, die aus dem Metabolismus von Methionin entsteht. Erhöhte Homocysteinwerte im Blut werden mit einem erhöhten Risiko für Herz-Kreislauf-Erkrankungen und andere Gesundheitsprobleme in Verbindung gebracht. Der Nachweis von Homocystein mit einem Immunoassay, bevorzugt in Form eines LFA als PoCT, bietet eine zuverlässige Methode zur Bewertung des kardiovaskulären Risikos einer Person und zur Überwachung der Wirksamkeit von Maßnahmen zur Senkung des Homocysteinspiegels.

Eicosapentaensäure (EPA) und Docosahexaensäure (DHA) sind Omega-3-Fettsäuren, die in bestimmten Fischölen vorkommen und für ihre potenziellen gesundheitlichen Vorteile bekannt sind, einschließlich der Unterstützung des Herz-Kreislauf-Systems und der kognitiven Fähigkeiten. Die Messung des EPA- und DHA-Spiegels im Blut gibt Aufschluss über den Omega-3-Fettsäurestatus einer Person und hilft bei der Erstellung von Ernährungsempfehlungen. Ein antikörperbasierter Immunoassay, bevorzugt in Form eines LFA als PoCT, der auf EPA und DHA abzielt, ermöglicht eine genaue Quantifizierung dieser Fettsäuren und hilft bei der personalisierten Bewertung der Ernährung.

Calcitriol ist die aktive Form von Vitamin D und spielt eine entscheidende Rolle bei der Aufrechterhaltung des Calcium- und Phosphorhaushalts, der Knochengesundheit und verschiedener physiologischer Prozesse. Die Überwachung des Calcitriolspiegels ist wichtig für die Beurteilung des Vitamin-D-Status und die Diagnose von Erkrankungen wie Rachitis und Osteoporose. Ein auf Antikörpern basierender Immunoassay, bevorzugt in Form eines LFA als PoCT, der spezifisch für Calcitriol ist, ermöglicht die präzise Messung dieses Hormons in Blutproben und hilft so bei der Bewertung des Vitamin-D-Status einer Person und der Festlegung geeigneter Maßnahmen.

In einer bevorzugten Ausführungsform wird der rekombinante Antikörper aus einer Kieselalge gewonnen. In einer besonders bevorzugten Ausführungsform ist die Kieselalge *Phaeodactylum tricornutum.* Dies ermöglicht die Bereitstellung eines Antikörpers mit den hierin offengelegten Eigenschaften, insbesondere hoher Homogenität und Reinheit, die die Bereitstellung eines erfindungsgemäßen Immunoassays ermöglichen.

Bevorzugt ist der Immunoassay ein Lateral-Flow-Immunoassay, welcher fluidverbunden mindestens einen Probenauftragsbereich, einen Konjugatbereich und einen Fangbereich, die auf einer Membran angeordnet sind, bereitstellt.

In einem Lateral-Flow-Immunoassay (hierin auch "LFA") finden typischerweise drei Antikörper Verwendung:

- ein erster immobilisierter Antikörper, der im Fangbereich (auch als Testzone bezeichnet) angeordnet ist und auch als Fängerantikörper bezeichnet wird. Dieser ist wie der zweite Antikörper (auch als Detektionsantikörper bezeichnet) ebenfalls gegen das Antigen gerichtet, vorzugsweise gegen ein anderes Epitop des Antigens als der Detektionsantikörper;

- ein zweiter Antikörper, der vorzugsweise im Konjugatbereich vorgesehen ist und auch als Detektionsantikörper bezeichnet wird, welcher gegen ein Epitop des Antigens gerichtet ist, z. B. ein Hormon, ein Protein oder ein Peptid, wie humanes Choriongonadotropin, wobei der zweite Antikörper vorzugsweise an Markerpartikel konjugiert ist, z. B. Gold-, Silber-, Latex-, Carbon-, Nanopartikel oder Enzyme.

- Ein weiterer Antikörper, der vorzugsweise in einem Kontrollbereich (Kontrollzone) angeordnet, vorzugsweise immobilisiert, ist und gegen einen Detektions- /Kontrollantikörper gerichtet ist.

Vorzugsweise werden der erste Antikörper (bspw. der Fängerantikörper), der zweite Antikörper (bspw. der Detektionsantikörper) und/oder der weitere Antikörper (bspw. der Fängerantikörper in der Kontrollzone), besonders bevorzugt alle Antikörper, aus einer Kieselalge, einzelligen Pflanze oder Viridiplantae gewonnen und zeichnen sich durch die hierin definierten Charakteristika aus.

Besonders bevorzugt werden der erste Antikörper (bspw. der Fängerantikörper), der zweite Antikörper (bspw. der Detektionsantikörper) und/oder der weitere Antikörper (bspw. der Fängerantikörper in der Kontrollzone), besonders bevorzugt alle Antikörper, aus einer Kieselalge oder einzelligen Pflanze gewonnen und zeichnen sich durch die hierin definierten Charakteristika aus.

In einer bevorzugten Ausführungsform wird der LFA als PoCT bereitgestellt. In einer besonders bevorzugten Ausführungsform wird der LFA als Kit bereitgestellt, umfassend mindestens das LFA und eine Anleitung. Dies erlaubt die einfache Benutzung durch den Patienten selbst und kann so eine schnellere Diagnostik außerhalb der Gesundheitssystem-Infrastruktur erlauben.

Bevorzugt ist der erste Antikörper in einer Ausführungsform, die sich auf LFA und Sandwich-ELISA bezieht, gegen eine erste Domain des biologisch aktiven Antigens (wie hierin definiert) gerichtet. Dabei ist bevorzugt ist der zweite Antikörper in einer Ausführungsform, die sich auf LFA und Sandwich-ELISA bezieht, gegen eine zweite Domain des biologisch aktiven Antigens (wie hierin definiert) gerichtet.

Bevorzugt ist der erste Antikörper in einer Ausführungsform, ELISA bezieht, gegen eine Domain des biologisch aktiven Antigens (wie hierin definiert) gerichtet und der zweite Antikörper ist bevorzugt gegen eine Domain des ersten Antikörpers gerichtet.

In einer besonders bevorzugten Ausführungsform wird ein Enzyme-Linked Immunosorbent Assay (ELISA) Immunoassay ausgeführt, wobei mindestens

a. ein Probenauftragsbereich zum Auftragen einer biologischen Probe bereitgestellt wird, welcher
b. von einem Fangbereich, der als eine Gefäßbegrenzung und/oder Teil einer Gefäßbegrenzung ausgeführt ist, bevorzugt als Mikrotiterplatte, begrenzt ist, und
c. einem Konjugatbereich, welcher räumlich im Probenauftragsbereich angeordnet ist.

ELISA (Enzyme-Linked Immunosorbent Assay) ist eine weit verbreitete Labortechnik zum Nachweis und zur quantitativen Bestimmung des Vorhandenseins spezifischer Proteine oder Antikörper in einer Probe. Bei einigen Formen des ELISA wird ein Zielantigen oder - antikörper auf einer festen Oberfläche immobilisiert, und anschließend werden spezifische, an Enzyme gebundene Antikörper zum Nachweis und zur Quantifizierung der Menge an Antigen, die mit der Menge an gebundenem Antikörper korreliert, eingesetzt. Bei einem Sandwich-ELISA werden zwei verschiedene Antikörper verwendet. Der erste Antikörper ist auf der ELISA-Platte immobilisiert und bindet spezifisch an das Antigen in der Probe. Der zweite (Nachweisantikörper) ist markiert, z. B. mit einem Enzym, und bindet an ein anderes Epitop auf demselben Antigen. Zu den technischen Vorteilen des ELISA gehört seine hohe Empfindlichkeit, die den Nachweis niedriger Konzentrationen von Zielmolekülen ermöglicht. Er ist vielseitig und eignet sich für eine breite Palette von Probentypen und Molekülen, einschließlich Proteinen, Peptiden und kleinen Molekülen. Ein ELISA kann quantitative Informationen über die Menge des in einer Probe vorhandenen Zielmoleküls liefern. Es stellt eines der wichtigsten Werkzeuge der Molekularbiologie dar und ist aus der Analytik und Diagnostik nicht mehr wegzudenken. Neben den Antikörpern und der zu analysierenden Probe wird eine Mikrotiterplatte als Trägermaterial sowie ein Lesegerät, ein sogenannter ELISA-Reader benötigt. Es gibt verschiedene Varianten der Durchführung, in der einfachsten Form wird das zu analysierende Antigen in die Vertiefungen der Mikrotiterplatten pipettiert, wo das Antigen fest an das Polystyrol der Mikrotiterplatten bindet. Verbleibende freie Bindungsstellen auf dem Polystyrol werden mit einem Blockierungsreagenz (z. B. veganes BSA) gesättigt, so dass die anschließend zugegebenen Antikörper nur an ihre Antigene und nicht an freie Bindungsstellen des Polystyrols binden können.

Die Natriumdodecylsulfat-Polyacrylamid-Gelelektrophorese (SDS-PAGE) ist eine weit verbreitete Labortechnik zur Trennung von Proteinen nach ihrem Molekulargewicht. Bei der SDS-PAGE werden die Proteine denaturiert und mit dem anionischen Detergens Natriumdodecylsulfat (SDS) beschichtet, um ihnen eine einheitliche negative Ladung zu verleihen. Anschließend werden sie in ein poröses Polyacrylamidgel geladen und einem elektrischen Feld ausgesetzt, wodurch die Proteine entsprechend ihrer Größe wandern. Zu den technischen Vorteilen der SDS-PAGE gehört ihr hohes Auflösungsvermögen, das eine genaue Trennung von Proteinen in komplexen Gemischen ermöglicht. Sie liefert quantitative und qualitative Informationen über Proteinproben und hilft so bei der Identifizierung und Charakterisierung von Proteinen. Die SDS-PAGE ist mit verschiedenen Probentypen kompatibel und kann sowohl denaturierte als auch mit einigen methodischen Modifikationen native Proteine verarbeiten.

Solchermaßen im SD-PAGE aufgetrennten Proteine können mittels Western Blot Transfer auf eine proteinbindende Membran, beispielsweise eine Nitrozellulose übertragen werden. Auf diese Weise werden die zuvor aufgetrennten Proteine zugänglich für die Bindung von Antikörpern, die nach Absättigung der nach dem Western Blot noch freien Membranbereiche zugegeben werden. Wie bei einem Immunoassay üblich, wird häufig eine Kaskade von mindestens zwei verschiedenen Antikörpern verwendet. Durch die zuvor durchgeführte Kaskade kommt es zu einer Signalverstärkung da der zweite Antikörper gegen den ersten Antikörper. Der zweite Antikörper ist häufig mit einem Enzym wie alkalischer Phosphatase (AP) oder Meerrettichperoxidase (HRP) gekoppelt, um die Bindung sichtbar zu machen. Darüber hinaus kommt es häufig zu einer Signalverstärkung, da der zweite Antikörper mehrfach an den ersten Antikörper binden kann. Nach der Zugabe wird je nach Menge des gebundenen Antigens oder Antikörpers durch die Enzymreaktion ein unlösliches Produkt gebildet, das direkt an der Stelle, zu der das Antigen im SDS-PAGE gelaufen ist, präzipitiert und das Antigen so eindeutig identifiziert.

Alternativ bevorzugt ist der Immunoassay ein Enzyme-linked Immunosorbent Assay (ELISA), bspw. ein direkter ELISA, ein indirekter ELISA, ein direkter Sandwich-ELISA oder ein indirekter Sandwich-ELISA, vorzugsweise ist der ELISA ein direkter Sandwich-ELISA oder ein indirekter Sandwich-ELISA.

Gemäß einer bevorzugten Ausführungsform können sich der Konjugatbereich, der Probenauftragsbereich und der Fangbereich räumlich in derselben Umgebung, beispielsweise in einem Gefäß, bevorzugt ausgeführt als Well einer Mikrotiterplatte oder Reagenzglas, befinden und durch zeitlich getrennte Zugaben und Waschschritte voneinander getrennt sein. In einer Ausführungsform, die mit einem ELISA assoziiert ist, wird ein Antigen, das einer biologischen Probe eines Individuums entspricht, an der Wand des Gefäßes immobilisiert und bildet somit den Probenauftragbereich. Anschließend wird ein erster, gegen das biologisch aktive Antigen gerichteter Antikörper zugegeben, welcher am Antigen bindet und somit an der Wand des Gefäßes immobilisiert wird, wodurch der Fangbereich gebildet wird. Nach einem optionalen Waschschritt wird ein zweiter Antikörper zugegeben, der gegen den ersten Antikörper gerichtet ist, wodurch der Konjugatbereich gebildet wird. Die Bereiche sind dabei fluidverbunden, aber zeitlich getrennt. In einer Ausführungsform, die mit einem Sandwich ELISA assoziiert ist, wird der erste Antikörper zunächst an der Wand des Gefäßes immobilisiert und bildet somit den Fangbereich. Anschließend wird eine biologische Probe eines Individuums hinzugegeben, wodurch der Probenauftragbereich gebildet wird. Nach einem optionalen Waschschritt wird ein zweiter Antikörper zugegeben, der gegen das biologisch aktive Antigen gerichtet ist, wodurch der Konjugatbereich gebildet wird. Die Bereiche sind dabei fluidverbunden, aber zeitlich getrennt.

In einer besonders bevorzugten Ausführungsform wird der zweite Antikörper als Antikörper-Enzym-Konjugat bereitgestellt. Dies erlaubt die Detektion eines ersten Antikörpers und Quantifizierung des Signals durch das Enzym in einem ELISA, wenn ein lösliches Produkt entsteht, bzw., in einem Immunoblot, wenn das Produkt der Enzymreaktion am Ort seiner Bildung präzipitiert.

In einer bevorzugten Ausführungsform ist ein erster Antikörper, der vorzugsweise aus einer Kieselalge gewonnen wird, gegen ein Antigen gerichtet, und ein zweiter Antikörper, der ebenfalls vorzugsweise aus einer Kieselalge gewonnen wird und an ein Enzym, vorzugsweise alkalische Phosphatase (AP) oder Meerrettichperoxidase (HRP), gekoppelt ist, entweder ebenfalls gegen das erste Antigen (LFA bzw. Sandwich ELISA) oder gegen den ersten Antikörper (ELISA oder Immunoblot) gerichtet. Vorzugsweise kann der zweite Antikörper mehrfach an den ersten Antikörper binden, was zu einer Signalverstärkung führen kann. Besonders bevorzugt kann der zweite Antikörper zwischen 1- und 10-mal an den ersten Antikörper binden, am meisten bevorzugt zwischen 1- und 5-mal. Die daraus resultierende Signalverstärkung hat somit vorzugsweise einen Faktor von 1-10, besonders bevorzugt 1-5. Durch diese starke Verstärkung kann der ELISA eine geringe Konzentration an Antigen bestimmen oder es ist eine geringe Menge an ersten Antikörper und/oder zweiten Antikörper notwendig, um eine Detektion über der Nachweisgrenze zu erlauben. Aufgrund der geringen Konzentration können entsprechende Antigene z. B. früher im Krankheitsverlauf nachgewiesen werden und so zu einer schnellen Erkennung und Behandlung beitragen, die z. B. Infektionsketten unterbrechen kann.

In einer Ausführungsform, die mit einem LFA assoziiert ist, stellt die Erfindung bevorzugt eine Vorrichtung bereit, aufweisend: einen Behälter, insbesondere ein Gehäuse, und einen darin angeordneten erfindungsgemäßen Immunoassay, insbesondere einen Lateral-Flow-Immunoassay.

Offenbart ist auch eine Vorrichtung, die ein Gehäuse (8), bspw. einen Behälter, insbesondere ein Gehäuse, und einen darin angeordneten Immunoassay (wie hierin definiert), insbesondere einen Lateral-Flow-Immunoassay umfasst.

Nach einer bevorzugten Ausgestaltung ist das Gehäuse der Vorrichtung im Wesentlichen, insbesondere ausschließlich aus einem Zellulosematerial, vorzugsweise Papier oder Papierkarton gebildet. Dadurch kann auf den Einsatz von Kunststoffen verzichtet werden.

Nach einer besonders Nach einer bevorzugten Ausgestaltung ist das Gehäuse der Vorrichtung im Wesentlichen, besonders z.B. mehr als 90 %, aus Biokunststoffen aufgebaut, vorzugsweise Polymilchsäure (PLA), Polyhydroxyalkanoate (PHA) oder stärkebasierte Kunststoffe. Als Biokunststoffe werden in diesem Zusammenhang Kunststoffe bezeichnet, die biologisch abbaubar sind, d.h. die sich z.B. in Kompostieranlagen oder in der Natur auf natürliche Weise in umweltverträgliche Stoffe auflösen können.

Vorzugsweise ist das Gehäuse der Vorrichtung aus zumindest zwei Lagen Zellulosematerial gebildet, die die Membran zumindest teilweise umschließen. Dabei können die Enden der Lagen des Zellulosematerials derart übereinandergelegt sein, dass diese bündig zueinander abschließen.

In einer bevorzugten Ausführungsform wird die Membran von einem Gehäuse im Sinne der vorliegenden Erfindung umgeben, die überwiegend, bevorzugt zu 90 bis 100 %, aus Zellulosefasern besteht, die einerseits wasserbeständig ist, bevorzugt zumindest für den Transport und die Nutzungsdauer, und andererseits aus nachhaltigen, vorzugsweise wiedergewonnenen Pflanzenfasern hergestellt wird und biologisch abbaubar ist, z. B. durch mikrobiologischen Abbau in der Umwelt oder durch Kompostierung.

Biologisch abbaubar bedeutet im Sinne der vorliegenden Erfindung, dass zumindest 90-100 % der Bestandteile, bevorzugt 95-100 % der Bestandteile, am meisten bevorzugt 99-100 % der Bestandteile biologisch, zum Beispiel durch mikrobiologischen Abbau in der Umwelt und/oder bevorzugt durch industrielle Kompostierung nach EN 13432 möglich ist.

In einer bevorzugten Ausführungsform sind an dem Gehäuse an der Membran Markierungen (Symbole, Zeichen, geometrische Formen) angeordnet, die die einfache Interpretation des Immunoassay-Ergebnisses erlauben. Insbesondere in der Ausführungsform eines Kits, in der neben dem Assay eine Anleitung bereitgestellt wird, ist die einfache Handhabung auch für nicht medizinisches Personal gegeben. Dadurch kann das Kit als PoCT verwendet werden.

Beispielsweise sind die Enden von zumindest zwei Lagen des Zellulosematerial, die vorzugsweise das Gehäuse der Vorrichtung bilden, an den Seitenbereichen, in denen sie einander kontaktieren, geprägt und/oder gestanzt, wodurch diese fest miteinander verbunden sind. Beispielsweise sind die Enden der Lagen durch eine Prägenaht (9) miteinander verbunden.

Nach einer bevorzugten Ausgestaltung ist der Probenauftragsbereich lediglich teilweise in dem Gehäuse angeordnet. Der Probenauftragsbereich kann dabei als Pad (Kissen) ausgebildet sein. Dabei kann das Pad aus einem bauschigen, porösen oder faserigen Material bestehen, das in der Lage ist, Flüssigkeit schnell zu absorbieren.

Nach einer bevorzugten Ausgestaltung weist die Membran im distalen Bereich (betrachtet in Strömungsrichtung vom Auftragungsbereich am proximalen Ende der Membran), vorzugsweise distal zum Fangbereich einen Fixierungsbereich (7) auf, durch den die Membran im Gehäuse der Vorrichtung fixiert ist.

Im distalen Bereich der Membran kann am Gehäuse der Vorrichtung eine Bruchstelle (10) als Beispiel für einen Trennbereich vorgesehen sein, durch den das distale Ende des Gehäuses, vorzugsweise mit dem distalen Ende der Membran abgetrennt werden kann.

Besonders bevorzugt wird Vorrichtung so bereitgestellt, dass der Behälter, bevorzugt ein Gehäuse, aus nachhaltigen und wasserbeständigen Materialien, vorzugsweise Papier und/oder Faserguss gestaltet ist. Dies erlaubt die Bereitstellung eines umweltverträglichen Immunoassay, insbesondere eines umweltverträglichen LFA Immunoassay. Allein durch Schwangerschaftstests werden jährlich ca. 900 t Plastik produziert, weshalb die Bereitstellung eines Gehäuses, das kein Plastik verwendet, sondern aus Papier oder Faserguss gestaltet wird, erstrebenswert ist.

Offenbart ist ein Kit, aufweisend eine Vorrichtung und eine Anleitung zur Durchführung des Immunoassay. Besonders bevorzugt enthält der Kit weitere Komponenten, z. B. Wattestäbchen oder andere Probennahmeutensilien, Pufferlösung als Laufmediummischung, weitere Lösungen, Dessikate zum Trocknen und andere dem Fachmann bekannte Hilfsmittel zur Durchführung eines Immunoassays als PoCT oder Testkit für medizinisch geschultes Personal.

Ferner offenbart ist ein Kit, das eine hierin definierte Vorrichtung und eine Anleitung zur Durchführung des in der Vorrichtung angeordneten Immunoassays umfasst.

Offenbart aber nicht beansprucht ist die Verwendung eines Immunoassay (wie hierin definiert) zum Nachweisen eines biologisch aktiven Proteins in einer biologischen Probe eines Individuums.

Offenbart ist auch ein Verfahren zum Nachweis eines biologisch aktiven Proteins in einer biologischen Probe eines Individuums mitumfasst, wobei das Verfahren die folgenden Schritte aufweist:

a) Gewinnen einer biologischen Probe von einem Individuum;

b) Analysieren der biologischen Probe unter Verwendung eines Immunoassays, der derart eingerichtet ist, dass dieser das biologisch aktive Protein nachweist.

Durch die Bereitstellung einer Anleitung in Kombination mit dem Immunoassay kann eine einfache Nutzung des Kits als PoCT gewährleistet werden. Dies bietet mehrere technische Vorteile, darunter schnelle Ergebnisse aufgrund der kürzeren Transportzeit, unmittelbare klinische Entscheidungen und ein verbessertes Patientenmanagement. PoCT minimiert mögliche präanalytische Fehler, erhöht die Effizienz in Notfallszenarien und unterstützt rechtzeitige Behandlungsmaßnahmen. Der dezentrale Charakter von PoCT erleichtert die Überwachung von chronischen Erkrankungen und Infektionskrankheiten.

In einer Ausführungsform wird die Verwendung des Immunoassay als Life-Style Produkt, insbesondere zur Detektion von Ernährungs-assoziierten Parametern, offenbart. Dies ermöglicht die Verfolgung von Parametern, die mit Mangel- oder Unterernährung in Verbindung stehen, und ist keine medizinische Empfehlung, sondern ermöglicht es einer Person, potenzielle Gesundheitsrisiken zu erkennen. Dies kann z. B. zur unabhängigen Kontrolle der Ernährung genutzt werden.

Ein Lifestyle-Produkt ist ein Gegenstand oder eine Dienstleistung, der/die aufgrund seiner/ihrer Übereinstimmung mit einem bestimmten Lebensstil, einer Reihe von Werten oder einer persönlichen Identität bereitgestellt wird. Diese Produkte gehen oft über ihren funktionalen Zweck hinaus und werden von den Verbrauchern ausgewählt, um ihre gewünschte Lebensweise, ihre Interessen und ihren Selbstausdruck zu reflektieren und zu verbessern, wobei nicht zwangsweise eine medizinische Empfehlung abgeleitet werden kann. In dieser Ausführungsform stellt der Immunoassay kein medizinisches Produkt, sondern ein Hilfsmittel da, mit den ein Subjekt seine Lebensweise überprüfen und optimieren kann.

Das vorliegende Dokument offenbart weiterhin ein Verfahren zum Nachweis eines biologisch aktiven Antigens in einer biologischen Probe, bevorzugt Urin, Vollblut, Speichel, Milch oder Serum, umfassend die folgenden Schritte:

a) Gewinnen einer biologischen Probe von einem Individuum;

b) Analysieren der biologischen Probe unter Verwendung eines Immunoassay nach einem der Ansprüche 1 bis 15, der derart eingerichtet ist, dass dieser das biologisch aktive Antigen nachweist.

Durch dieses Verfahren kann die Anwendung des Immunoassays gewährt werden, wodurch schnell und präzise biologisch aktive Antigene analysiert werden können. In einer besonders bevorzugten Ausführungsform wird die biologische Probe von dem Individuum, das den Immunoassay durchführt, selbst bereitgestellt, in einer alternativen Ausführungsform wird die Probe durch eine medizinisch geschulte Fachkraft gewonnen. Vorzugsweise erfolgt keine weitere Behandlung der biologischen Probe nach der Gewinnung. Besonders bevorzugt kann die biologische Probe direkt auf den Immunoassay aufgetragen werden. Dies erlaubt eine schnelle und direkte Bestimmung eines biologisch aktiven Proteins.

In einer Ausführungsform wird die Verwendung des Immunoassays als Point-of-Care-Diagnostik (PoC) offenbart, insbesondere für den Nachweis von Antigenen, die Teil des Epitops eines Virus sind, vorzugsweise eines pathogenen Virus, z. B. Influenza, SARS-CoV-2, RSV, Adenovirus, Strep A, Norovirus, Rotavirus, HIV, und/oder einen rekombinanten Antikörper umfasst, der gegen humanes Choriongonadotropin gerichtet ist, vorzugsweise ist der rekombinante Antikörper ein hCG-Antikörper. Sie bietet mehrere technische Vorteile, darunter schnelle Ergebnisse aufgrund der kürzeren Transportzeit, unmittelbare klinische Entscheidungen und ein verbessertes Patientenmanagement. PoCT minimiert mögliche präanalytische Fehler, erhöht die Effizienz in Notfallszenarien und unterstützt rechtzeitige Behandlungsmaßnahmen. Der dezentrale Charakter von PoCT erleichtert die Überwachung von chronischen Erkrankungen und Infektionskrankheiten.

In einem bevorzugten Ausführungsform wird weiterhin mindestens ein Hilfsprotein zum Abdeck-, Maskierund/oder Unterstützungsprotein in einem Immunoassay offenbart, wobei das Hilfsprotein rekombinant aus einer stabil transformierten Kieselalge oder einzelligen Pflanze gewonnen wird.

Immunoassays, z. B. ELISAs, Dot Blots, Immunoblot sowie LFAs, benötigen neben den Antikörpern Hilfsproteine, die einerseits die Antikörperlösungen stabilisieren können und andererseits dazu dienen, die proteinbindenden Oberflächen zu blockieren. Bei Immunoassays, LFAs und Dot Blots ist die Oberfläche dabei bevorzugt Nitrocellulose, bei ELISAs vorzugsweise Polystyrol. Im Stand der Technik werden hierbei überwiegend tierische Proteine, wie Rinder- bzw. Kälberserum oder Magermilchpulver bzw. Kasein eingesetzt. Um einen veganen Immunoassay, bevorzugt in einem veganen LFA und/oder veganen ELISA, bereitstellen zu können, müssen diese durch tierfreie Alternativen ersetzt werden. Es sind synthetisch hergestellte Alternativen bekannt, z. B. ROTI®Block, diese sind jedoch nicht für alle Anwendungen geeignet und sehr kostenintensiv und wirtschaftlich nicht konkurrenzfähig.

Dieses Dokument offenbart, analog zu rekombinanten Antikörpern, die Bereitstellung von Hilfsproteinen, die aus einer stabil transformierten Kieselalge oder einzelligen Pflanze oder höheren Pflanzen stammen. Dazu wird, analog zu den Antikörpern, die Sequenz von Rinderserumalbumin (BSA) codon-optimiert und als Gen in *Phaeodactylum tricornutum* eingeführt und dort exprimiert.

Nach dem Aufschluss der Kieselalgen kann das vegane BSA gereinigt werden, vorzugsweise durch Affinitätschromatographie, oder, in einer bevorzugten Ausführungsform, direkt ohne weitere Reinigung als Rohextrakt in verschiedenen immunbiochemischen Verfahren verwendet werden.

Besonders bevorzugt wird das Hilfsprotein im Sinne der vorliegenden Erfindung, bevorzugt ein vegan aus einer Kieselalge exprimiertes Protein, ausgewählt aus der Liste beinhaltend BSA, Kasein, oder Gelatine. Rinderserumalbumin (BSA) wird häufig als Blockierungsmittel verwendet, um die unspezifische Bindung von Antikörpern an die Testoberflächen zu verhindern und das Hintergrundrauschen zu verringern. Kasein ist ein weiteres Blockierungsmittel, das dazu beiträgt, unspezifische Wechselwirkungen zu verhindern und das Signal-Rausch-Verhältnis in Assays zu verbessern, Gelatine ist ein von Kollagen abgeleitetes Protein, das ebenfalls zur Blockierung unspezifischer Bindungen in Immunoassays verwendet werden kann.

Die Aufreinigung der Antikörper folgt in einer bevorzugten Ausführungsform einen dem Fachmann geläufigen Verfahren, d. h. nach der Lyse und Zentrifugation und/oder einer Ultrafiltration erfolgt die Aufreinigung entweder über Protein A, Protein G oder über die eingesetzten Tag-Sequenzen, beispielsweise den 6xHis-Tag. Hier liegt ein weiterer Vorteil des erfindungsgemäßen Verfahrens, denn im Gegensatz zu höheren Pflanzen, die transient oder stabil Antikörper produzieren, enthalten die Kieselalgen keine die Aufreinigung der Antikörper enorm erschwerenden Fasern. Dadurch ist eine technisch effiziente und wirtschaftlich sinnvolle Nutzung der Gewonnen Antikörper möglich. Die Aufreinigung entspricht daher zu großen Teilen dem Verfahren, wie es beispielsweise für tierische Zellkulturen, wie CHO-Zellen, eingesetzt wird und ist einen Fachmann geläufig und wird im Folgenden beschrieben.

Bevorzugt werden die Zellen des Produktionsklons, in diesem Beispiel einer Kieselalge, durch einen so genannten schonenden Aufschluss zerstört, wobei das Produkt, der Antikörper im Sinne der vorliegenden Erfindung, geschützt wird. Zu den schonenden Methoden gehören z. B. Hochdruck, elektrische Spannung, Ultraschall oder Aufschluss durch Kollision in sogenannten Schwingmühlen. In einer besonders bevorzugten Ausführung erfolgt die Trennung durch eine Kombination aus Manton-Gaulin-Homogenisator und folgender Ultraschall-Behandlung mit Covaris E220 Focused Ultrasonicator.

Nach dem Aufschluss liegt das sogenannte Lysat vor, das den gesamten Zellinhalt enthält.

Um einen funktionsfähigen Antikörper zu erhalten, werden bevorzugt weitere Aufreinigungsschritte durchgeführt. Nicht lösliche Bestandteile werden von den löslichen Bestandteilen abgetrennt, z. B. durch Zentrifugation oder Filtration, besonders bevorzugt durch eine Zentrifugationssequenz. Pigmenthaltige Bestandteile werden z. B. durch Filtration, Kühlung, chemische Fällung, Ionenaustauschchromatographie oder Größenaustauschchromatographie abgetrennt. Bevorzugt wird eine Sequenz aus Filtration, Kühlung, chemische Fällung, Ionenaustauschchromatographie oder Größenaustauschchromatographie genutzt, um Bestandteile sequenziell und effizient abzutrennen.

Die Antikörper werden durch sogenannte Affinitätschromatographie gereinigt. Dies kann mit typischen Aufreinigungsmethoden für Antikörper wie z. B. Protein A oder G oder über sogenannte Affinitäts-Tags erfolgen. Zusätzlich werden Verunreinigungen durch Tangentialflussfiltration oder Dead-End-Filtration und/oder Dialyse entfernt werden. Schließlich wird der Antikörper in eine geeignete Pufferlösung überführt.

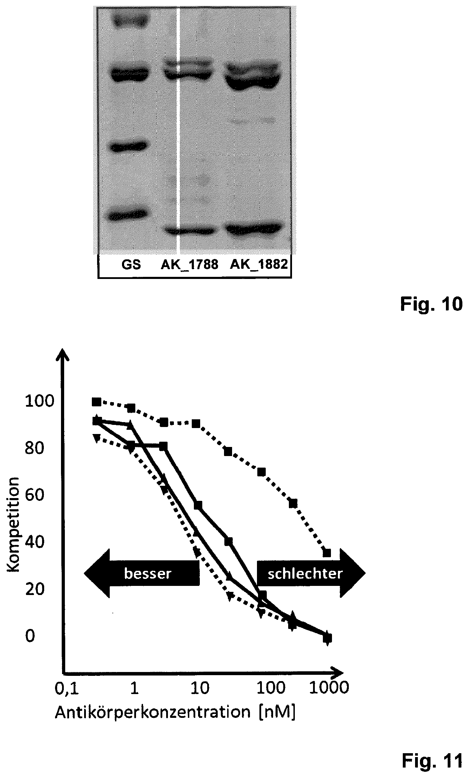

Um eine gute Leistung zu erzielen, müssen die hergestellten Antikörper einen bestimmten Reinheitsgrad aufweisen. Die Reinheit der hergestellten Antikörper kann durch die so genannte Polyacrylamid-Gelelektrophorese überprüft werden. **Fig. 10** zeigt ein Beispiel für zwei verschiedene erfindungsgemäße Anti-hCG-Antikörper, hier mit AK_1788 und AK_1882 bezeichnet, die einen hohen Reinheitsgrad aufweisen.

Die Gelelektrophorese ist eine Labortechnik, mit der Moleküle wie DNA, RNA und Proteine anhand ihrer Größe und Ladung getrennt und analysiert werden können. Dazu werden die Moleküle in eine Gelmatrix gegeben und ein elektrisches Feld angelegt, das die Moleküle dazu bringt, durch das Gel zu wandern. Kleinere Moleküle bewegen sich schneller und wandern weiter, was zu deutlichen Banden oder Mustern führt, die sichtbar gemacht werden können. Zu den technischen Vorteilen der Gelelektrophorese gehört ihre Fähigkeit, komplexe Molekülmischungen mit hoher Auflösung zu trennen. Sie ist vielseitig, kann verschiedene Arten von Molekülen aufnehmen und liefert qualitative und halbquantitative Informationen über deren Eigenschaften.

Ein Dot Blot ist eine Labortechnik, die in der Molekularbiologie und Immunologie eingesetzt wird, um spezifische Biomoleküle wie Proteine oder Nukleinsäuren (DNA oder RNA) in einer Probe nachzuweisen, zu analysieren und zu quantifizieren. Bei einem Dot Blot wird eine kleine Menge des Ziel-Biomoleküls auf einem festen Träger, in der Regel einer Membran, immobilisiert oder "gekleckst". Diese Immobilisierung kann durch direktes Tupfen der Probe auf die Membran erfolgen.

## BEZUGSZEICHENLISTE

1 Immunoassay / Vorrichtung
2 Probenauftragsbereich
3 Fangbereich
4 Konjugatbereich
5 Kontrollzone
6 Membran
7 Fixierungsbereich
8 Gehäuse
9 Prägenaht
10 Bruchstelle
11 Sequenzoptimierung der Nukleinsäuresequenz
12 Einbringen der Nukleinsäuresequenz in einen Vektor
13 Transformation
14 Screening-Verfahren
15 Verfahren zur Herstellung von rekombinanten Proteinen
16 Vektor
17 Zellen
18 Zellkultur

Ausführungsbeispiele

Anhand folgender Figuren und Ausführungsbeispiele wird die vorliegende Erfindung näher erläutert, ohne die Erfindung auf diese zu beschränken.

Dabei zeigt

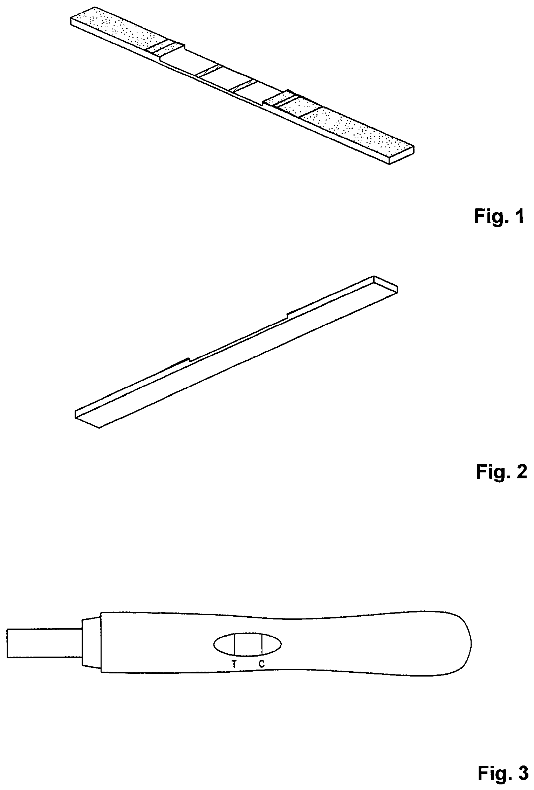

Fig. 1: einen Immunoassay, insbesondere einen Lateral-Flow-Immunoassay (Prüfstreifen) in einer schematischen Ansicht von oben;

Fig. 2: einen Immunoassay, insbesondere einen Lateral-Flow-Immunoassay (Prüfstreifen) in einer schematischen Ansicht von unten;

**Fig. 3:** die schematische Ansicht einer Vorrichtung, bei der ein Immunoassay, insbesondere ein Lateral-Flow-Immunoassay (Prüfstreipen), innerhalb eines Gehäuses angeordnet ist;

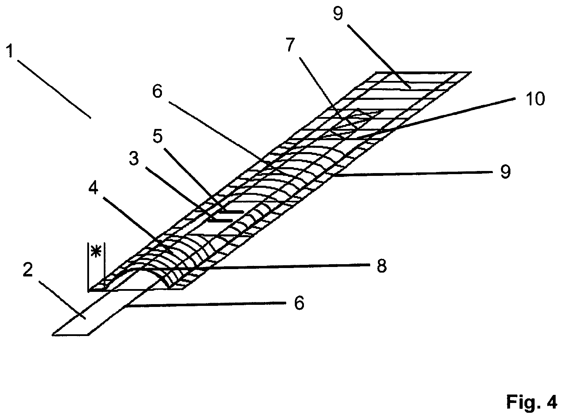

**Fig. 4:** die schematische Ansicht einer Vorrichtung, bei der das Gehäuse aus zwei übereinander angeordneten Lagen Papier oder Papierkarton gebildet ist;

**Fig. 5:** Glykananalyse zur Bestimmung der Zusammensetzung der Glykosylierung eines IgG (Antikörpers) in einer Hamsterzellkultur;

**Fig. 6:** Glykananalyse zur Bestimmung der Zusammensetzung der Glykosylierung des IgG (Antikörpers) aus Fig. 5 in Expi-Zellen (humane Zellkultur);

**Fig. 7:** Glykananalyse zur Bestimmung der Zusammensetzung der Glykosylierung des IgG (Antikörpers) aus Fig. 5 und 6 in Phaeodactylum.

**Fig. 8:** Eine schematische Übersicht über die Bereitstellung erfindungsgemäßer Antikörper.

**Fig. 9:** die Repetition von zwei Promotoren

**Fig. 10:** Polyacrylamid-Gelelektrophorese mit anti-hCG Antikörper; GS= Größenstandard, AK_1788 und AK_1882: Anti-hCG Antikörper, mit Coomassie eingefärbt.

**Fig. 11:** Ein Diagramm zum Vergleich der Bindungsaffinität zweier Kieselalgen Antikörper(formate) mit den Sequenzgleichen aus humaner Zellkultur (Expi[293F])

**Fig. 12:** ELISA zur Detektion des humanen beta-Choriongonadotropin (hCG) durch in Kieselalgen hergestellte Antikörper

**Fig. 13:** Einsatz von erfindungsgemäßen Antikörpern aus Kieselalgen als mit Meerrettichperoxidase markierte, sekundäre Antikörper zur Detektion von Primärantikörpern in einem Immunoblot (SDS-Page). mIgG: monoklonaler IgG gegen humanes Interleukin 15 aus Maus; mIgG_1: Ein in Ziege hergestellter, affinitätsgereinigter polyklonaler Antikörper (Goat-a-mouse-IgG-HrRP), gegen die Leichte und Schwere Kette von mouse-IgG gerichtet (Vergleichsantikörper); mIgG_2: erfindungsgemäßer Antikörper aus P. *tricornutum,* gegen die Schwere Kette von mouse-IgG gerichtet (erfindungsgemäßer Antikörper für Immunoassay)

**Fig. 14:** ELISA gegen den Herceptin 2 Rezeptor (Her2), durchgeführt mit einem Trastuzumab-Biosimilar aus Kaninchen (Trastuzumab A-C) und mit einem Biosimiliar aus der Kieselalge P. *tricornutum* (hIgG4_D-F).

**Fig. 15:** Dot Blot mit anti-hCG Antikörpern AK_1788 und AK_1882

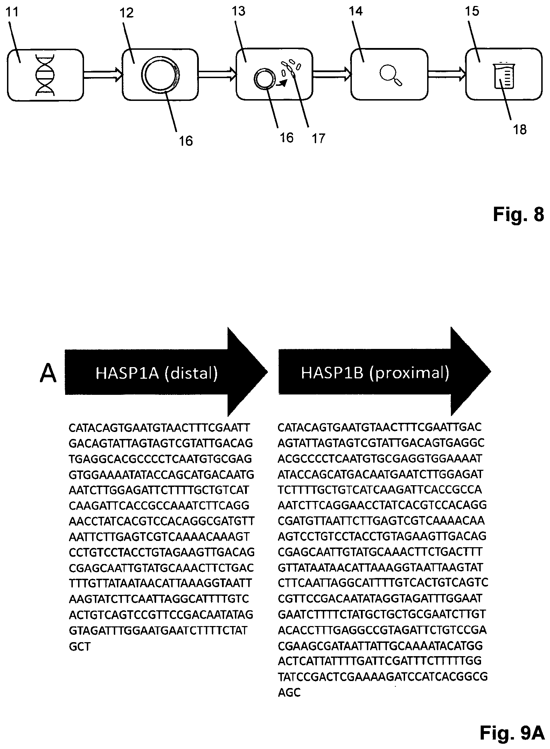

Die **Figur 8** zeigt eine schematische Übersicht über den Zusammenhang der Bereitstellung einer Kieselalge zur Produktion der rekombinanten Antikörper des Immunoassays. Alle dargestellten Teilschritte (einzeln oder in Kombination) führen zu einer Verbesserung/Optimierung der heterologen Produktion von Proteinen, insbesondere von Antikörpern, insbesondere in der Kieselalge *Phaeodactylum tricornutum.*

Zunächst erfolgt eine Sequenzoptimierung (11) einer Nukleinsäuresequenz. Diese kann beispielsweise eine Codonoptimierung und/oder die Verwendung besonders Proteaseresistenter genetischer Elemente (wie hierin beschrieben), wie beispielsweise Hinge-Regionen, die aus equinen IgGs (Immunoglobulin G) abgeleitet wurden, beinhalten.

Die Nukleinsäuresequenz wird anschließend in einen Vektor (16) (oder eine isolierte Nukleinsäure) eingebracht, wobei einzelne genetische Elemente und/oder komplette Expressionskassetten repetitiv verwendet werden. Des Weiteren werden spezielle induzierbare Promotoren verwendet, insbesondere ein Promotorelement aus der Nukleinsäuresequenz von SEQ ID NO: 1.

Durch die Nutzung bestimmter Signalsequenzen (wie hierin beschrieben) erfolgt die Expression der heterolog zu produzierenden Proteine in das Endoplasmatische Retikulum der Zellen, was zu einem Schutz der Proteine vor Proteasen und zu einer erfolgreichen Glykosylierung führt, wodurch die Ausbeute gesteigert und die Funktionalität der Proteine beibehalten wird.

In einem nächsten Schritt erfolgt die Transformation (13) des Vektors (oder der Nukleinsäure) in Zielzellen, bevorzugt in photosynthetisch aktiven Zellen, insbesondere Zellen einer einzelligen Pflanze oder Viridiplantae, bevorzugt Zellen von *Phaeodactylum tricornutum.* Die Transformation kann dabei ballistisch oder mittels Elektroporation in einem geeigneten Medium erfolgen.

Im Anschluss erfolgt ein Screening-Verfahren (14) zur Auswahl von Zellen mit einer erhöhten Expressionsrate einer Nukleinsäuresequenz. Dabei kann ein Screening mittels Reportergenen nach Hochleistungsproduzenten, sprich nach Zellen mit erhöhter Expressionsrate durchgeführt werden. Weiterhin kann die Korrelation zwischen der Expression der Reportergene und der Expressionsstärke der zu produzierenden Proteine ermittelt werden. Vorzugsweise werden sowohl für das Screening-Verfahren als auch für die Kulturüberwachung Multiwell-Platten verwendet.

Im Anschluss an das Screening-Verfahren kann ein Verfahren zur Herstellung von rekombinanten Proteinen, vorzugsweise rekombinanten Antikörpern, auf Basis der im Screening-Verfahren ermittelten Zellen mit erhöhter Expressionsrate erfolgen.

Die Herstellung rekombinanter Proteine (15) erfolgt dabei in einem Kulturmedium, welches auf die verwendeten genetischen Elemente, insbesondere auf die verwendeten Promotoren, abgestimmt ist. Auch die Kultivierungsbedingungen, wie beispielsweise Mindestlichtstärken und Belüftungsmengen, sind angepasst.

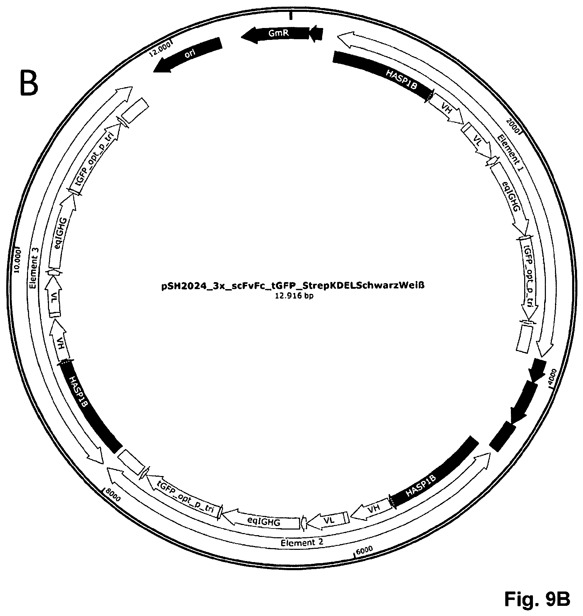

Sowohl in Bezug auf die Nukleinsäuresequenz als auch in Bezug auf den Vektor oder die isolierte Nukleinsäure führt die Verwendung repetitiver genetischer Elemente zu einer enormen Steigerung der Expressionsraten in *P. tricornutum.* **Figur 9A** zeigt die Repetition von zwei HASP1^mod-Promotoren. In **Figur 9B** ist beispielhaft eine Ausführungsform eines erfindungsgemäßen Vektors dargestellt, in welchen eine Expressionskassette repetitiv eingesetzt wurde, hier in Form einer dreifachen Kassette für die Produktion von Antikörpern im scFv-Fc-Format. Insbesondere führt diese repetitive Verwendung der Expressionskassette zu einer deutlichen Steigerung der Ausbeute sowie der Anzahl an Klonen bzw. dem Anteil an Klonen, die eine detektierbare Produktion zeigen, wodurch insgesamt der Prozessaufwand minimiert wird. Die Expressionskassette weist im dargestellten Ausführungsbeispiel ein Promotorelement (9.1), eine erste Transkriptionseinheit, welche für ein rekombinant zu erzeugendes Protein codiert, sowie eine zweite Transkriptionseinheit, welche für das Reportergen *gfp* codiert, auf, wobei die einzelnen genetischen Elemente beispielhaft an einer Expressionskassette angetragen sind, jedoch in den anderen Expressionskassetten ebenfalls vorliegen.

**Fig. 12** zeigt einen ELISA zur Detektion des humanen beta-Choriongonadotropin (hCG) durch in Kieselalgen hergestellte Antikörper. In **Fig. 12** wurde der ELISA nach diesem Verfahren durchgeführt:

Nach der Kopplung von humanem Choriongonadotropin (hCG) an die ELISA-Platte wurde nach der Absättigung mit einem tierproduktfreiem Blockingreagenz der gegen das hCG gerichtete erste Antikörper zugegeben, welcher in Kieselalgen hergestellt und über Affinitätschromatographie aufgereinigt wurde.

Nach dem Waschschritt wurde ein zweiter Kieselalgen-basierter Antikörper zur Verstärkung und ein mit dem Enzym HRP gekoppelter Antikörper zur Signalgenerierung eingesetzt, um nach Zugabe des Substrats die Nachweisreaktion zu starten.

In diesem ELISA wurden insgesamt 13 Versuchsreihen angesetzt, wovon zwei Proben ein starkes Signal zeigen sollen und elf als Kontrolle dienen, um ein aussagekräftiges Resultat zu erhalten. In Tabelle 1 sind die entsprechenden Ansätze aufgelistet, wobei die Reihenfolge von links nach rechts den Arbeitsschritten entspricht. Die Anzahl der Schritte kann variieren. In diesem Fall wurden 5 Schritte durchgeführt. Zuerst wurde das Antigen, hier hCG, an die Platte gebunden. Anschließend wurden freie Bindestellen mittels sog. Blocklösung besetzt. Ab dem 3. Schritt unterschieden sich die Zugaben, siehe Tabelle 1. Diese verschiedenen Zugaben ermöglichen die Charakterisierung der Antikörper und dienen unter anderem dazu, unspezifische Bindungen auszuschließen.

Insgesamt wurden drei verschiedene Antikörper, die in *Phaeodactylum tricornutum* produziert werden, in diesem beispielhaften ELISA verwendet:

Zum einen wurden zwei verschiedene Antikörper, die gegen die Beta-Untereinheit von hCG gerichtet sind und einem menschlichen IgG4 entsprechen (AK_1788 und AK_1892) eingesetzt. Zum anderen wurde ein aus Kieselalgen gewonnener, sekundärer Antikörper verwendet, der einem gegen menschliches IgG4 gerichteten Maus-Antikörper entspricht (AK_2073). In diesem Beispiel erfolgte der Nachweis mit einem polyklonalen Antikörper, der gegen Maus-IgG gerichtet und an eine Meerrettichperoxidase (HRP) konjugiert ist (G-α-mIgG-HRP).

In Tabelle 1 sind die mit dem Bild verbundenen Antikörperzusätze aufgeführt. Ein Signal wird nur in den Proben 9 und 13 erwartet, alle anderen Proben dienen der Kontrolle.

Tabelle 1: Gruppen für ELISA mit aus Kieselalgen gewonnen Antigenen. anti-mIgG-HRP = Mit HRP markierter Antikörper, der gegen den Antikörper 2073 (AK_2073) gerichtet ist. AK_1788 und AK_1882 sind Antikörper, die gegen hCG gerichtet sind, AK_2073 ist ein Antikörper, der gegen die Antikörper 1788 und 1882 gerichtet ist.

| Gruppe | Coating | 1. Zugabe | 2. Zugabe | 3. Zugabe ; | 4. Zugabe |
|---|---|---|---|---|---|
| 1 | Blocklösung | Blocklösung | Blocklösung | Blocklösung | |
| 2 | hCG | Blocklösung | Blocklösung | Blocklösung | |
| 3 | hCG | Blocklösung | Blocklösung | Blocklösung | anti-mIgG-HRP |

(fortgesetzt)

| Gruppe | Coating | 1. Zugabe | 2. Zugabe | 3. Zugabe ; | 4. Zugabe |
|---|---|---|---|---|---|
| 4 | hCG | Blocklösung | Blocklösung | AK_2073 | |
| 5 | hCG | Blocklösung | Blocklösung | AK_2073 | anti-mIgG-HRP |
| 6 | hCG | Blocklösung | AK_1788 | Blocklösung | |
| 7 | hCG | Blocklösung | AK_1788 | Blocklösung | anti-mIgG-HRP |
| 8 | hCG | Blocklösung | AK_1788 | AK_2073 | |
| **9** | hCG | **Blocklösung** | **AK_1788** | **AK_2073** | **anti-mIgG-HRP** |
| 10 | hCG | Blocklösung | AK_1882 | Blocklösung | |
| 11 | hCG | Blocklösung | AK_1882 | Blocklösung | anti-mIgG-HRP |
| 12 | hCG | Blocklösung | AK_1882 | AK_2073 | |
| **13** | **hCG** | **Blocklösung** | **AK_1882** | **AK_2073** | **anti-mIgG-HRP** |

Aus **Fig. 12** ist ersichtlich, dass nur die in Tabelle 1 hervorgehobenen Gruppen 9 und 13 ein starkes Signal mit einer Intensität von 0,57 bzw. 0,54 aufweisen. Dies zeigt, dass der erste und zweite Antikörper eine hohe Affinität zum Antigen bzw. zum ersten Antikörper haben. Durch Zugabe von G-$\alpha$-mIgG-HRP als Nachweis-Antikörper wurde gezeigt, dass diese Antikörper selektiv für den Nachweis von humanem beta-Choriongonadotropin (hCG) sind. Dabei zeigen alle anderen Kontrollgruppen keinen Wert von über 0,25. Dies lässt die Aussage zu, dass sowohl die Antikörper AK_1788 als auch AK_1892 das Antigen, hier hCG, selektiv binden.

Für den im LFA benötigten sogenannten Kontroll-Antikörper 2073, der auf der Kontrolllinie platziert ist, kann gezeigt werden, dass er die Antikörper AK_1788 und AK_1892 wie gewünscht binden kann.

Somit sind die hier gezeigten Antikörper vollständig und funktionsfähig für die Konstruktion eines LFA, in diesem Fall eines Schwangerschaftstests, und ermöglichen den Nachweis einer Schwangerschaft sowie die Färbung der Kontrolllinie eines LFA.

**Fig. 13** zeigt einen beispielhaften Einsatz eines Antikörpers in einem Immunoassay (Immunoblot) in der SDS PAGE Variante. Hier werden die Antikörper aus Kieselalgen als sekundäre Antikörper verwendet, die mit Meerrettichperoxidase für den Nachweis von primären Antikörpern markiert sind. In Fig. 13 steht mIgG für ein monoklonales IgG gegen menschliches Interleukin 15 aus der Maus, und Pt ist die Abkürzung für einen Proteinextrakt aus der Kieselalge *Phaeodactylum tricornutum* (Pt), der als Negativkontrolle verwendet wird, um zu zeigen, dass die Antikörper keine Proteine aus den Kieselalgen erkennen. Sowohl mIgG als auch Pt wurden über eine SDS-PAGE aufgetrennt, die aufgetrennten Proteine aus dem Gel anschließend mittels Western Blot auf eine Membran übertragen und im Anschluss der mIgG mit einem kommerziellen Vergleichsantikörper (mIgG_1) sowie zwei unterschiedlichen Konzentrationen eines Antikörpers nachgewiesen.

Der verwendete Vergleichsantikörper ist mIgG_1, ein kommerzieller, polyklonaler, affinitätsgereinigter Antikörper (Goat-$\alpha$-mouse-IgG-HrRP), der in einer Ziege hergestellt wird, gegen die Leichte und die Schwere Kette des Maus-IgG gerichtet und mit Meerrettichperoxidase (HRP) gekoppelt ist (Vergleichsantikörper). Der Antikörper mIgG_2 (Pt-$\alpha$-Maus-IgG-HRP) wird in zwei Konzentrationen (0,75 $\mu$g/mL und 0,15 $\mu$g/mL) verwendet. Dieser wurde aus der Kieselalge *Phaeodactylum tricornutum* gewonnen und ist gegen die Schwere Kette von Maus-IgG gerichtet und mit Meerrettichperoxidase (HRP) gekoppelt. Als Längenstandard (M) wurde BlueStar der Firma Nippon Genetics verwendet.

Der in Ziegen erzeugte Antikörper Goat-$\alpha$-mouse-IgG-HRP erkennt sowohl die leichte als auch die Schwere Kette von Maus-mIgG, weshalb neben zahlreichen unspezifischen, zwei starke spezifische Signale zu sehen sind (Pfeile). Der Antikörper aus Kieselalgen (mIgG_2) ist nur gegen die Schwere Kette (HC, nur oberer Pfeil) gerichtet, weshalb die Leichte Kette, die beim Vergleichsantikörper die untere spezifische Bande erzeugt, nicht angezeigt wird. Der Antikörper aus Kieselalgen (mIgG_2) ist nur gegen die Schwere Kette (HC, nur oberer Pfeil) gerichtet, weshalb die untere Bande nicht erkannt wird. Der Antikörper aus Kieselalgen liefert schon in geringen Mengen ähnlich deutliche und vor allem spezifischere Signale. Vor allem mit dem kommerziellen tierischen Antikörper (mIgG_1) treten mehrere unspezifische Banden (*) auf. Die obersten Banden sind noch komplette IgGs (▼), bei denen die für die SDS-PAGE charakteristische Denaturierung nicht zu einer Auftrennung des IgGs in die Leichte und Schwere Kette geführt hat. So zeigen die Antikörper nicht nur eine starke Signalverstärkung bei geringerer Konzentration, sondern auch eine höhere Spezifität im Vergleich zu den Zielantikörpern, weshalb sie eine spezifischere Alternative zu tierischen Antikörpern darstellen. Diese erhöhte Empfindlichkeit ermöglicht eine geringere Menge an benötigten Antikörpern, während die höhere Spezifität eine zuverlässigere Bestimmung und die Vermeidung von falsch-positiven Ergebnissen ermöglicht. Hierbei konnte ebenfalls gezeigt werden, dass die Antigene keine Affinität zu den kieselalgenspezifischen Proteinen aufweisen, was durch die fehlenden Banden in den aufgetrennten Proteinextrakten aus P. *tricornutum* (Pt) demonstriert wird.

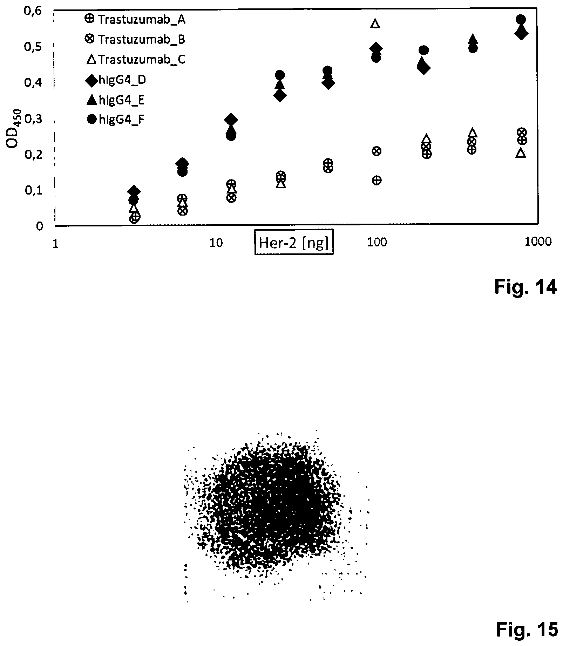

**Fig. 14** zeigt einen ELISA von tierischen sowie Antikörpern gegen den Herceptin 2 Rezeptor (Her2), durchgeführt mit einem Trastuzumab-Biosimilar aus Kaninchen (Trastuzumab A-C) und mit einem Biosimiliar aus der Kieselalge *Phaeodactylum tricornutum* (hIgG4_D-F).

Der OD-Wert bezieht sich auf den Wert der optischen Dichte. Er ist ein Maß für die Absorption von Licht durch eine Probe in einer Mikroplattenvertiefung. Der OD-Wert wird verwendet, um das Vorhandensein oder die Konzentration eines bestimmten Moleküls, in diesem Fall eines Antigens, in der getesteten Probe zu quantifizieren. Höhere OD-Werte weisen im Allgemeinen auf eine höhere Konzentration des Zielmoleküls in der Probe hin, während niedrigere Werte niedrigere Konzentrationen anzeigen. Diese Messung ist eine entscheidende Komponente bei der Bewertung der Ergebnisse von ELISA-Experimenten und der Bestimmung der Stärke der Reaktion zwischen Antigen und Antikörper.

Die OD-Werte in diesem Beispiel zeigen, dass über alle Konzentrationsbereiche die Antikörper, die aus der Kieselalge *Phaeodactylum tricornutum* gewonnen wurden (hIgG4_D-F) eine höhere spezifische Aktivität gegenüber den Antigen Herceptin 2 Rezeptor zeigen als die tierischen Analoga (Trastuzumab A-C). Dies ist wahrscheinlich auf eine höhere Reinheit der Antikörper zurückzuführen, die unter anderem auch in **Fig. 5-7** (Glykosylierungsmuster), **Fig. 13** (SDS-Page Immunoblot) demonstriert ist. Die höhere Aktivität ermöglicht einen empfindlicheren Nachweis der Antigene und damit eine Herabsetzung der Nachweisgrenze. Aufgrund der niedrigen Konzentration können entsprechende Antigene z. B. früher im Krankheitsverlauf nachgewiesen werden und so zu einer schnellen Erkennung und Behandlung beitragen, die z. B. Infektionsketten unterbrechen kann.

**Fig. 15** zeigt einen Dot Blot Test. In dem Dot Blot Assay wird einer der Antikörper (AK_1788, gegen hCG gerichtet) mit kolloidalen Gold konjugiert und ein anderer Antikörper (AK_1882, gegen ein anderes Epitop auf hCG gerichtet) kreisförmig auf einer geeigneten Membran aufgebracht. Anschließend werden die Membran und die mit kolloidalen Gold markierten Antikörper gemeinsam mit einer hCG-haltigen Lösung inkubiert. Die verwendeten Anti-hCG-Antikörper aus dem ELISA **(Fig. 13)** werden also in einem Aufbau getestet, der einem LFA-Modell entspricht. Sowohl die membrangebundenen als auch die mit kolloidalen Gold markierten Antikörper binden gleichzeitig das hCG aus der Lösung. Wenn beide Antikörper in der Lage sind, das hCG zu binden, kommt es an der Position des membrangebundenen Antikörpers zu einer deutlichen Verfärbung im Vergleich zum umgebenden Bereich. In Fig. 15 ist diese Verfärbung mit den beiden Anti-hCG-Antikörpern dargestellt. Dies zeigt, dass beide Antikörper gleichzeitig an das hCG-Molekül binden und somit hCG unter Verwendung eines typischen LFA-Aufbaus nachweisen können.

**Beispiel** 1: Produktion von Antikörper aus Kieselalge und Aufreinigung für Immunoassay

Das Ausführungsbeispiel, dessen Ablauf in **Fig. 8** skizziert ist, zeigt die Produktion eines Antikörpers mit dem Verfahren. Dieser Antikörper im IgG-Format ist gegen equines Interleukin 31 gerichtet und wurde mit mehr als 160 mg aufgereinigtem eqIgG * L$^{-1}$ Zellkultur innerhalb einer 14-tägigen Kulturdauer produziert.

In einem ersten Schritt wird die Codon-Nutzung an *P. tricornutum* angepasst. Im vorliegenden Fall stammt die Ausgangssequenz aus einer humanen scFv-Bank und wurde vor Einsatz in den Kieselalgen Codon-optimiert. Die benötigten genetischen Elemente, typischerweise die variable Region der leichten Kette ($V_L$) sowie die variable Region der schweren Kette ($V_H$) wurden von der Firma IDT-DNA (Coralville, Iowa) synthetisiert, so dass sie optimal in die erstellten Vektoren passen **(Fig. 9B).** Dabei wurden auch störende Restriktionsstellen entfernt, bzw. modifiziert. Eine Variante der Vektoren für die Produktion von Antikörpern im scFv-Fc-Format ist in **Fig. 9B** dargestellt. Hier kann die variable Region der leichten Kette (kappa- oder lambda-Format ($V_{L\kappa}$ oder $V_{L\lambda}$)) sowie die variable Region der schweren Kette ($V_H$) eingesetzt werden. In diesem Beispiel stammen die konstanten Bereiche beider Ketten aus dem Pferd. Es wurden auch Konstrukte generiert, die konstante Antikörperbereiche aus anderen Wirtsorganismen (z. B. Maus oder Mensch) enthalten. Weitere Varianten der Vektoren sind für die heterologe Produktion anderer Formate wie Fab oder scFv-Fc hergestellt und erfolgreich eingesetzt worden. Das in **Fig. 9B** dargestellte Beispiel enthält das fertige Vektorkonstrukt und neben den in *P. tricornutum* einzubringenden Elementen auch bakterielle, genetische Elemente, die der Klonierung in *E. coli* dienen (colE1-Origin und Gentamycin-Resistenzgen). Im Konstrukt wurde das Gene of Interest in drei Kopien eingesetzt. Nach der ballistischen oder auf Elektroporation beruhenden Transformation von *P. tricornutum* müssen die erhaltenen Klone nicht nur auf die Aufnahme der Antikörpergene hin überprüft werden, sondern auch die schnelle Identifizierung von Klonen, die das eingebrachte Antikörpergen besonders stark exprimieren. Das zugrundeliegende erfindungsgemäße Screeningverfahren ermöglicht die Korrelation der durch *gfp* (Green Fluorescent Protein) bewirkten Fluoreszenz, welche in einem speziellem Mikrotiterplatten-Reader gemessen wird, mit der später zu erwartenden Menge an gebildetem Antikörper.

Im Vergleich zu im Stand der Technik beschriebenen Produktionsraten von maximal 3 mg Antikörper * L$^{-1}$ Kultur konnte in dem System aus 1 L Kultur 160 mg aufgereinigter Antikörper erhalten werden. Um dies zu erreichen, wurden neue Medienzusammensetzungen, eine Belüftung mit mehr als 3 L * min$^{-1}$ Druckluft sowie eine Beleuchtung mit einer Lichtstärke von 100 - 1000 W * m$^{-2}$ in einer Reaktorsäule eingesetzt. Eine beispielhafte Medienzusammensetzung ist in Tabelle 2 angegeben:

**Tabelle 2:** Medienzusammensetzung

| Substanz | Konzentration |
|---|---|
| Tris-HCl pH 8 | 10 mM |
| $NaNO_3$ | 30 mM |
| Vitamine für P. *tricornutum* | Über 10x (f/2 Medium) |
| Spurenelemente für P. *tricornutum* | Über 10x (f/2 Medium) |
| Glycerin | Mindestens 100 mM |
| $NH_2PO_4$ | Mindestens 360 $\mu$M |

Nach typischerweise vierzehn bis zwanzig Tagen Fed-Batch-Kultur können die Kieselalgen geerntet und aufgeschlossen werden.

Die Aufreinigung folgt dem klassischen Verfahren, d. h. nach der Lyse und Zentrifugation und/oder einer Ultrafiltration erfolgt die Aufreinigung entweder über Protein A, Protein G oder über die eingesetzten Tag-Sequenzen, beispielsweise den 6xHis-Tag. Hier liegt ein weiterer Vorteil des Immunoassays, denn im Gegensatz zu höheren Pflanzen die transient oder stabil Antikörper produzieren, enthalten die Kieselalgen keine die Aufreinigung der Antikörper enorm erschwerenden Fasern. Die Aufreinigung entspricht daher zu großen Teilen dem Verfahren, wie es beispielsweise für tierische Zellkulturen, wie CHO-Zellen, eingesetzt wird und ist einen Fachmann geläufig und wird im Folgenden beschrieben. Die Zellen des Produktionsklons, in diesem Beispiel einer Kieselalge, werden durch einen so genannten schonenden Aufschluss zerstört, wobei das Produkt, der Antikörper im Sinne der vorliegenden Erfindung, geschützt wird. Zu den schonenden Methoden gehören z. B. Hochdruck, elektrische Spannung, Ultraschall oder Aufschluss durch Kollision in sogenannten Schwingmühlen, in diesem Fall wurde eine Kombination aus Manton-Gaulin-Homogenisator und folgender Ultraschall-Behandlung im Ultrasonicator durchgeführt. Nach dem Aufschluss liegt das sogenannte Lysat vor, das den gesamten Zellinhalt enthält. Um einen funktionsfähigen Antikörper zu erhalten, werden Aufreinigungsschritte durchgeführt. Nicht lösliche Bestandteile werden von den löslichen Bestandteilen abgetrennt, z. B. durch Zentrifugation oder Filtration, in diesem Fall eine Zentrifugationssequenz. Pigmenthaltige Bestandteile werden durch eine Sequenz aus Filtration, Kühlung, chemische Fällung, Ionenaustauschchromatographie oder Größenaustauschchromatographie abgetrennt.

Die Antikörper werden durch sogenannte Affinitätschromatographie gereinigt. Diese erfolgt entweder mit einer typischen Aufreinigungsmethode für Antikörper, wie z. B. Protein A oder G, oder über sogenannte Affinitäts-Tags. Zusätzlich werden Verunreinigungen durch Tangentialflussfiltration oder Dead-End-Filtration und/oder Dialyse entfernt. Schließlich wird der Antikörper in eine geeignete Pufferlösung überführt.

Um eine gute Leistung zu erzielen, müssen die hergestellten Antikörper einen bestimmten Reinheitsgrad aufweisen. Die Reinheit der hergestellten Antikörper wird in diesem Beispiel durch die so genannte Polyacrylamid-Gelelektrophorese überprüft. **Fig. 10** zeigt ein Beispiel für zwei verschiedene Anti-hCG-Antikörper, hier mit A-hCG-1 und A-hCG-12 bezeichnet, die einen hohen Reinheitsgrad aufweisen.

**Beispiel 2:** Bereitstellung eines Schwangerschaftstests

Der Lateral-Flow-Schwangerschaftstest verwendet intrazellulär produzierte Antikörper aus der Kieselalge *Phaeodactylum tricornutum.* Diese Antikörper weisen bei der Herstellung eine Konzentration von etwa 160 -360 mg/L pro Liter Kultur auf. Diese Antikörper sind speziell auf das menschliche Choriongonadotropin (hCG) ausgerichtet, das aus einer $\alpha$-Untereinheit mit 92 Aminosäuren und einer $\beta$-Untereinheit mit 145 Aminosäuren besteht. Der Test umfasst einen ersten Antikörper, ein Fängerantikörper, einen zweiten Antikörper, ein Detektionsantikörper und einen weiteren Antikörper, ein Kontrollantikörper.

Die Nachweisantikörper zeichnen sich dadurch aus, dass sie mit kolloidalen Goldnanopartikeln markiert sind. Der erste immobilisierte Antikörper, der sich in der Fängerzone befindet, fungiert als Fängerantikörper. Der zweite Antikörper, der sich in der Konjugationszone befindet und als Detektionsantikörper bezeichnet wird, ist ebenfalls gegen das Antigen gerichtet, vorzugsweise gegen ein anderes Epitop des Antigens als der Detektionsantikörper. Dieser zweite Antikörper ist mit einem Markerpartikel konjugiert, in diesem Fall mit kolloidalen Goldnanopartikeln.

In einer bevorzugten Ausführungsform des Immunoassay wird der erste Antikörper und/oder der zweite Antikörper, insbesondere der mobilisierte zweite Antikörper, mit einem Farbstoff und/oder einem optisch aktiven Nanopartikel, insbesondere einem Goldnanopartikel, markiert.

Ein weiterer Antikörper, der sich vorzugsweise in der Kontrollregion (Kontrollzone) befindet und immobilisiert ist, richtet sich gegen einen Detektionsantikörper.

Die gereinigten Antikörper, einschließlich des ersten, zweiten und nachfolgenden Antikörpers, werden mit einem pflanzlichen Deckprotein (Kieselalgen-exprimiertes BSA) kombiniert und auf eine Nitrocellulosemembran aufgebracht. Mit dieser Konfiguration wird ein Lateral-Flow-Immunoassay geschaffen, der einen Probenauftragsbereich, einen Konjugatbereich und einen auf der Membran integrierten Einfangbereich aufweist. Probenauftragsbereich und ein Fangbereich sind auf der Membran über einen Strömungsweg miteinander fluidverbunden sind, wobei der Konjugatbereich sich im Strömungsweg befindet. Dieses Design erleichtert die Durchführung des Tests und die Interpretation der Ergebnisse.

Diese Membran ist von einem Gehäuse im Sinne der vorliegenden Erfindung umgeben, die überwiegend (zu mindestens 90 %) aus Zellulosefasern besteht, die einerseits wasserbeständig ist (zumindest für den Transport und die Nutzungsdauer) und andererseits aus nachhaltigen, vorzugsweise wiedergewonnenen Pflanzenfasern hergestellt wird und biologisch abbaubar ist (z. B. durch Kompostierung).

Für eine einfache Nutzung als PoCT werden an dem Gehäuse an der Membran Markierungen (Symbole, Zeichen, geometrische Formen) angebracht, die die einfache Interpretation des Immunoassay-Ergebnisses erlauben. Insbesondere in der Ausführungsform eines Kits, in der neben dem erfindungsgemäßen Assay eine Anleitung bereitgestellt wird, ist die einfache Handhabung auch für nicht medizinisches Personal gegeben.

## Patentansprüche

1.  **Immunoassay** zum Nachweisen eines biologisch aktiven Antigens, insbesondere eines Hormons, Proteins oder Arzneistoffs in einer biologischen Probe eines Individuums, aufweisend:

    - einen Probenauftragsbereich zum Auftragen einer biologischen Probe eines Individuums, wobei die biologische Probe vorzugsweise Urin, Vollblut, Speichel, Milch oder Serum ist;
    - einen Fangbereich, wobei der Fangbereich einen immobilisierten ersten Antikörper aufweist, der gegen das biologisch aktive Antigen, insbesondere gegen das Hormon, das Protein, das Peptid oder den Arzneistoff, gerichtet ist;
    - einen Konjugatbereich, wobei der Konjugatbereich einen zweiten Antikörper aufweist, der gegen das biologisch aktive Antigen, insbesondere gegen das Hormon, das Protein, das Peptid oder den Arzneistoff, gerichtet ist;

    **dadurch gekennzeichnet, dass**

    der erste Antikörper und der zweite Antikörper ein rekombinanter Antikörper ist, der aus einer Kieselalge oder einzelligen Pflanze gewonnen wurde,
    wobei der Immunoassay zumindest einen weiteren Antikörper und/oder ein Hilfsprotein aufweist,
    wobei das Hilfsprotein als Blocker freie Oberflächen eines Reaktionsgefäßes oder einer Membran absättigt,
    wobei der weitere Antikörper und das Hilfsprotein aus einer Kieselalge und/oder Viridiplantae und/oder einzelligen Pflanze gewonnen wurde, und
    wobei der rekombinante Antikörper in einer Reinheit von mindestens 90 % bereitgestellt wird, und
    wobei der Immunoassay vegan ist.

2.  Immunoassay nach Anspruch 1, wobei der rekombinante Antikörper in einer Konzentration von 20-1000 mg/L, bevorzugter 30-800 mg/L, alternativ mindestens 30-160 mg/L der Kultur einer Kieselalge gewonnen wird.

3.  Immunoassay nach einem der Ansprüche 1 oder 2, wobei die Glykosylierung des ersten Antikörpers und/oder des zweiten Antikörpers ein modifiziertes Glykosylierungsmuster, im Vergleich zum entsprechenden nativen Antikörper aufweist, vorzugsweise weist die Glykosylierung ein homogeneres Muster mit einem Homogenitätsfaktor im Bereich von 1 bis 3 auf, besonders bevorzugt weist die Glykosylierung ein homogenes, mannosereiches N-Glykanmuster mit einem Homogenitätsfaktor im Bereich vorzugsweise im Bereich von 1 bis 3 auf.

4.  Immunoassay nach einem der Ansprüche 1 bis 3,
    wobei die Aminosäuresequenz des ersten Antikörpers und/oder des zweiten Antikörpers

    a) einen vertebralen, bevorzugt mammalen, besonders bevorzugt einen humanen, Antikörper aufweist; oder
    b) eine Aminosäuresequenz aufweist oder aus dieser besteht, die mindestens 80 %, vorzugsweise zumindest 85 %, besonders bevorzugt zumindest 90 %, ganz besonders bevorzugt zumindest 95 %, insbesondere zumindest 97 % Sequenzidentität zu homologen Sequenzbereichen eines vertebralen und/oder mammalen und/oder humanen Antikörper aufweist.

5. Immunoassay nach einem der Ansprüche 1 bis 4, wobei der erste Antikörper und/oder der zweite Antikörper ein Antikörper ist, der gegen das humane Choriongonadotropin (hCG) gerichtet ist.

6. Immunoassay nach einem der Ansprüche 1 bis 5, wobei das biologisch aktive Antigen, ein Teil des Epitopes eines Virus, bevorzugt eines pathogenen Virus, beispielsweise Influenza, SARS-CoV-2, RSV, Adenovirus, Strep A, Norovirus, Rotavirus, HIV, ist.

7. Immunoassay nach einem der Ansprüche 1 bis 6, wobei es sich bei dem biologisch aktiven Antigen um eine Tumor-assoziierte Sequenz handelt, bevorzugt um einen HLA-Komplex und/oder ein sequenziertes Teil eines Tumor-Epitops und/oder einen Tumor-Marker, beispielsweise IFN-$\gamma$, IL-8, PSA, CEA, AFP, DCP, CA 125, HER2/neu.

8. Immunoassay nach einem der Ansprüche 1 bis 7, wobei es sich bei dem biologisch aktiven Antigen um eine charakteristische Sequenz zur Identifikation einesProteins, vorzugsweise eines Enzym-Tags, besonders bevorzugt ausgewählt aus der Liste bestehend aus His$_6$-Tag, Strep-Tag, c-Myk-Tag, Flag-Tag und GST-Tag.

9. Immunoassay nach einem der Ansprüche 1 bis 8, wobei es sich bei dem biologisch aktiven Antigen um eine Sequenz handelt, die mit Ernährungsparametern assoziiert ist, beispielsweise Transcobalamin II, Ferritin, Homocystein, Eicosapentaensäure (EPA) und Docosahexaensäure (DHA), oder Calcitriol.

10. Immunoassay nach einem der Ansprüche 1 bis 9, wobei der Immunoassay ein Lateral-Flow-Immunoassay ist, welcher fluidverbunden mindestens einen Probenauftragsbereich, einen Konjugatbereich und einen Fangbereich, die auf einer Membran fluidverbunden angeordnet sind, bereitstellt.

11. Immunoassay nach einem der Ansprüche 1 bis 10, wobei der Immunoassay als ein Enzyme-Linked Immunosorbent Assay (ELISA) Immunoassay ausgeführt ist, wobei mindestens

   a. ein Probenauftragsbereich zum Auftragen einer biologischen Probe bereitgestellt wird, welcher
   b. von einem Fangbereich, der als eine Gefäßbegrenzung und/oder Teil einer Gefäßbegrenzung ausgeführt ist, bevorzugt als Mikrotiterplatte, begrenzt ist, und
   c. einem Konjugatbereich, welcher räumlich im Probenauftragsbereich angeordnet ist.

12. **Vorrichtung,** aufweisend:

   - einen Behälter, insbesondere ein Gehäuse, und
   - einen darin angeordneten Immunoassay nach einem der Ansprüche 1 bis 11, insbesondere einen Lateral-Flow-Immunoassay.

13. Vorrichtung nach Anspruch 12, bei dem der Behälter, bevorzugt ein Gehäuse, aus nachhaltigen und wasserbeständigen Materialien, vorzugsweise Papier und/oder Faserguss gestaltet ist.

14. **Verfahren** zum Nachweis eines biologisch aktiven Proteins in einer biologischen Probe, bevorzugt Urin, Vollblut, Speichel, Milch oder Serum, umfassend die folgenden Schritte:

   a. Gewinnen einer biologischen Probe von einem Individuum;
   b. Analysieren der biologischen Probe unter Verwendung eines Immunoassays nach einem der Ansprüche 1 bis 11, der derart eingerichtet ist, dass dieser das biologisch aktive Protein nachweist.

15. Verwendung eines **Hilfsproteins** als Abdeck-, Maskier- und/oder Unterstützungsprotein in einem Immunoassay nach einem der Ansprüche 1 bis 11,
   **dadurch gekennzeichnet, dass**
   das Hilfsprotein aus einer Kieselalge gewonnen wird und vegan ist.

**Claims**

1. **Immunoassay** for detecting a biologically active antigen, particularly a hormone, protein or pharmaceutical substance in a biological sample of an individual, provided with:

- a sample application area for applying a biological sample of an individual, wherein the biological sample is preferably urine, whole blood, saliva, milk or serum;
- a capture area, wherein the capture area is provided with an immobilized first antibody that is directed against the biologically active antigen, in particular against the hormone, the protein, the peptide or the pharmaceutical substance;
- a conjugate area, wherein the conjugate area is provided with a second antibody directed against the biologically active antigen, particularly against the hormone, protein, peptide or pharmaceutical substance;

**characterized in that**

the first antibody and the second antibody is a recombinant antibody obtained from a diatom or unicellular plant, wherein the immunoassay comprises at least a further antibody and/or an auxiliary protein, wherein the auxiliary protein saturates free surfaces of a reaction vessel or a membrane as a blocker, wherein the further antibody and the auxiliary protein were obtained from a diatom and/or Viridiplantae and/or unicellular plant, and wherein the recombinant antibody is provided in a purity of at least 90%, and wherein the immunoassay is vegan.

2. Immunoassay according to claim 1, wherein the recombinant antibody is obtained in a concentration of 20-1000 mg/L, preferably 30-800 mg/L, alternatively at least 30-160 mg/L of the culture of a diatom or unicellular plant.

3. Immunoassay according to one of claims 1 or 2, wherein the glycosylation of the first antibody and/or the second antibody has a modified glycosylation pattern compared to the corresponding native antibody, preferably the glycosylation has a more homogeneous pattern with a homogeneity factor in the range from 1 to 3, particularly preferred the glycosylation has a homogeneous, mannose-rich N-glycan pattern with a homogeneity factor in the range preferably in the range from 1 to 3.

4. Immunoassay according to any one of claims 1 to 3, wherein the amino acid sequence of the first antibody and/or the second antibody

a) comprises a vertebral, preferably mammalian, particularly preferred a human antibody; or
b) comprises or consists of an amino acid sequence which has at least 80 %, preferably at least 85 %, particularly preferably at least 90 %, most preferably at least 95 %, in particular at least 97 % sequence identity to homologous sequence regions of a vertebral and/or mammalian and/or human antibody.

5. Immunoassay according to any one of claims 1 to 4, wherein the first antibody and/or the second antibody is an antibody directed against human chorionic gonadotropin (hCG).

6. Immunoassay according to any one of claims 1 to 5, wherein the biologically active antigen is a part of the epitope of a virus, preferably a pathogenic virus, for example influenza, SARS-CoV-2, RSV, adenovirus, Strep A, norovirus, rotavirus, HIV.

7. Immunoassay according to any one of claims 1 to 6, wherein the biologically active antigen is a tumor-associated sequence, preferably an HLA complex and/or a sequenced part of a tumor epitope and/or a tumor marker, for example IFN-$\gamma$, IL-8, PSA, CEA, AFP, DCP, CA 125, HER2/neu.

8. Immunoassay according to any one of claims 1 to 7, wherein the biologically active antigen is a characteristic sequence for identifying a protein, preferably an enzyme tag, particularly preferred selected from the list consisting of His$_6$-tag, Strep-tag, c-Myk-tag, Flag-tag and GST-tag.

9. Immunoassay according to any one of claims 1 to 8, wherein the biologically active antigen is a sequence associated with nutritional parameters, for example transcobalamin II, ferritin, homocysteine, eicosapentaenoic acid (EPA) and docosahexaenoic acid (DHA), or calcitriol.

10. Immunoassay according to any one of claims 1 to 9, wherein the immunoassay is a lateral flow immunoassay providing fluid-connected at least a sample application area, a conjugate area and a capture area fluid-connected on a membrane.

11. Immunoassay according to any one of claims 1 to 9, wherein the immunoassay is carried out as an enzyme-linked immunosorbent assay (ELISA) immunoassay, wherein at least

    a. a sample application area for applying a biological sample is provided, which is confined by
    b. a capture area which is designed as a vessel boundary and/or part of a vessel boundary, preferably as a microtiter plate, and
    c. a conjugate area, which is spatially arranged in the sample application area.

12. **Device,** provided with:

    - a container, particularly a housing, and
    - an immunoassay according to any one of claims 1 to 11 arranged therein, particularly a lateral flow immunoassay.

13. Device according to claim 12, wherein the container, preferably a housing, is configured from sustainable and water-resistant materials, preferably paper and/or fiber cast.

14. **Method** for detecting a biologically active protein in a biological sample, preferably urine, whole blood, saliva, milk or serum, comprising the following steps:

    a. Obtaining a biological sample from an individual;
    b. Analyzing the biological sample with the use of an immunoassay according to any one of claims 1 to 12, which is adapted to detect the biologically active protein.

15. Use of an **auxiliary protein** as covering, masking and/or support protein in an immunoassay according to claim 1 to 12,
**characterized in that**
the auxiliary protein is obtained from a diatom and is vegan.

## Revendications

1. Immunodosage pour la détection d'un antigène biologiquement actif, en particulier d'une hormone, d'une protéine ou d'un médicament, dans un échantillon biologique d'un individu, présentant :

    - une zone d'application d'échantillon pour l'application d'un échantillon biologique d'un individu, l'échantillon biologique étant de préférence de l'urine, du sang total, de la salive, du lait ou du sérum ;
    - une zone de capture, la zone de capture présentant un premier anticorps immobilisé qui est dirigé contre l'antigène biologiquement actif, en particulier contre l'hormone, la protéine, le peptide ou le médicament ;
    - une zone de conjugué, la zone de conjugué présentant un deuxième anticorps, qui est dirigé contre l'antigène biologiquement actif, en particulier contre l'hormone, la protéine, le peptide ou le médicament ;

    **caractérisé en ce que**

    le premier anticorps et le deuxième anticorps sont des anticorps recombinants, qui ont été obtenus à partir d'une diatomée ou d'une plante monocellulaire, l'immunodosage présentant au moins un anticorps supplémentaire et/ou une protéine auxiliaire,
    la protéine auxiliaire, en tant que bloqueur, saturant les surfaces libres d'un récipient de réaction ou d'une membrane,
    l'anticorps supplémentaire ou la protéine auxiliaire ayant été obtenu(e) à partir d'une diatomée et/ou d'une plante verte et/ou d'une plante monocellulaire et l'anticorps recombinant étant mis à disposition à une pureté d'au moins 90% et
    l'immunodosage étant végan.

2. Immunodosage selon la revendication 1, l'anticorps recombinant étant obtenu à une concentration de 20-1000 mg/l, plus préférablement de 30-800 mg/l, en variante d'au moins 30-160 mg/l de culture d'une diatomée.

3. Immunodosage selon l'une des revendications 1 ou 2, la glycosylation du premier anticorps et/ou du deuxième

EP 4 587 833 B1

anticorps présentant un motif de glycosylation modifié, par rapport à l'anticorps natif correspondant, de préférence, la glycosylation présentant un motif plus homogène d'un facteur d'homogénéité dans la plage de 1 à 3, de manière particulièrement préférée, la glycosylation présentant un motif N-glycane homogène, riche en mannose présentant un facteur d'homogénéité de préférence dans la plage de 1 à 3.

4. Immunodosage selon l'une des revendications 1 à 3, la séquence d'acides aminés du premier anticorps et/ou du deuxième anticorps

   a) présentant un anticorps des vertébrés, de préférence mammifère, de manière particulièrement préférée humain ; ou
   b) présentant une séquence d'acides aminés, ou étant constitué par celle-ci, qui présente une identité de séquence d'au moins 80%, de préférence d'au moins 85% de manière particulièrement préférée d'au moins 90%, de manière tout particulièrement préférée d'au moins 95%, en particulier d'au moins 97%, par rapport à des zones de séquence homologue d'un anticorps des vertébrés et/ou mammifère et/ou humain.

5. Immunodosage selon l'une des revendications 1 à 4, le premier anticorps et/ou le deuxième anticorps étant un anticorps qui est dirigé contre la choriogonadotropine humaine (hCG).

6. Immunodosage selon l'une des revendications 1 à 5, l'antigène biologiquement actif étant une partie de l'épitope d'un virus, de préférence d'un virus pathogène, par exemple l'influenza, le SARS-CoV-2, le VRS, un adénovirus, un streptocoque du groupe A, le norovirus, le rotavirus, le VIH.

7. Immunodosage selon l'une des revendications 1 à 6, l'antigène biologiquement actif étant une séquence associée à une tumeur, de préférence un complexe HLA et/ou une partie séquencée d'un épitope tumoral et/ou un marqueur tumoral, par exemple IFN-γ, IL-8, PSA, CEA, AFP, DCP, CA 125, HER2/neu.

8. Immunodosage selon l'une des revendications 1 à 7, l'antigène biologiquement actif étant une séquence caractéristique pour l'identification d'une protéine, de préférence une étiquette d'enzyme, de manière particulièrement préférée choisie dans la liste constituée par $His_6$-Tag, Strep-Tag, c-Myk-Tag, Flag-Tag et GST-Tag.

9. Immunodosage selon l'une des revendications 1 à 8, l'antigène biologiquement actif étant une séquence associée à des paramètres alimentaires, par exemple la transcobalamine II, la ferritine, l'homocystéine, l'acide eicosapentaénoïque (EPA) et l'acide docosahexaénoïque (DHA) ou le calcitriol.

10. Immunodosage selon l'une des revendications 1 à 9, l'immunodosage étant un immunodosage à flux latéral, qui met à disposition, de manière fluidiquement reliée, au moins une zone d'application d'échantillon, une zone de conjugué et une zone de capture, qui sont agencées de manière fluidiquement reliée sur une membrane.

11. Immunodosage selon l'une des revendications 1 à 10, l'immunodosage étant réalisé sous forme d'un immunodosage ELISA (Enzyme-Linked Immunosorbent Assay - dosage d'immuno-absorption enzymatique) dans lequel au moins

   a. une zone d'application d'échantillon pour l'application d'un échantillon biologique est mise à disposition, laquelle zone
   b. est limitée par une zone de capture, qui est réalisée comme une limite de récipient et/ou une partie d'une limite de récipient, de préférence comme une plaque de microtitrage et
   c. par une zone de conjugué, qui est agencée spatialement dans la zone d'application d'échantillon.

12. Dispositif, présentant :

   - un récipient, en particulier un boîtier, et
   - un immunodosage selon l'une des revendications 1 à 11, en particulier un immunodosage à flux latéral, agencé dans celui-ci.

13. Dispositif selon la revendication 12, dans lequel le récipient, de préférence un boîtier, est créé en matériaux durables et résistants à l'eau, de préférence en papier et/ou en cellulose moulée.

14. Procédé de détection d'une protéine biologiquement active dans un échantillon biologique, de préférence de l'urine, du sang total, de la salive, du lait ou du sérum, comprenant les étapes suivantes :

EP 4 587 833 B1

a. obtention d'un échantillon biologique à partir d'un individu ;
b. analyse de l'échantillon biologique à l'aide d'un immunodosage selon l'une des revendications 1 à 11, qui est conçu de telle sorte que celui-ci détecte la protéine biologiquement active.

15. Utilisation d'une protéine auxiliaire comme protéine de recouvrement, de masquage et/ou de soutien dans un immunodosage selon l'une des revendications 1 à 11,
**caractérisé en ce que**
la protéine auxiliaire est obtenue à partir d'une diatomée et est végane.

35

**Fig. 1**

**Fig. 2**

**Fig. 3**

Fig. 4

**Fig. 5 (Start)**

Fig. 5 (fortgesetzt)

**Fig. 6 (Start)**

**Fig. 6(fortgesetzt)**

Fig. 7 (Start)

**Fig. 7 (fortgesetzt)**

**Fig. 8**

A  HASP1A (distal)  →  HASP1B (proximal) →

CATACAGTGAATGTAACTTTCGAATT
GACAGTATTAGTAGTCGTATTGACAG
TGAGGCACGCCCCTCAATGTGCGAG
GTGGAAAATATACCAGCATGACAATG
AATCTTGGAGATTCTTTTGCTGTCAT
CAAGATTCACCGCCAAATCTTCAGG
AACCTATCACGTCCACAGGCGATGTT
AATTCTTGAGTCGTCAAAACAAAGT
CCTGTCCTACCTGTAGAAGTTGACAG
CGAGCAATTGTATGCAAACTTCTGAC
TTTGTTATAATAACATTAAAGGTAATT
AAGTATCTTCAATTAGGCATTTTGTC
ACTGTCAGTCCGTTCCGACAATATAG
GTAGATTTGGAATGAATCTTTTCTAT
GCT

CATACAGTGAATGTAACTTTCGAATTGAC
AGTATTAGTAGTCGTATTGACAGTGAGGC
ACGCCCCTCAATGTGCGAGGTGGAAAAT
ATACCAGCATGACAATGAATCTTGGAGAT
TCTTTTGCTGTCATCAAGATTCACCGCCA
AATCTTCAGGAACCTATCACGTCCACAGG
CGATGTTAATTCTTGAGTCGTCAAAACAA
AGTCCTGTCCTACCTGTAGAAGTTGACAG
CGAGCAATTGTATGCAAACTTCTGACTTT
GTTATAATAACATTAAAGGTAATTAAGTAT
CTTCAATTAGGCATTTTGTCACTGTCAGTC
CGTTCCGACAATATAGGTAGATTTGGAAT
GAATCTTTTCTATGCTGCTGCGAATCTTGT
ACACCTTTGAGGCCGTAGATTCTGTCCGA
CGAAGCGATAATTATTGCAAAATACATGG
ACTCATTATTTTGATTCGATTTCTTTTTGG
TATCCGACTCGAAAAGATCCATCACGGCG
AGC

**Fig. 9A**

**Fig. 9B**

**Fig. 10**

**Fig. 11**

**Fig. 12**

| M | Pt | mIgG | Pt | Pt | mIgG | Pt | Pt | mIgG | Pt |
|---|----|----|----|----|----|----|----|----|----|
| | mIgG_1 (0,75 µg/mL) | | | mIgG_2 (0,75 µg/mL) | | | mIgG_2 (0,15 µg/mL) | | |

**Fig. 13**

**Fig. 14**

**Fig. 15**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 6080560 A
- EP 2671950 A1
- EP 2444495 A1
- EP 2660323 A1